(19) Europäisches Patentamt
European Patent Office
Office européen des brevets

(11) **EP 4 215 042 A1**

(12) **EUROPEAN PATENT APPLICATION**

(43) Date of publication:
**26.07.2023 Bulletin 2023/30**

(21) Application number: **22152603.1**

(22) Date of filing: **21.01.2022**

(51) International Patent Classification (IPC):
**A01K 67/027** (2006.01)   **C07K 14/725** (2006.01)
**C07K 14/74** (2006.01)   **C12N 15/85** (2006.01)

(52) Cooperative Patent Classification (CPC):
**A01K 67/027; C07K 14/7051; C07K 14/70539;
C12N 15/8509;** A01K 2207/15; A01K 2217/052;
A01K 2217/15; A01K 2267/01; C12N 2015/8518;
C12N 2800/90; C12N 2840/20

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicants:
• **Max-Delbrück-Centrum für Molekulare Medizin
13125 Berlin (DE)**
• **T-Knife GmbH
13125 Berlin (DE)**

(72) Inventors:
• **BLANKENSTEIN, Thomas
Berlin (DE)**

• **DHAMODARAN, Arunraj
Berlin (DE)**
• **KNACKSTEDT, Lorenz
Berlin (DE)**
• **PONCETTE, Lucia
Berlin (DE)**
• **KIEBACK, Elisa
Berlin (DE)**

(74) Representative: **Schiweck Weinzierl Koch
Patentanwälte Partnerschaft mbB
Ganghoferstraße 68 B
80339 München (DE)**

Remarks:
The complete document including Reference
Table(s) and the Sequence Listing(s) can be
downloaded from the EPO website

(54) **A NON-HUMAN MAMMAL COMPRISING IN ITS GENOME AT LEAST TWO HUMAN LEUKOCYTE ANTIGEN (HLA) CLASS I ALLELES, METHODS OF MAKING SUCH MAMMAL AND USES THEREOF**

(57)    The present invention relates to a non-human mammal comprising in its genome at least two human leukocyte antigen (HLA) class I alleles, wherein said at least two human HLA alleles are functionally expressed in that the corresponding MHC I polypeptides are expressed on the surface of cells of said mammal and present MHC antigens to which the non-human mammal provides an antigen-specific CD8+ T cell response and optionally wherein said at least two human HLA class I alleles comprise: and optionally wherein said at least two human HLA class I alleles comprise:

a) at least one human HLA-A allele, and/or
b) at least one human HLA-B allele, and/or
c) at least one human HLA-C allele, and/or
d) (a) and (b); and/or
e) (a) and (c); and/or
f) (b) and (c); and/or
g) (a), (b) and (c).
    The present invention further relates to uses of such a transgenic mammal for generating one or more T cell receptors that are capable of binding to an antigen of interest.

EP 4 215 042 A1

**Description**

**SEQUENCE LISTING**

This application contains a Sequence Listing in computer readable form, which is incorporated herein by reference.

**TECHNICAL FIELD**

The present invention relates to a non-human mammal comprising in its genome at least two human leukocyte antigen (HLA) class I alleles which can be used as a novel tool for the isolation of human T cell receptors selected on a distinct human HLA class I haplotype for adoptive T cell therapy of cancer. The present invention further relates to a nucleic acid construct comprising a nucleic acid encoding at least two human HLA class I alleles, an expression vector comprising such a nucleic acid construct as well as respective host cell comprising such an expression vector. The invention further relates to a method of modifying endogenous HLA alleles of a non-human mammal and to a method of producing a non-human, mammalian oocyte carrying a modified target sequence in its genome. The invention also relates to a method of producing a non-human mammal carrying a modified target sequence in its genome. The invention also relates to a method of generating one or more T cell receptors that are capable of binding to an antigen of interest as well as to a method of identifying an epitope to which an immune response can be elicited.

**BACKGROUND OF THE INVENTION**

For efficacious adoptive T cell therapy (ATT), the appropriate selection of tumour-specific antigens (TSAs) is crucial. Recurrent somatic driver mutations yield ideal TSAs for ATT to achieve complete tumour elimination, possibly without relapse. *In silico* algorithms can predict epitopes as TSAs for ATT, however, with low probability. Thus, the accuracy of prediction algorithms still needs validation in describing processed neoepitopes.

Simultaneous validation of proteasomal processing of predicted TSAs and identification of neoantigen-specific T cell receptors (TCRs) is possible from transgenic mice. One such model developed by Li et al. (Nature Medicine, Vol. 16, No. 9 (2010), pages 1029-1034) are the ABabDII mice which have the complete human TCR gene loci, knocked out for murine TCR and MHC class I gene loci, and it is transgenic for human HLA class I allele, HLA-A*02:01 (can also be referred to herein as HLA-A2). Though isolation of human TCRs is possible from ABabDII mice, the model is limited as a source of obtaining only HLA-A2-restricted TCRs, thereby restricting epitope identification to HLA-A2 antigens. Moore et al "Humanization of T cell-mediated immunity in mice" Sci. Immunol. 6, eabj4026 (2021) also report transgenic mice, term "VelociT" mice which have been engineered with one human MHC-I (HLA-A2) and one MHC-II (HLA-DR2) allele, while eliminating the respective mouse MHC.

There is thus a need for broadening of TCR repertoire for HLA restricted TCR isolation. Accordingly, it is an object of the invention to provide tools and methodologies that allows broadening the TCR repertoire.

**SUMMARY OF THE INVENTION**

This object is inter alia accomplished by a transgenic non-human mammal, nucleic acid constructs, compositions, methods and uses having the features of the respective independent claims.

In a first aspect, the invention provides a non-human mammal that comprises in its genome at least two human leukocyte antigen (HLA) class I alleles (e.g., at least three HLA class I alleles, at least four HLA class I alleles, at least five HLA class I alleles, or more), wherein said at least two human HLA alleles are functionally expressed in that the corresponding MHC I polypeptides are expressed on the surface of cells of said mammal and present MHC antigens to which the non-human mammal provides an antigen-specific CD8+ T cell response. In some embodiments, the at least two human HLA class I alleles comprise: (a) at least one human HLA-A allele; and/or (b) at least one human HLA-B allele; and/or (c) at least one human HLA-C allele; and/or (d) (a) and (b); and/or e) (a) and (c); and/or f) (b) and (c); and/or g) (a), (b) and (c). In an embodiment the non-human mammal comprises in its genome at least three human leukocyte antigen (HLA) class I alleles, wherein said at least three human HLA class I alleles comprise: at least one human HLA-A allele; at least one human HLA-B allele; and at least one human HLA-C allele.

In a second aspect, the invention provides a nucleic acid construct comprising a nucleic acid encoding at least two human HLA class I alleles, wherein said nucleic acid construct is capable of functionally expressing said at least two human HLA alleles in that the corresponding MHC I polypeptides are expressed on the surface of cells of said mammal and present MHC antigens to which the non-human mammal provides an antigen-specific CD8+ T cell response and, optionally, wherein said at least two human HLA class I alleles comprise: (a) at least one human HLA-A allele; and/or (b) at least one human HLA-B allele; and/or (c) at least one human HLA-C allele; and/or (d) (a) and (b); and/or

(e) (a) and (c); and/or (f) (b) and (c); and/or (g) (a), (b) and (c). In an embodiment the nucleic acid construct comprises a nucleic acid encoding least three HLA class I alleles, wherein said at least three human HLA class I alleles comprise: at least one human HLA-A allele; at least one human HLA-B allele; and at least one human HLA-C allele.

In a third aspect, the invention provides an expression vector comprising a nucleic acid construct as described herein.

In a fourth aspect, the invention provides a host cell (e.g., an isolated and/or recombinant host cell) comprising a nucleic acid construct as described herein and/or an expression vector as described herein.

In a fifth aspect, the invention provides a method of modifying endogenous HLA alleles of a non-human mammal, the method comprising: transducing and/or transplanting a non-human mammal as described herein with a nucleic acid construct and/or an expression vector as described herein.

In a sixth aspect, the invention provides a method of producing a non-human, mammalian oocyte carrying a modified target sequence in its genome, the method comprising the steps of introducing into a non-human, mammalian oocyte a nucleic acid construct and/or an expression vector as described herein.

In a seventh aspect, the invention provides a method of producing a non-human mammal carrying a modified target sequence in its genome, the method comprising:

(a) producing an oocyte as described herein;
(b) analysing the offspring delivered by the non-human female host to which the oocyte obtained in (a) has been transferred for the presence of the modification.

In an eight aspect, the invention provides a non-human mammal produced and/or modified by a method of producing a non-human mammal carrying a modified target sequence in its genome as described herein.

In a ninth aspect, the invention provides a method of generating one or more T cell receptors that are capable of binding to an antigen of interest, the method comprising administering to a non-human animal as described herein an antigen of interest and/or a nucleic acid encoding the antigen of interest.

In a tenth aspect, the invention provides a T cell receptor obtained by or obtainable by a respective method as described herein.

In an eleventh aspect, the invention provides a method of identifying an epitope to which an immune response can be elicited, the method comprising administering to a non-human animal as described herein an antigen of interest suspected to contain an epitope to which an immune response can be elicited.

In a twelfth aspect, the invention provides an epitope to which an immune response can be elicited, the epitope being identified by a respective method as described herein. An epitope can be natural or non-natural. Non-natural applications of the epitope, such as an ex vivo mixture comprising (i) a peptide having the amino acid sequence of an epitope identified by a method described herein and (ii) one or more MHC proteins (e.g., one or more MHC class I or class II proteins).

In a thirteenth aspect, the invention provides the use of a non-human animal as described herein for generating one or more T cell receptors that are capable of binding to an antigen of interest.

In a fourteenth aspect, the invention provides the use of a non-human animal as described herein for identifying an epitope to which an immune response can be elicited.

In a fifteenth aspect, the invention provides the use of a non-human animal as described herein for identifying one or more T cell receptors that are capable of binding to an antigen of interest.

## BRIEF DESCRIPTION OF THE DRAWINGS

The invention will be better understood with reference to the detailed description when considered in conjunction with the non-limiting examples and the drawings, in which:

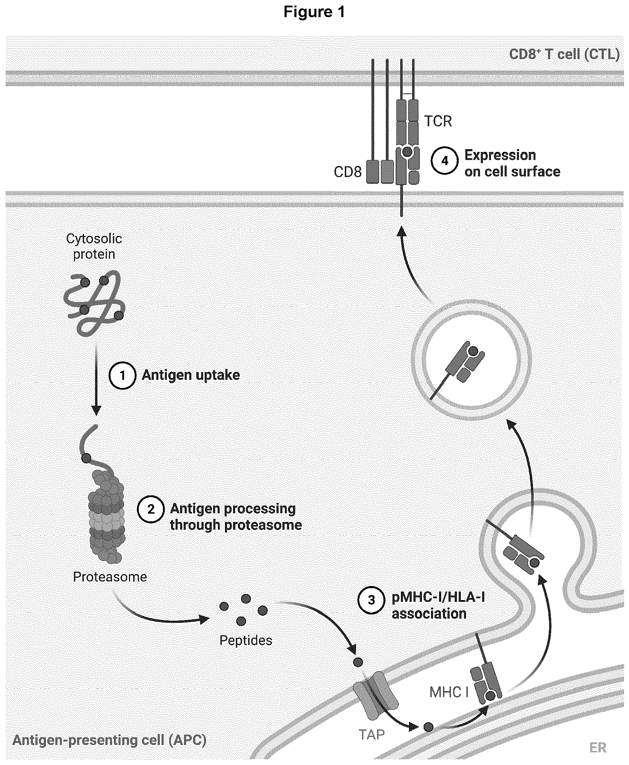

**Fig. 1** shows a schematic representation of the pMHC-I/HLA-I antigen processing pathway (this simplified version does not represent all codomains and adapter proteins involved as chaperones in the antigen processing pathway). 1. Antigen uptake in the cytoplasm by the proteasome. 2. Proteins are processed by protease activity inside the proteasomal core. 3. TAP protein complex facilitate the transport of peptides into ER. PLC, Erp57, and CRT proteins instruct the peptide loading onto MHC/HLAs. 4. Peptide transport through the secretory pathway to the cell surface for presentation to CTLs.

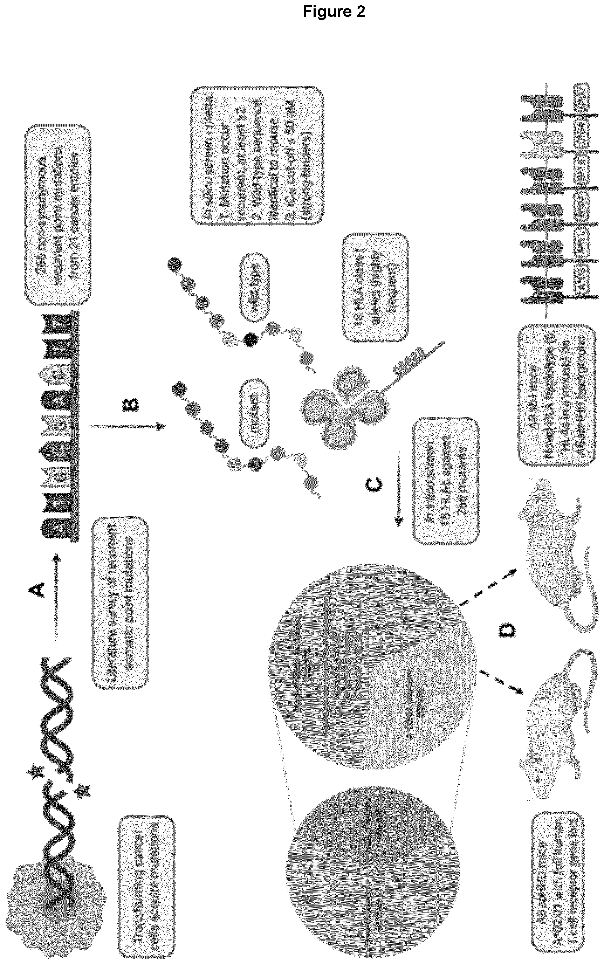

**Fig. 2** shows an illustration of tumour-specific neoantigens binding a distinct human HLA class I haplotype. Step A is a schematic representation that depicts recurrent neoantigens derived from cancer-driving point mutations. Step B illustrates wild-type and mutant configurations screened against 18 class I alleles using the affinity prediction software, NetMHC Server-DTU, while step C depicts HLA binding strengths of 266 mutants revealed non-HLA-A2 binders. Step D shows in silico screen that provided the basis for the generation of ABab.I mice that are described herein as an example of a non-human transgenic mammal as described herein with six novel HLA class I human alleles.

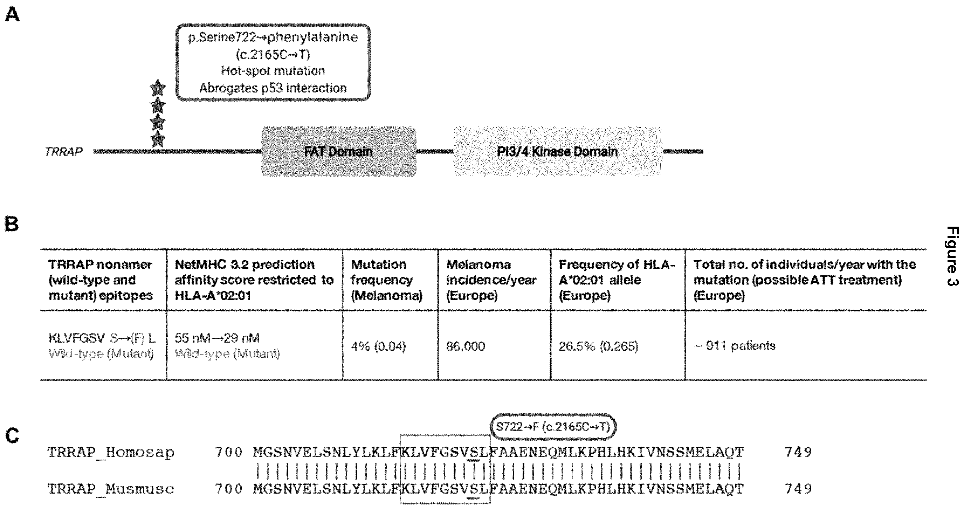

**Figs. 3A-3C** show HLA-A2-restricted TRRAP-S722F neoantigen as a target for ATT. **Fig. 3A** depicts the TRRAP gene. Asterisks indicate the location of the recurrent mutation in the proline-rich N-terminal domain. **Fig. 3B** shows the nonamers, mutant epitope-S722F ('aa' change highlighted in red), and the wild-type epitope-S722 (in green), with predicted IC50 (to HLA-A2) of 29 nM and 55 nM respectively. TRRAP-S722F mutation as a target for ATT of melanoma could benefit approx. 911 patients/year. **Fig. 3C** shows the presence of human TRRAP orthologue in the mouse. Position of mutation underlined. Nonameric epitope highlighted in red square. TRAP nonamer wild type (SEQ ID NO: 27), TRAP nonamer mutant (SEQ ID NO: 30), TRRAP_Homosap (SEQ ID NO: 28), TRRAP_Musmusc (SEQ ID NO: 29).

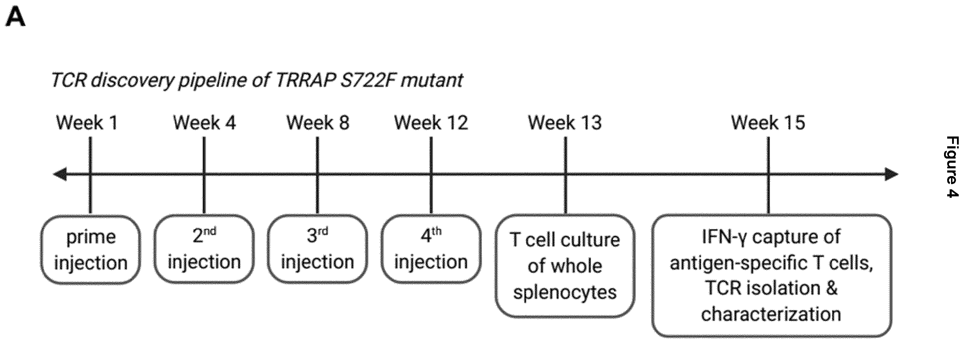

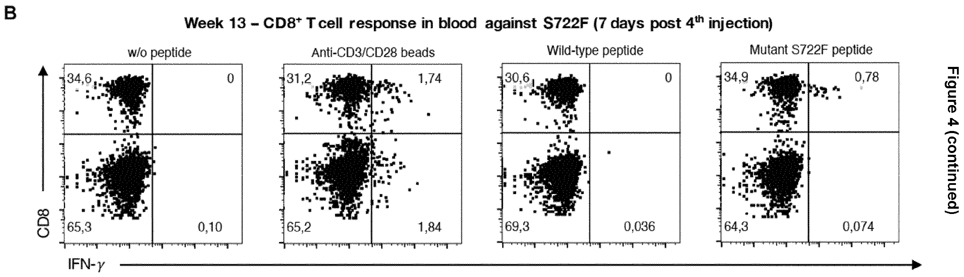

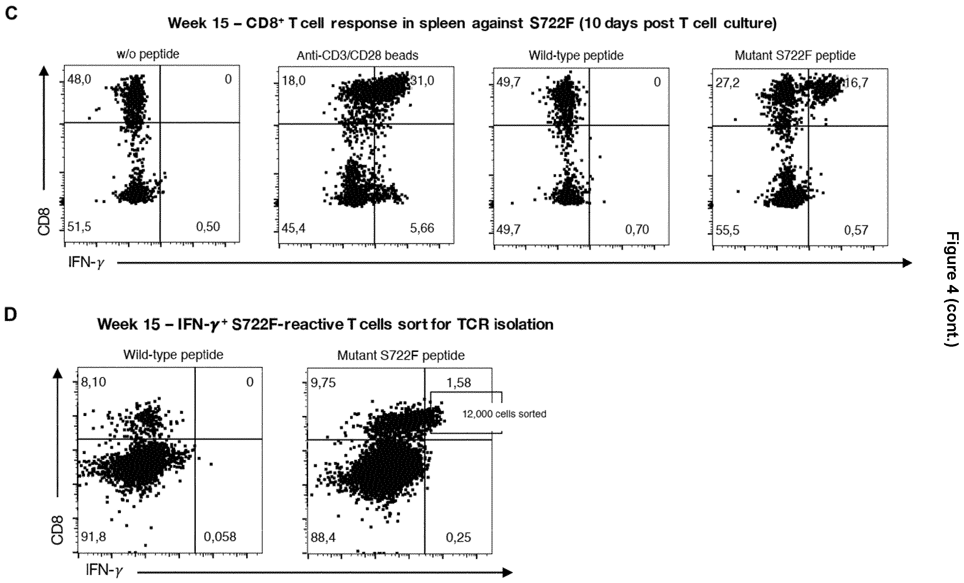

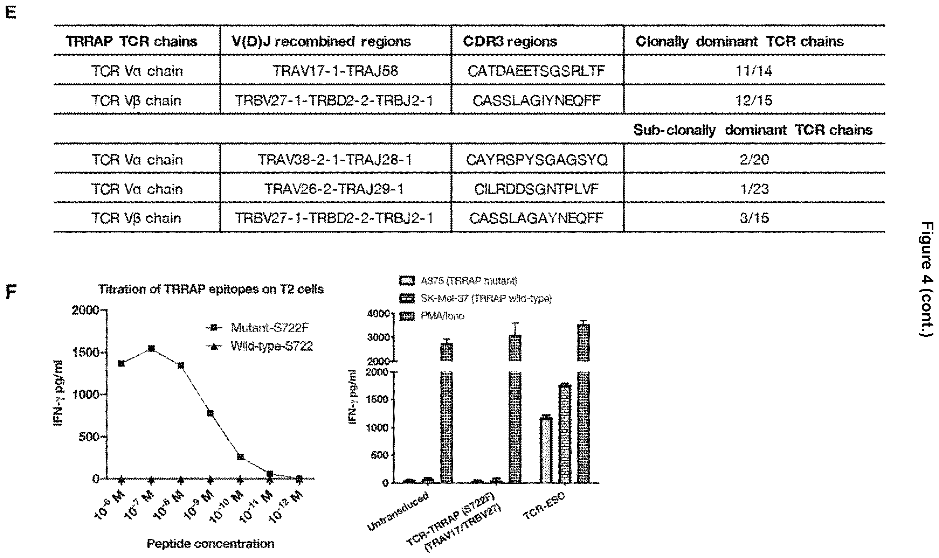

**Figs. 4A-4F** show the generation and characterization of TRRAP S722F TCRs in ABabDII mice. **Fig. 4A** depicts the TCR discovery pipeline with the immunization schedule as used in an illustrative example. **Fig. 4B** depicts that CD8+ T cells from ABabDII showed S722F peptide reactivity by IFN-$\gamma$ production (gated on CD3+ cells, n = 3). Controls with wild-type S722 peptide or without (w/o) peptide. One representative flow cytometry plot out of three responder mice is given. **Fig. 4C** shows an in vitro cultured splenic CD8$^+$ T cells (CD4-depleted) from responder mouse produced IFN-$\gamma$ to $10^{-9}$ M peptide. **Fig. 4D** shows an IFN-$\gamma$ capture assay enriched S722F-specific T cells (CD8$^+$IFN-$\gamma^{high}$). **Fig. 4E** shows a 5'-RACE RT-PCR amplified clonally dominant and sub-dominant TCR variable chains. **Fig. 4F** shows on the **left,** a dominant V$_{\alpha\beta}$ chains transduced in human T cells were co-cultured with TAP$^{-/-}$ T2 cells ($1 \times 10^5$) loaded with titrated peptide nonamers (concentration: $10^{-6}$ M to $10^{-12}$ M) and IFN-$\gamma$ release was measured by ELISA. **Fig. 4F** shows on the **right,** an endogenous processing assay with cell lines naturally expressing mutant TRRAP. IFN-$\gamma$ levels measured after overnight co-culture of PBMCs ($1 \times 10^5$) either with A375 cells (mutant-S722F$^+$, NY-ESO$^+$) or with SK-Mel-37 (wild-type-S722$^+$, NY-ESO$^+$). The TCR-ESO recognizes the NY-ESO$_{157-165}$ epitope. Bar graph represents mean $\pm$ SD (n = 2). One representation out of three co-culture experiments is given.

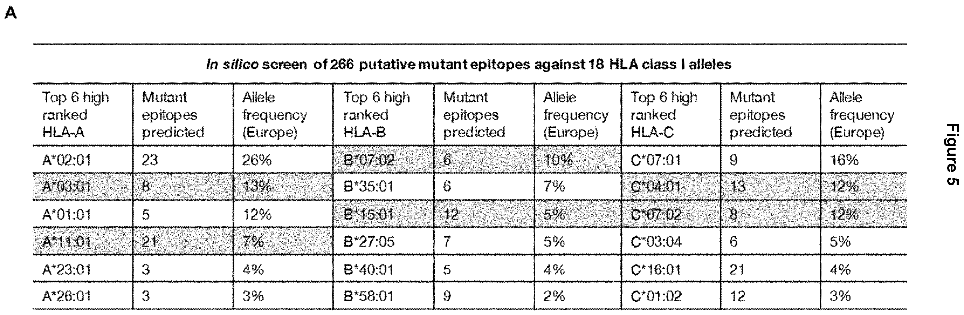

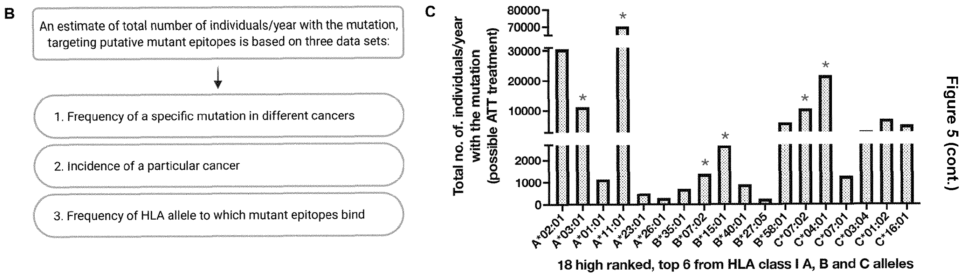

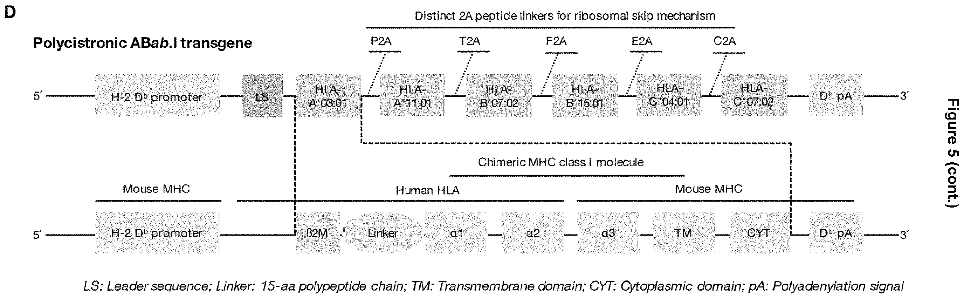

**Figs. 5A-5D** show the design and construction of polycistronic ABab.I transgene. **Fig. 5A** shows an overview of total numbers of in silico predicted mutant epitopes across 18 HLA alleles. The top 6 HLA alleles (shaded in blue) reflect a natural human HLA haplotype (the genes present in the ABab.I transgene). **Fig. 5B** shows a multiplying the three data sets estimated the no. of individuals/year with the mutation. **Fig. 5C** shows a total no. of individuals/year with the mutation (possible for ATT treatment) was calculated for 175 mutant binders against 18 alleles, using data from A. and B. Red asterisks highlight top high-ranked HLA alleles. **Fig. 5D** shows on the **top,** a polycistronic ABab.I transgene designed to encode six HLA alleles segregated via five different 'self-cleaving' viral 2A peptide linkers driven by a single H-2 Db promoter and polyadenylation signal. **Fig. 5D** shows on the **bottom,** a single monochain construct design. Each HLA is a chimeric human-mouse fusion monochain containing $\alpha$1 and $\alpha$2 heavy chain cDNA regions from respective human HLA alleles, while $\alpha$3, transmembrane and cytoplasmic domains from murine H-2 Db gene fused by a glycineserine polylinker to human $\beta$2m.

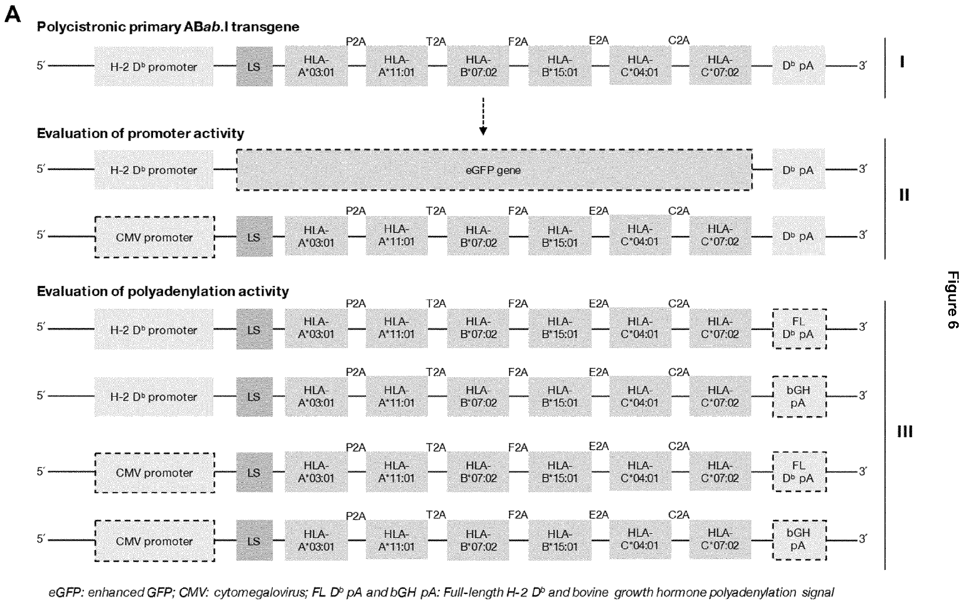

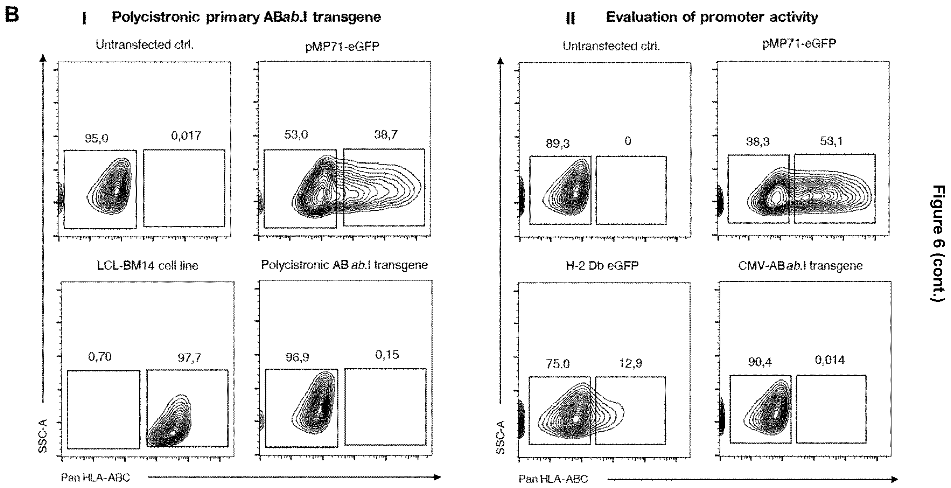

**Figs. 6A-6B** show an HLA protein expression analysis of the polycistronic ABab.I transgenes. **Fig. 6A** shows the ABab.I transgenes: primary construct (I) and modified versions (II, III) from the initial design. I. Primary transgene in which viral peptide 2A linkers segregate the HLAs. II. Modified transgenes: H-2D$^b$ promoter-driven eGFP gene and CMV promoter-driven HLAs. **III.** Modified transgenes with different polyadenylation signal sequences. Dotted boxes represent modifications to the initial primary construct. **Fig. 6B** shows a surface staining of transiently transfected MCA205 cells with constructs from **A. I. and II.** is shown (**III.** is not shown). Cells were stained 48 hours post-transfection using pan HLA-ABC antibody (clone: W6/32). Controls: pMP71- eGFP (GFP vector, measured in same channel) and LCL-BM14 (lymphoblastoid cells), positive for HLA ABC. One representation out of multiple flow cytometry experiments is given (n > 5).

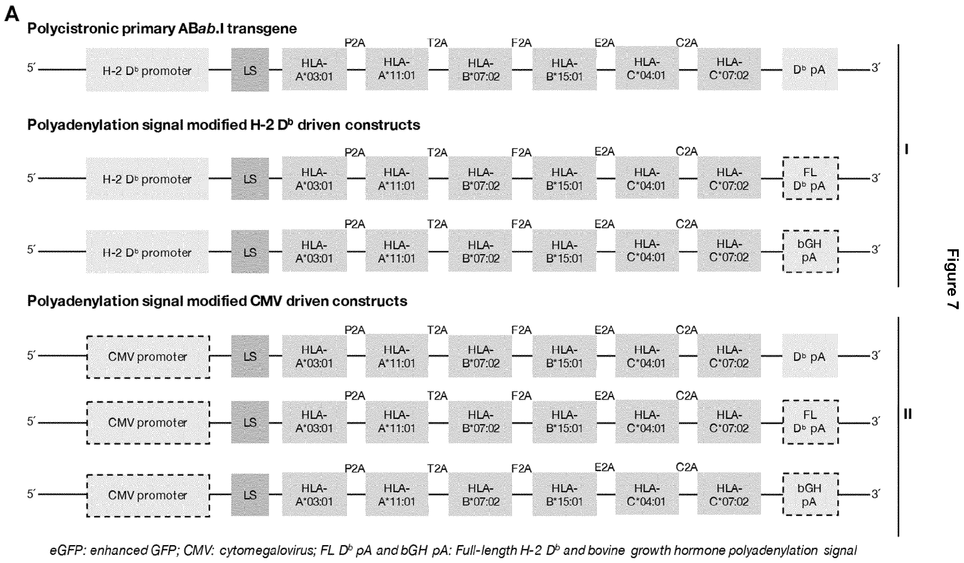

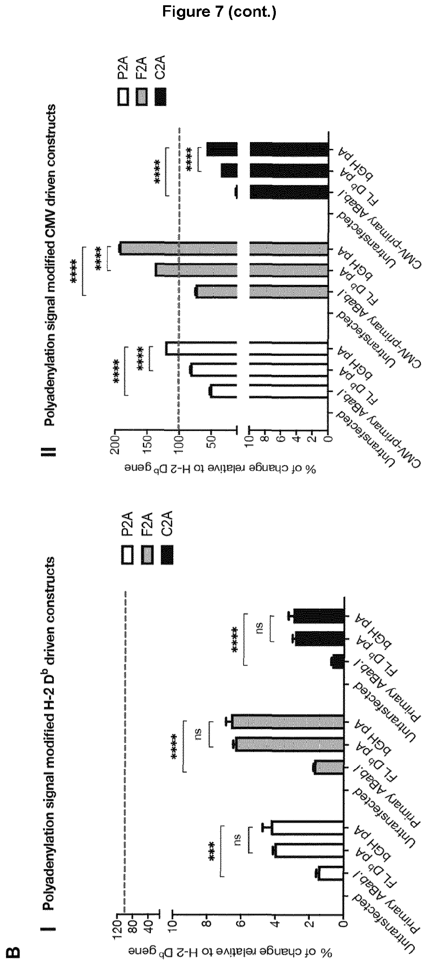

**Figs. 7A-7B** show a mRNA expression profile of polycistronic ABab.I transgenes. **Fig. 7A** shows **I., Top,** The ABab.I primary transgene. **I., Bottom,** Polyadenylation (pA) signal-modified constructs driven by H-2 D$^b$ or **II.** CMV viral promoter. **Fig. 7B** shows a quantitative RT-PCR analysis of mRNA transcripts from constructs shown in **Fig. 7A., I. and II.** cDNA regions of ABab.I transgenes were amplified from MCA205 cell lysates 48 hours after transfection using primers flanking 2A peptide linkers (P2A, F2A, and C2A). Percentage change relative to constitutive H-2 D$^b$, mouse MHC class I gene (red dotted lines) is compared. Bar graphs represent mean $\pm$ SD (n = 3). P values: ****, P $\leq$ 0.0001; ***, P $\leq$ 0.001; ns, not significant (unpaired two-tailed t-test).

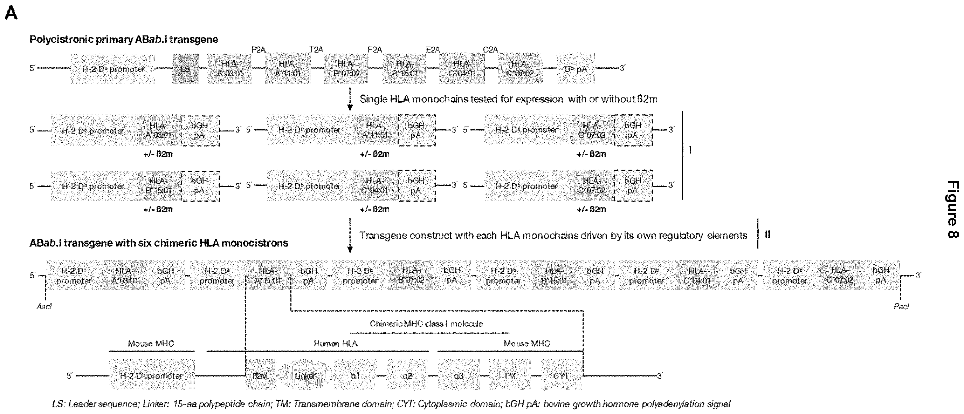

**Figs. 8A-8C** show a HLA expression analysis of single monocistrons from ABab.I transgene. **Fig. 8A** shows in **I.,**

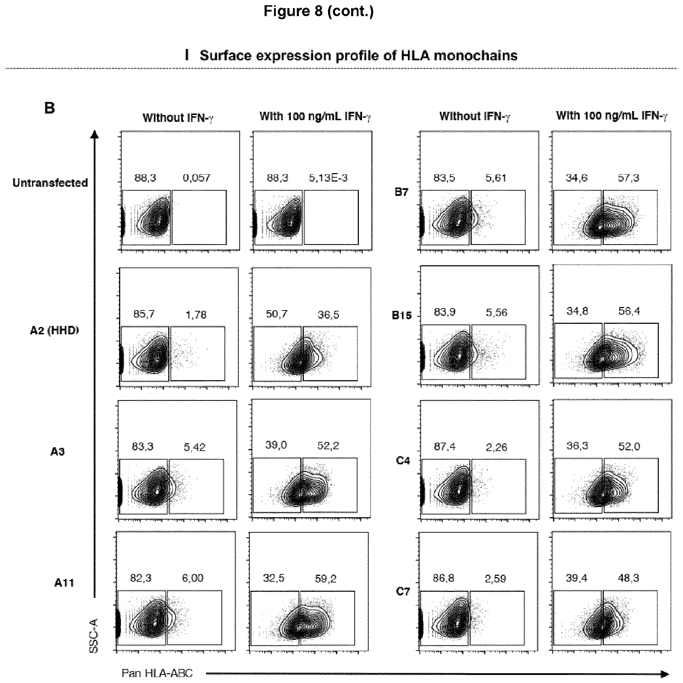

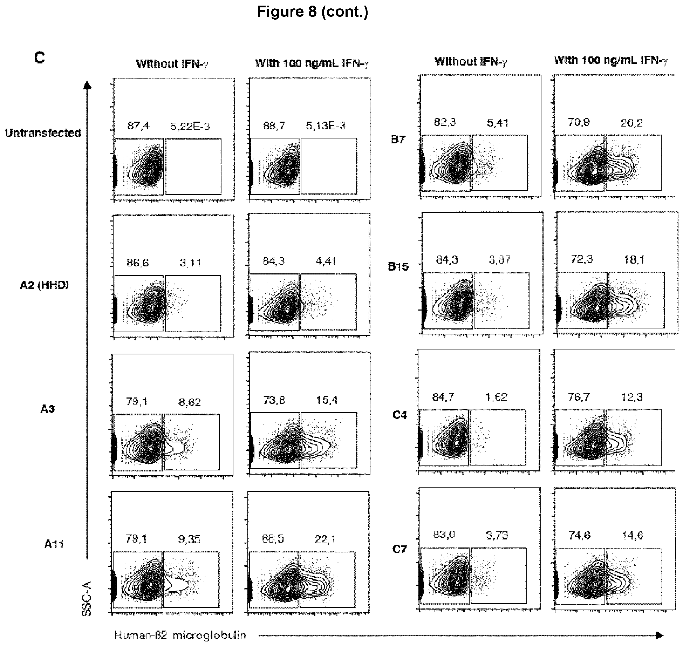

a single monochains, with or without β2m, were cloned from ABab.I primary transgene. Every HLA allele as a monocistron is driven by its own H-2Db promoter and pA signal. Dotted boxes represent additional modifications from the primary construct. **II.** The ABab.I transgene expresses six monocistrons (as shown in **Fig. 8A. I.**) as a single haplotype (5'-to-3') with its own regulatory elements. **Fig. 8B** and **Fig. 8C** show MCA205 cells expressing six monochains that were surface stained with or without IFN-γ pre-treatment. The HLA-A2 protein was stained for reference. Cells were stained 48 hours post-transfection using, a pan HLA-ABC antibody (clone: W6/32) in **Fig. 8B,** and a human-β2 microglobulin antibody (clone: TÜ99) in **Fig. 8C**. One representative flow cytometry plot out of three experiments is given.

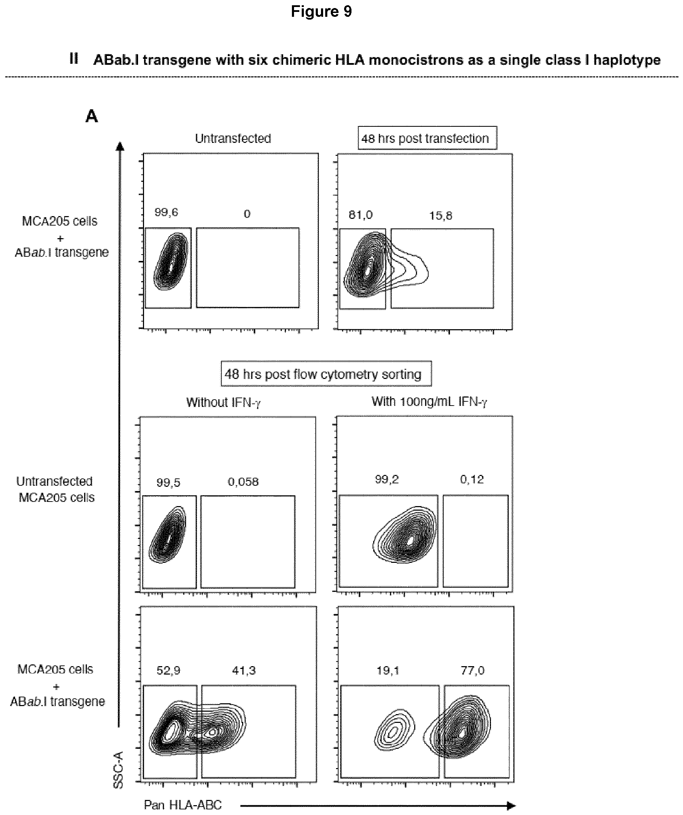

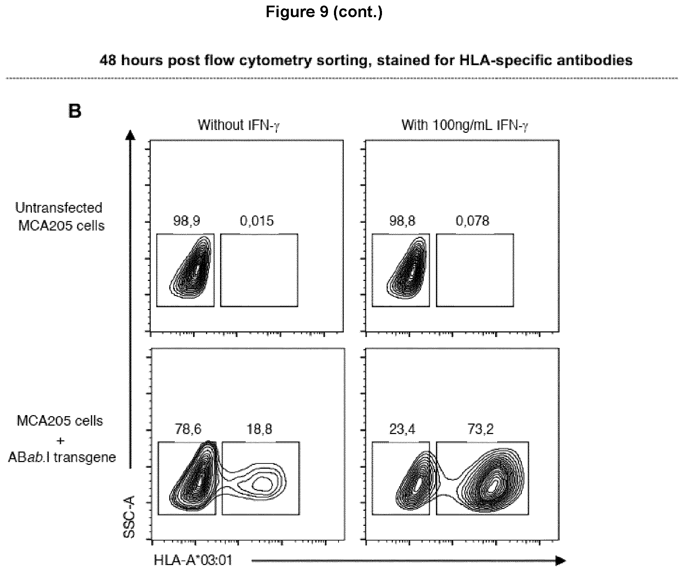

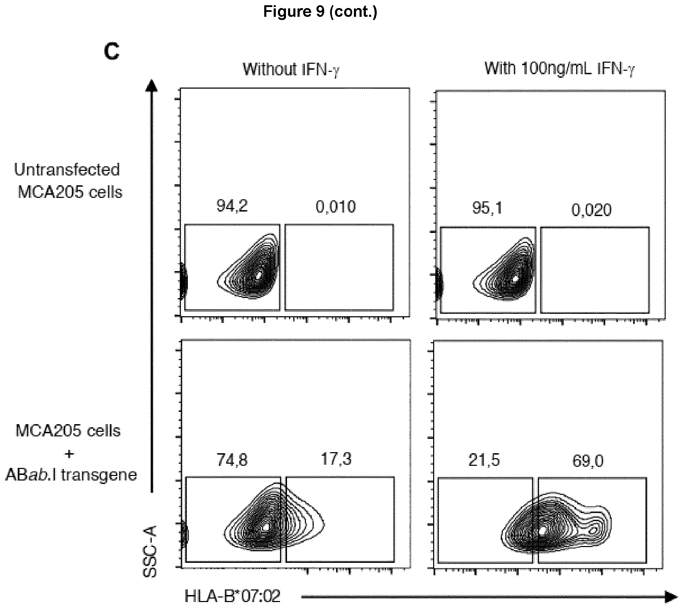

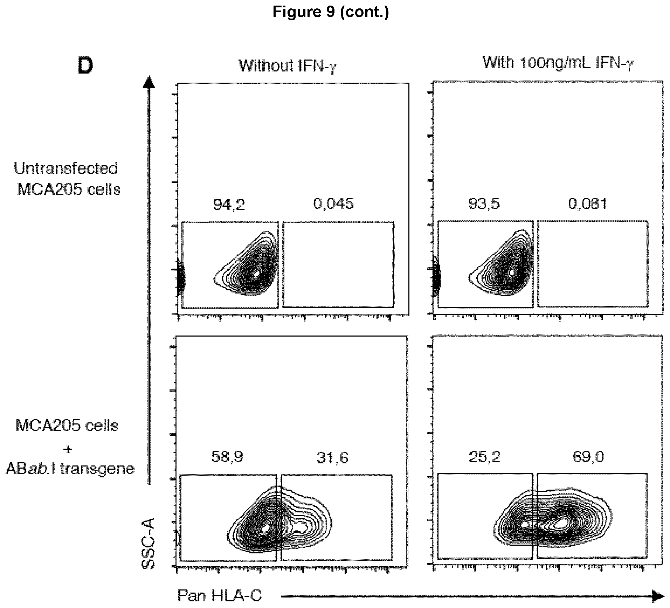

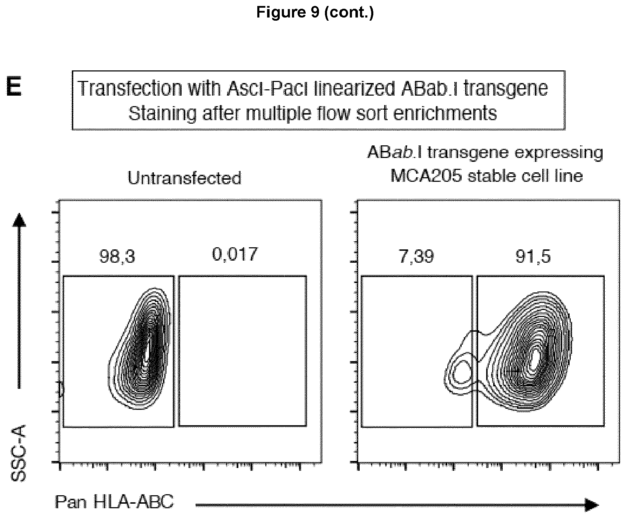

**Figs. 9A-9E** show an HLA haplotype expression profile of ABab.I transgene in MCA205 cell line. **Fig. 9A** shows a MCA205 cells were stained using pan HLA-ABC antibody. **Top,** HLA class I haplotype expressing six alleles (depicted in Figure 8 A. II.) was stained 48 hours post-transfection. **Bottom,** Transfected MCA205 cells were enriched for HLA+ cell population and stained for HLA alleles with or without IFN-γ pre-treatment (48 hours prior staining). **Fig. 9B., Fig. 9C.,** and **Fig. 9D** show post- enrichment, MCA205 cells were stained using HLA-specific antibodies, pre-treated with or without IFN-γ. **Fig. 9B** shows a HLA-A*03:01 specific antibody (clone: GAP.A3) stained HLA-A3 allele. **Fig. 9C** shows a HLA-B*07:02 specific antibody (clone: BB7.1) stained HLA-B7 allele. **Fig. 9D** shows a pan HLA-C antibody (clone: H-5) stained HLA-C alleles. **Fig. 9E** shows a MCA205/ABab.I stable cell line was stained using pan HLA-ABC antibody post multiple cell sorting by flow cytometry. One representative example out of multiple flow cytometry staining experiments is given (n > 5).

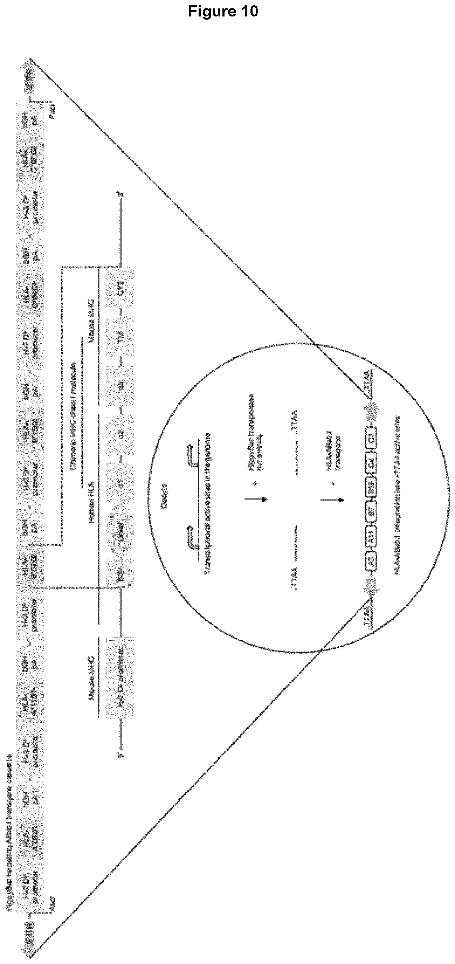

**Fig. 10** shows PiggyBac transposons target ABab.I transgene into oocytes. Schematic representation of PiggyBac (PB) transposon strategy for the generation of ABab.I mice using pronuclei microinjection technology. The PB targeting ABab.I targeting transgene (flanked by ITRs for PB transposase catalysis) has repeating elements of six chimeric HLA genes constructed one after another (5'-to-3') as depicted in **Fig. 7 A. II.** each with its own promoter and 3'-UTR components.

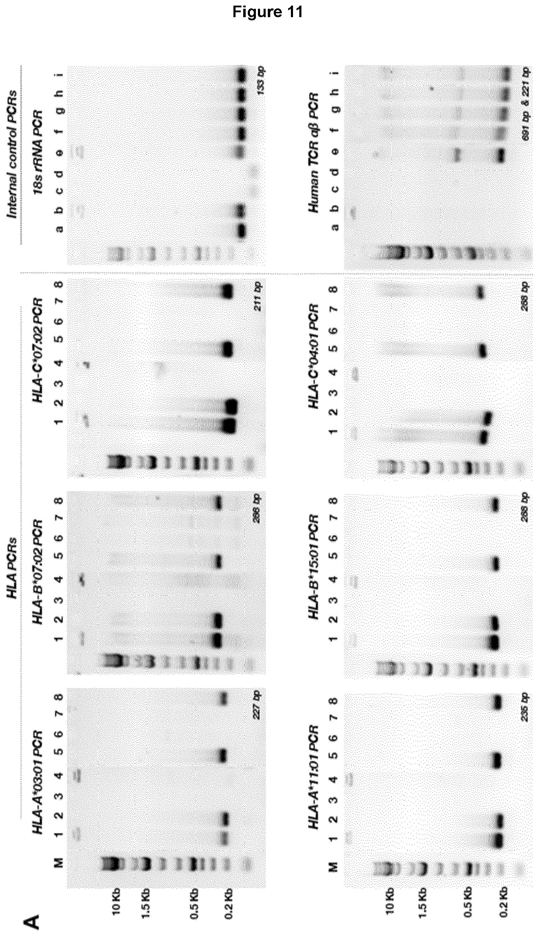

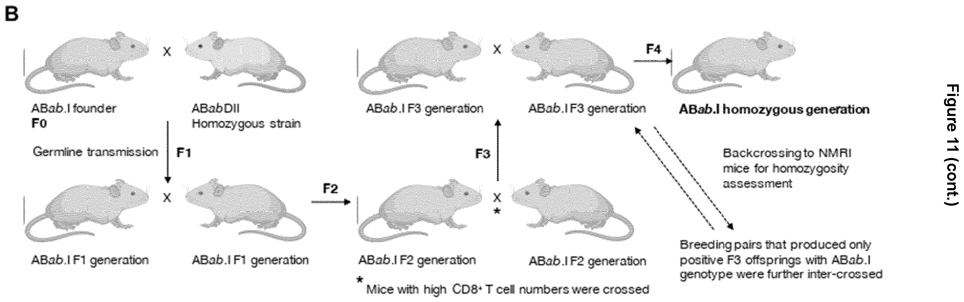

**Figs. 11A-11B** show a genotyping profile and breeding scheme of ABab.I transgenic founders. **Fig. 11A** on the **left**, shows HLA-PCR reactions: PCR genotyping data of 2 founder mice (F0), Q4115 and Q4118. HLA-A*03, A*11, B*07, B*15, C*04, and C*07 PCRs: Genomic (g) DNA from MCA205/ABab.I stable cell line (lane 1), ABab.I transgene as plasmid DNA (lane 2), control H2O (lane 3), gDNA from C57BL6/N mice (lane 4), positive ABab.I, 1st founder: Q4115 (lane 5), two negative mice (lane 6 and 7), positive ABab.I, 2nd founder: Q4118 (lane 8). **Fig. 11A** on the **right** shows an internal control PCRs: 18s rRNA and human TCR αβ PCRs: Genomic (g) DNA from C57BL6/N mice (lane a), gDNA from MCA205/ABab.I stable cell line (lane b), ABab.I transgene as plasmid DNA (lane c), control H2O (lane d), gDNA from ABabDII mice (lane e), 1st founder: Q4115 (lane f), two negative mice (lane g and h), positive ABab.I, 2nd founder: Q4118 (lane i). **Fig. 11B** shows breeding scheme representation from F0 to F4 homozygous mice. F0 backcrossed with ABabDII for germline transmission. NMRI backcrossing was used for homozygosity testing.

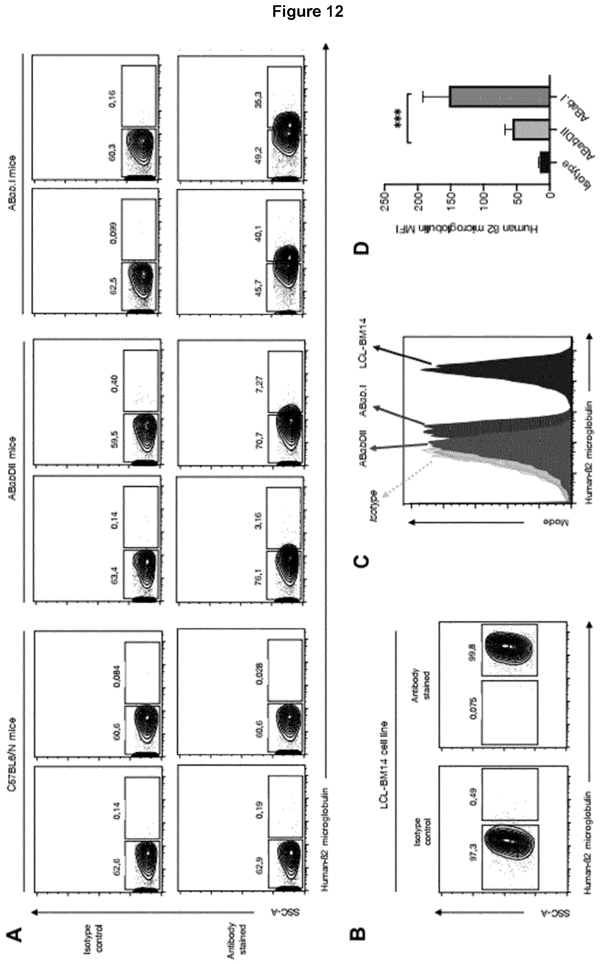

**Figs. 12A-12D** show a human HLA class I haplotype expression in ABab.I mice. **Fig.** 12A shows a HLA staining of peripheral blood cells from C57BL6/N, ABab.I and ABabDII mice was performed using β2 microglobulin (β2m) antibody. Two representative plots (two mice/strain) out of multiple experiments are given (n > 10). **Fig. 12B** shows a LCL-BM14 (lymphoblastoid cells), positive for HLA ABC, stained with β2m antibody. **Fig. 12C depicts** a histogram showing mean fluorescence intensity (MFI) of β2m staining among mouse strains: ABabDII and ABab.I, and cell line, LCL-BM14. One representative staining out of multiple mice is given (n > 20). **Fig. 12D** shows a β2m MFI compared between ABabDII and ABab.I mice. Bar graph represents mean ± SD (n = 5). P value: ***, P ≤ 0.001 (unpaired two-tailed t-test).

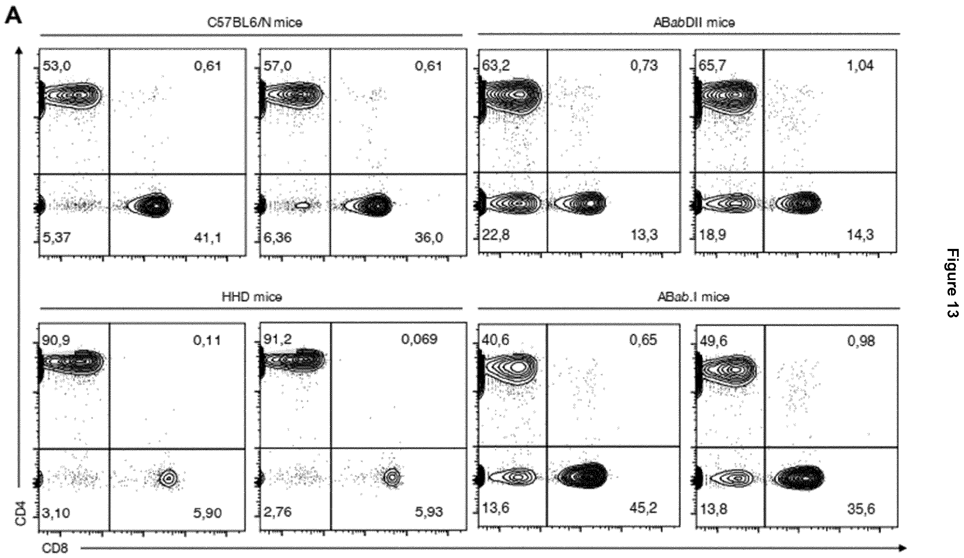

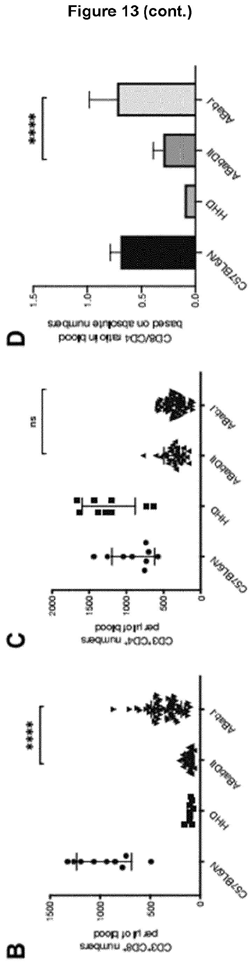

**Figs. 13A-13D** show a phenotypic characterization of peripheral blood T cells in ABab.I mice. **Fig. 13A** shows CD8+ CD4+ profiling of peripheral blood cells (on CD3+ cells) from young (8-12 weeks) mice by flow cytometry, in which indicated mouse strains were stained with antibodies specific for CD3, CD8, and CD4 chains and analyzed by flow cytometry (gated on CD3$^+$ lymphocytes). Two representative plots (two mice/strain) out of multiple experiments is given (n > 30). **Fig. 13B and Fig. 13C** show absolute T cell numbers/μl blood, **Fig. 13B** shows a CD3$^+$CD8$^+$ and **Fig. 13C** shows a CD3$^+$CD4$^+$ were quantified using CountBright™ beads by flow cytometry. Each data point represents a single young mouse from indicated strains. Horizontal intervals on scattered charts represent mean ± SD. Summarized data from, C57BL6/N (n = 9), HHD (n = 9), ABabDII (n = 32), and ABab.I (n = 60) mice. **Fig. 13D** shows a CD8/CD4 ratio based on absolute numbers in peripheral blood among different mice strains. Bar graph represents mean ± SD (n = 7). P values in **Fig. 13B, Fig. 13C and Fig. 13D** indicate: ****, P ≤ 0.0001; ns, not significant (unpaired two-tailed t-test).

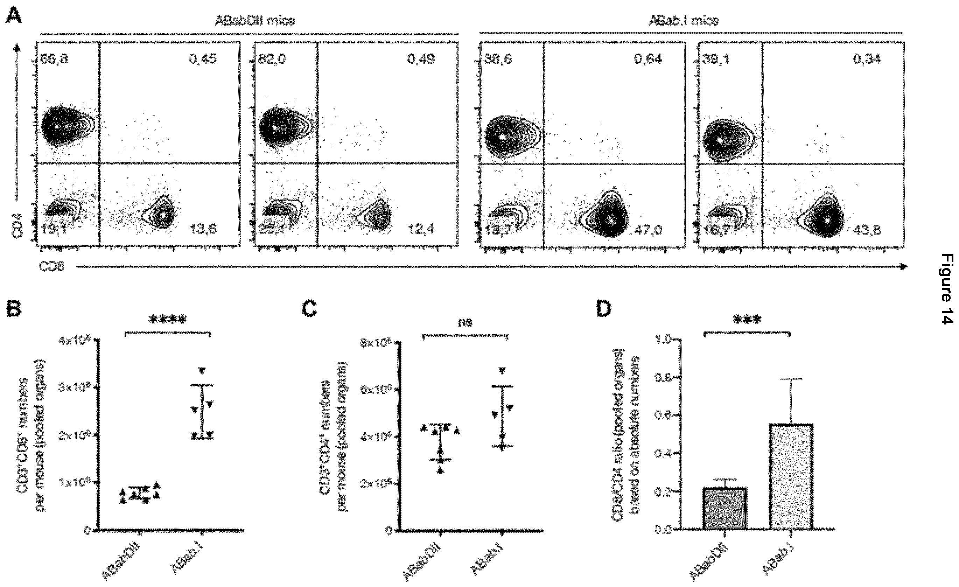

**Figs. 14A-14D** show a phenotypic characterization of T cells from lymphoid organs in ABab.I mice. **Fig. 14A** shows spleen and lymph nodes including whole blood from young (8-12 weeks) indicated mice strains were stained with antibodies specific for CD3, CD8, and CD4 chains and analyzed by flow cytometry (gated on CD3$^+$ lymphocytes). Two representative plots (two mice/strain) out of multiple experiments are given (n > 10). **Fig. 14B and Fig. 14C** show absolute T cell numbers/mouse, **Fig. 14B** shows a CD3$^+$CD8$^+$ and **Fig. 14C** shows a CD3$^+$CD4$^+$ were quantified using CountBright™ beads by flow cytometry. Each data point represents a single young mouse from indicated strains. Horizontal intervals on scattered charts represent mean ± SD. Summarized data from, ABabDII (n = 7), and ABab.I (n = 5) mice. **Fig. 14D** shows a CD8/CD4 ratio based on absolute numbers in pooled organs among different *mice strains. Bar graphs represent mean ± SD (n = 5). P values in* **Fig. 14B, Fig. 14C and Fig. 14D** *indicate: ****, P ≤ 0.0001; ***, P ≤ 0.001; ns, not significant (unpaired two-tailed t-test).*

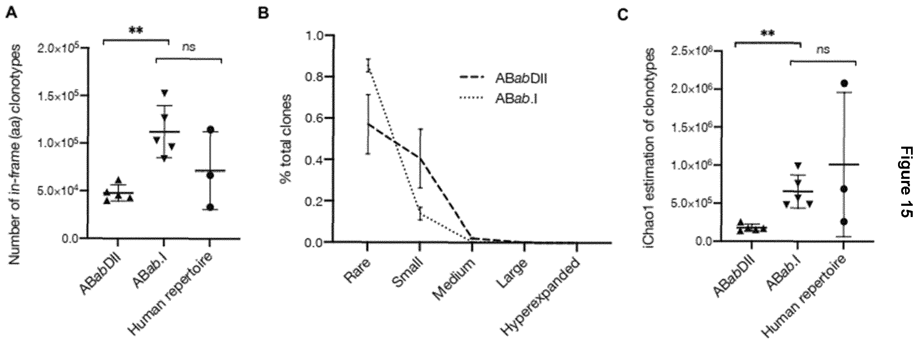

**Figs. 15A-15C** show a comparison of TCRβ repertoire among ABabDII, ABab.I, and human donors. **Fig. 15A** shows absolute numbers of unique TCRβ amino acid (aa) clonotypes within $3 \times 10^5$ (average in mice strains) or $1.8 \times 10^5$ human CD8+ T cells. **Fig. 15B** shows a clonal distribution of TCRβ aa clonotypes of different sizes: rare, 0 < x ≤ 0.001%; small, 0.001% < x ≤ 0.01%; medium 0.01% < x ≤ 0.1%; large, 0.1% < x < 1%; hyperexpanded, 1% < x < 10%. Human donors were excluded from this analysis. **Fig. 15C** shows a TCRβ diversity was calculated using the iChao1 estimator (lower bound richness of total numbers of unique templates within an individual repertoire). Horizontal intervals on scattered charts represent mean ± SD. Data are from, ABabDII (n = 5); ABab.I (n = 5) mice, and humans (n = 2 for **Fig. 15B**; n = 3 for **Fig. 15A and Fig. 15C**). P values in **Fig. 15A and Fig. 15C** indicate: **, P ≤ 0.01; ns, not significant (unpaired two-tailed t-test).

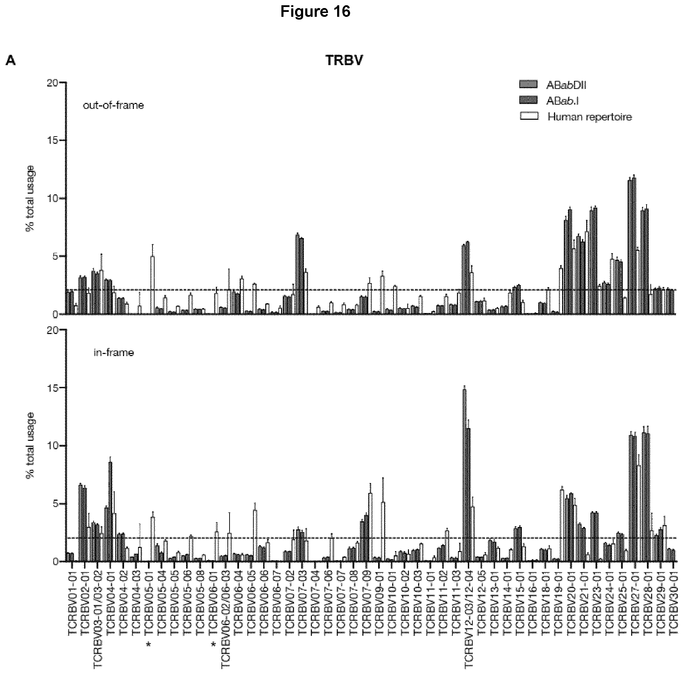

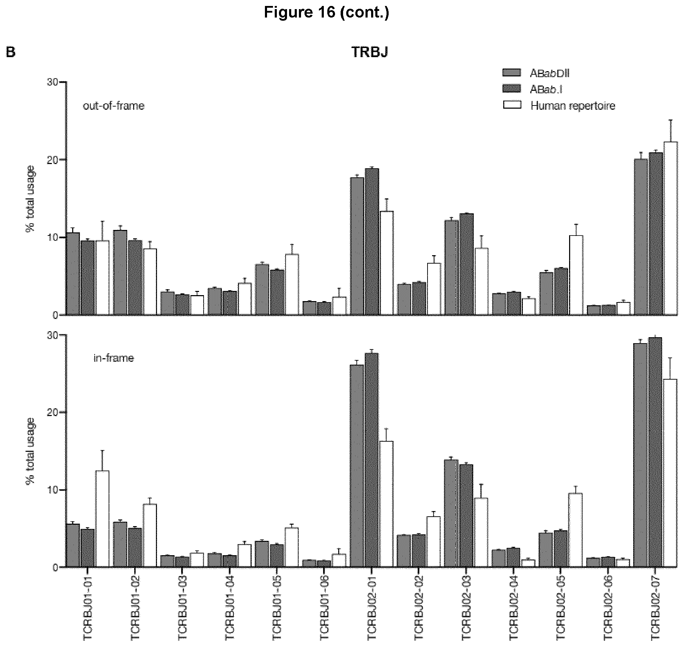

**Figs. 16A-16B** show V$_\beta$ and J$_\beta$ gene usage frequencies of in-frame and out-of-frame TCRβ clonotypes. **Fig. 16A** and **Fig. 16B** show frequencies of V$_\beta$ and J$_\beta$ gene usages of unique TCRβ clonotypes in CD8$^+$ T cells of ABabDII and ABab.I mice, and humans. **Fig. 16A** and **Fig. 16B** show on the **top,** out-of-frame and, on the **bottom,** in-frame frequencies. Arrangement of V$_\beta$ gene (**A.**) or J$_\beta$ (**B.**) segments mentioned on the *x* -axis according to their position on the human chromosome from 5' to 3'. The dotted line in **Fig. 16A** represents the frequency of random V$_\beta$ gene usage (TRBV, 2.1%). Bar graphs represent mean ± SD. Data are from, ABabDII (n = 5); ABab.I (n = 5) mice, and humans (n = 3). * Expression of V$_\beta$ genes is missing in both ABabDII and ABab.I mice.

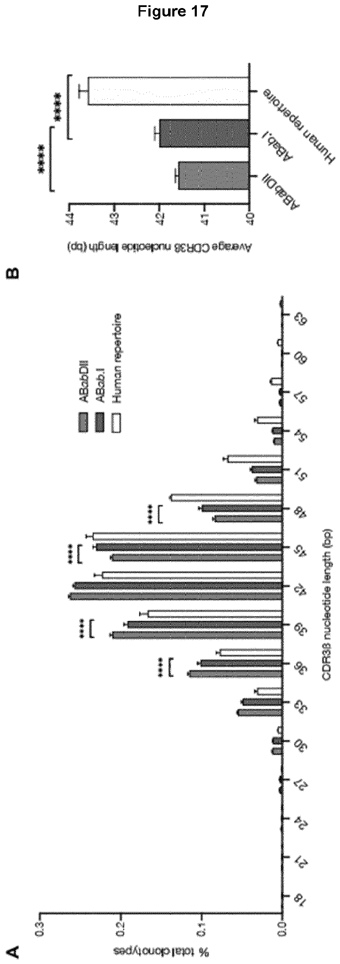

**Figs. 17A-17B** show a CDR3β region analysis among ABabDII, ABab.I, and human donors. **Fig. 17A** shows a CDR3 length distribution of TCRβ clonotypes. In-frame frequencies of different CDR3β lengths for ABabDII and ABab.I mice, and humans are shown in the bar graph. **Fig. 17B** shows average CDR3β lengths were compared between ABabDII and ABab.I mice, and humans. Bar graphs represent mean ± SD. Data are from, ABabDII (n = 5); ABab.I (n = 5) mice, and humans (n = 3). P values in **Fig. 17A** and **Fig. 17B** indicate: ****, P ≤ 0.0001 (unpaired two-tailed t-test).

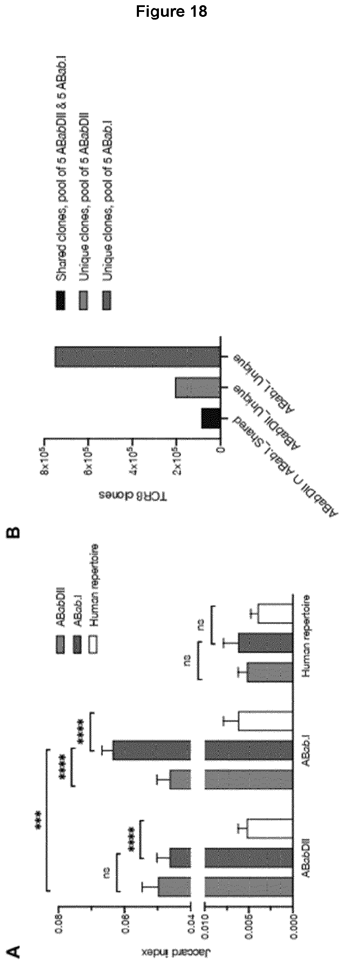

**Figs. 18A-18B** show a shared repertoire analysis among ABabDII, ABab.I and human donors. **Fig. 18A** shows the Jaccard index representing a score based on the number of shared TCRβ clones within and between groups of ABabDII and ABab.I mice, and human repertoire. Bar graph represents mean ± SD. Data are from, ABabDII (n = 5); ABab.I (n = 5) mice, and humans (n = 3). P values indicate: ****, P ≤ 0.0001; ***, P ≤ 0.001; ns, not significant (unpaired two-tailed t-test). **Fig. 18B** shows total numbers of unique TCRβ clones in ABabDII and ABab.I from pooled data. Five mice per strain were pooled for analysis.

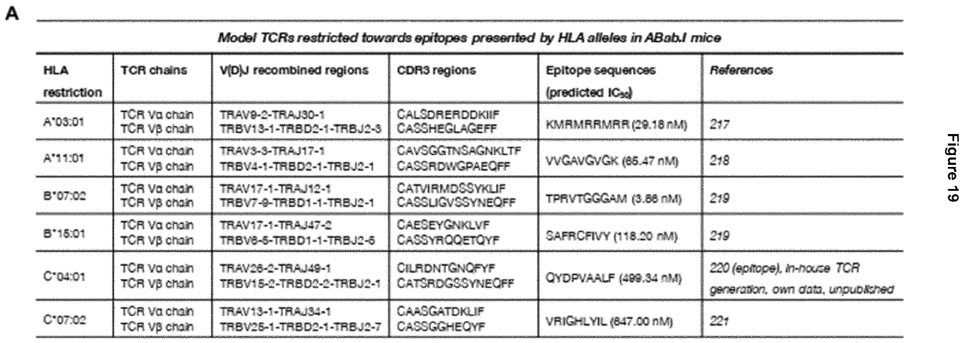

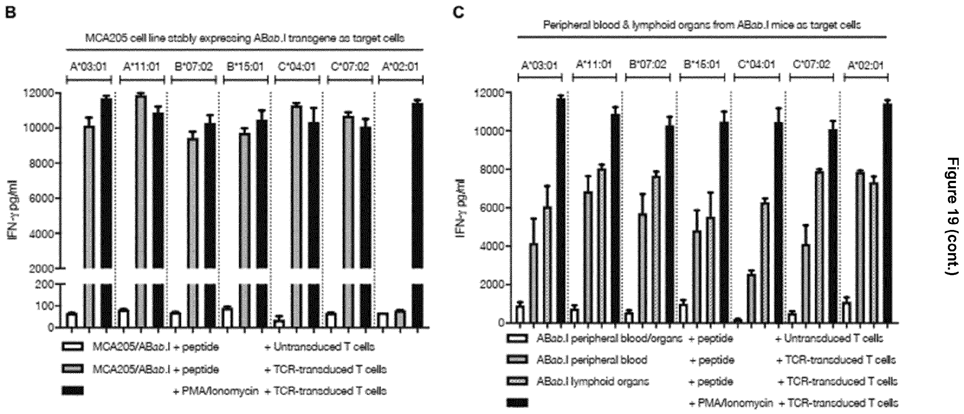

**Figs. 19A-19C** show a functional characterization of HLA alleles present in ABab.I mice. **Fig. 19A** shows a list of model TCRs restricted towards HLA alleles in ABab.I with known peptide binders. In addition to this list, HLA-A2-restricted T1367 MAGE-A1 TCR was used as an internal reference in all presentation assays. **Fig. 19B** and **Fig. 19C** show a functional activity of ABab.I HLA alleles through surface expression and model peptide presentation on, in **Fig. 19B,** MCA205 cell line stably expressing six HLA alleles as in ABab.I mice, except HLA-A2 or in **Fig. 19C,** ex vivo-isolated peripheral blood or cells from lymphoid organs, co-cultured with model TCR-transduced or un-transduced T cells. Cytokine levels were measured after overnight co-culture by mouse IFN-γ ELISA assay. pHLA-TCR pairs are mentioned in **Fig. 19A**. Bar graphs represent mean of intra-assay duplicates ± SD. One representation out of multiple co-culture experiments is given (n > 5).

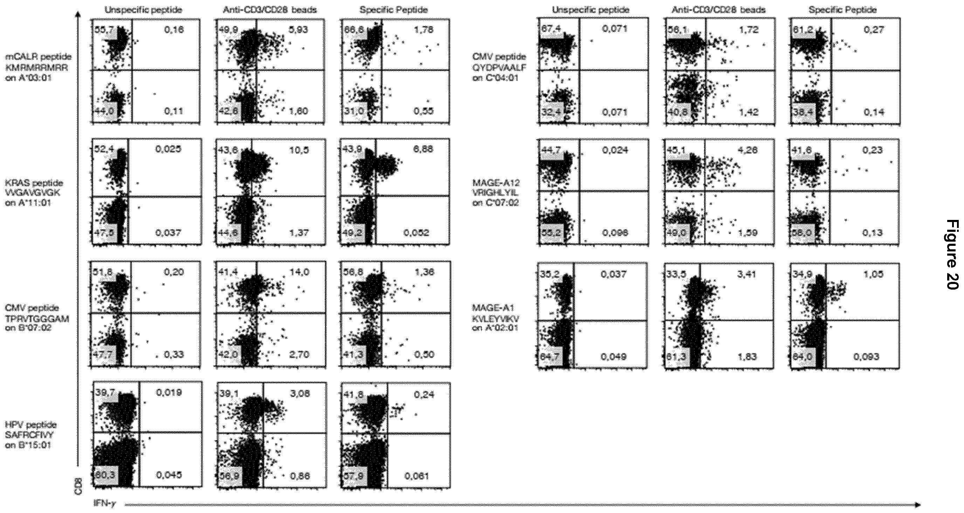

**Fig. 20** shows CD8$^+$ T cell responses against a panel of human antigens in ABab.I mice. Specific CD8$^+$ T cell responses in ABab.I mice was observed after immunization with a panel of model antigens (mentioned along *x*-axis). Young ABab.I mice were immunized (minimum twice) with an interval of at least 4 weeks between peptide injections. Seven days post- injection, peripheral blood cells were stimulated in vitro with the same specific peptides used for immunization (**right**), or unspecific peptides (**left**), or CD3/CD28 beads (**middle**, positive control). Cells were analyzed for CD3, CD8, and intracellular IFN-γ expression by flow cytometry. Dot plots depict CD8⁺ IFN-γ⁺ T cells (gated on CD3⁺ lymphocytes). Unspecific NY- ESO-APRGPHGGAASGL peptide (SEQ ID NO: 172) was used only for in vitro stimulation. One representative flow cytometry plot out of multiple responders is given (out of 6-7 mice/antigen, 6 mice responded against KRAS, 3 mice against MAGE-A1 and HPV, 1 mouse responded against mCALR, CMV, and MAGE-A12 peptides).

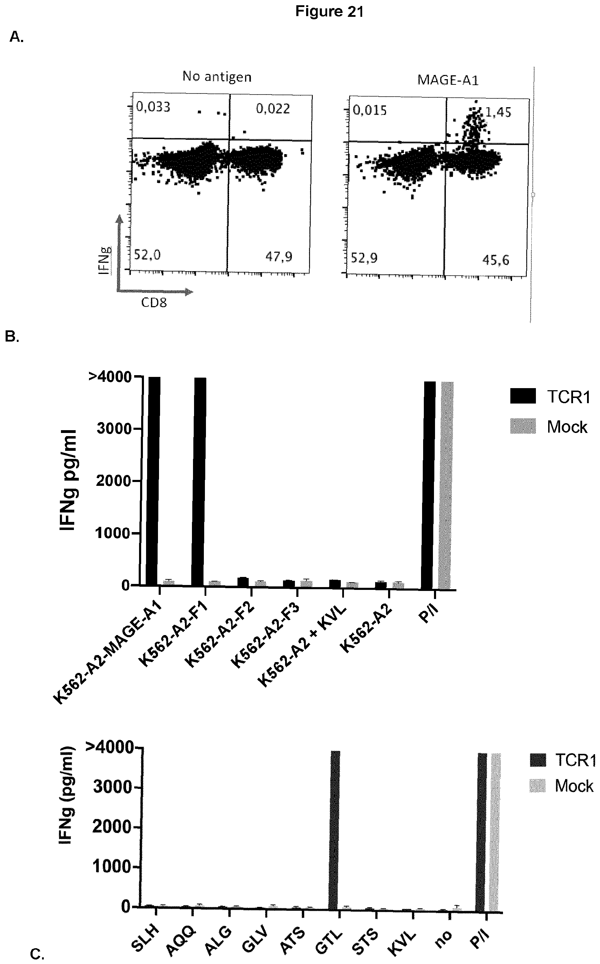

**Fig. 21A** shows the Immune response to MAGE-A1 restricted by HLA-A*02 in ABab.I mice following vaccination. An ABab.I mouse was immunized with full-length MAGE-A1 mutated at position 279 (V→D) to destroy the dominant HLA-A*02-restricted epitope KVLEYVIKV (278-286). On day 7 after the 3rd immunization, peripheral blood was stimulated with MAGE-A1-expressing cells and stained intracellularly for IFNg. **Fig. 21B** shows TCR1 isolated from an ABab.I mouse recognizes a novel HLA-A*02-restriced MAGE-A1 epitope. TCR1 was isolated from an ABab.I mouse immunized with full-length MAGE-A1 mutated at position 279 (V→D) to destroy the dominant HLA-A*02-restriced epitope KVL 278-286. TCR1-transduced human T cells were co-cultured with K562-A2 cells expressing MAGE-A1, fragments of MAGE-A1 (F1-F3) or loaded with KVLEYVIKV (KVL). F1-F3 span fragments of MAGE-A1 as follows: F1 1-97, F2 98-222, F3 223-309. **Fig. 21C** shows MAGE-A1-reactive TCR1 recognizes the nonamer GTLEEVPTA. TCR1-transduced human T cells were co-cultured with K562-A2 cells loaded with the predicted nonamer binders (**Table 15**) or with KVLEYVIKV (KVL).

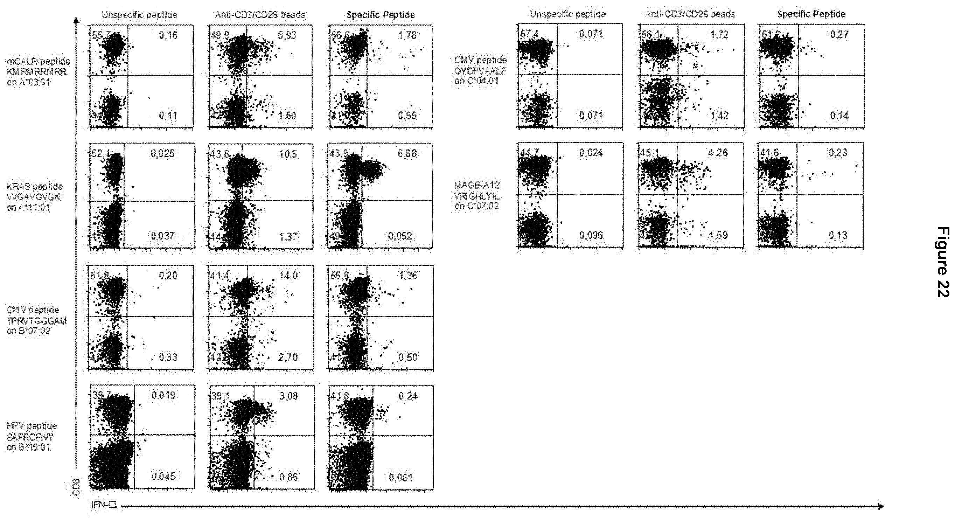

**Fig. 22** shows a flow cytometry analysis. By flow cytometry, CD8⁺ T cells were in vitro measured for CD3, CD8, and intracellularly IFN-γ expression. Dot plots in the figure depict CD8⁺ IFN-γ⁺ T cells (gated on CD3⁺ lymphocytes). As an unspecific peptide, NY-ESO peptide (APRGPHGGAASGL) was used (as a negative control) only for in vitro stimulations. One flow cytometry plot out of multiple responders is given (out of 6-7 mice/epitope, 6 mice responded. against KRAS, 3 mice against MAGE-A1 and HPV, 1 mouse responded against mCALR, CMV, and MAGE-A12 peptides).

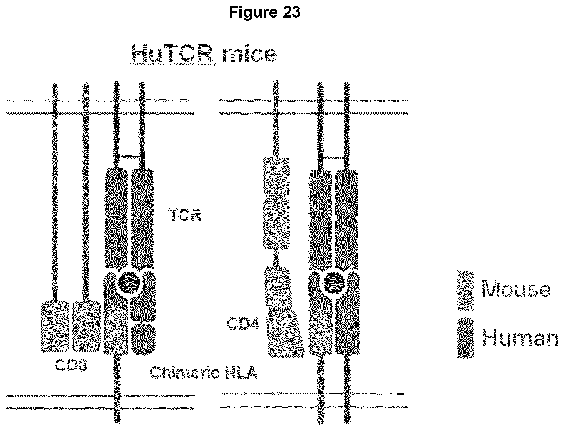

**Fig. 23** shows an extent of humanization in HuTCR mice of the present invention. Chimeric HLA in HuTCR mice facilitates CD8/CD4 co-receptor, which are extensively validated to function in human T cells.

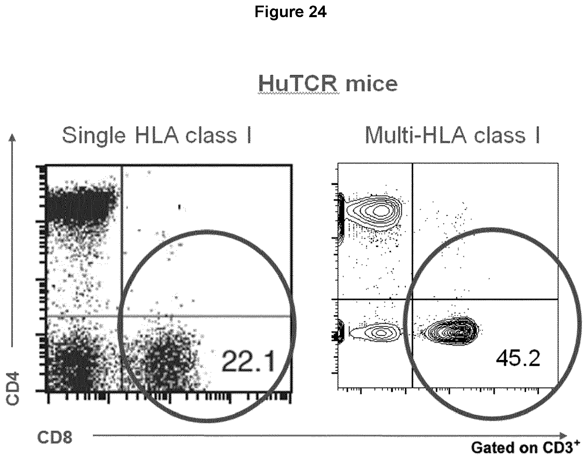

**Fig. 24** shows higher CD8 T cell numbers in HuTCR mice of the present invention compared to a single HLA class I mouse. High CD8 T cell numbers in the HuTCR mice of the present invention increase the probability to isolate TCRs against any known tumor targets and suggests that the TCR-HLA interaction is more optimal compared to that of a single HLA class I mouse.

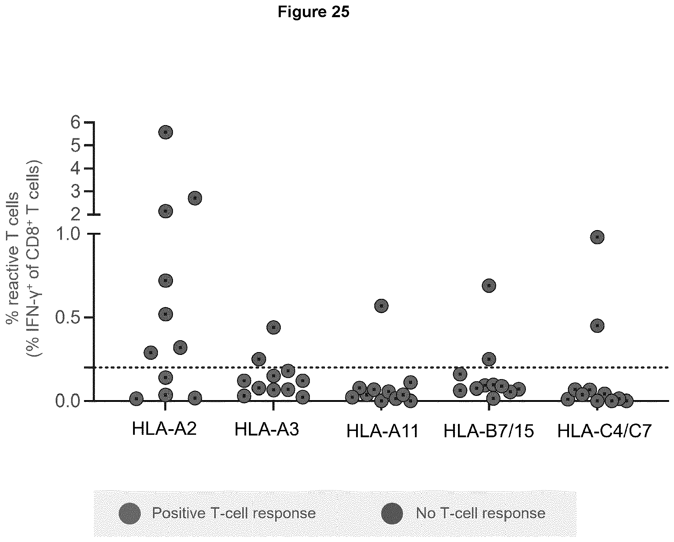

**Fig. 25** shows the active T-cell response in blood of multi-HLA HuTCR mice of the present invention that were immunized several times with DNA encoding full-length MAGE-A1 which was mutated to delete epitope 278-286. For all eleven mice used in this experiments the T cell response was examined individually for each of HLA-A*02:01, HLA-A*03:01, HLA-A*11:01, the HLA combination of HLA-B*07:02 and HLA-B*15:01 and for the HLA combination of HLA-C*04:01 and HLA-C*07:02. Mice having a positive T cell response are indicated by circles located above the dashed background line while mice having a negative T cell response are indicated by circles located below the dashed background line. For all tested HLA alleles or HLA allele combinations, mice were found with an active T-cell response directed towards a MAGE-A1-derived epitope in blood.

## DETAILED DESCRIPTION OF THE INVENTION

The present invention relates to a novel non-human mammal that comprises in its genome at least two or three human leukocyte antigen (HLA) class I alleles. Optionally, the at least two or at least three human HLA class I alleles comprise: at least one human HLA-A allele, at least one human HLA-B allele, and at least one human HLA-C allele. Such a transgenic non-human has been found to provide a plurality of advantages. First, it has been found herein that integrating into the genome of the non-human mammal such multiple human HLA class I alleles allows isolation of TCRs from a broad TCR repertoire, in particular of TCRs that are non-HLA-A2 restricted. For example, transgenic mice which carry two human HLA-A alleles, two HLA-B alleles, and two human HLA-C alleles (HLA-A*03:01, HLA-A*11:01, HLA-B*07:02, HLA-B*15:01, HLA-C*04:01, and HLA-C*07:02, see the Experimental Section) represent about 80 % of the US or European population with respect to their HLA class I genetic profile. In another example, transgenic mice which carry the following two human HLA-A alleles, two HLA-B alleles, and two human HLA-C alleles (HLA-A*24:02, HLA-A*33:03, HLA-B*40:01, HLA-B*46:01, HLA-C*01:02, and HLA-C*03:04) represent more than 80 % of an ethnic Chinese population with respect to their HLA class I genetic profile. It is noted here that such mice closely resemble the class I haplotype like in humans as every (human) individual bears six HLA class I alleles. Thus, as a second advantage by providing a non-human mammalian animal that carries in its genome multiple human HLA class I alleles, the invention allows one to specifically adapt the TCR repertoire to any given human (sub)population for which TCRs should be provided. As also illustrated by the further examples given herein for sets of HLA class I alleles that are representative for an ethnic Japanese or Indian population, the class I alleles that are to be integrated into the genome of the transgenic animals can be chosen according to their prevalence in the respective human population. Thus, the invention provides for the first time a "universal tool box" to mimic any human population of interest with respect to its TCR repertoire. In this context, also the following is noted. When multiple such non-human mammalian animals (say, for example, 5, 10 or even up to 20 transgenic mice) carrying the same set of, for example, six human HLA class I alleles are used for immunization, the present invention allows, as a third advantage, to represent/mimic a chosen human (sub)population even closer, thereby further increasing the chance to obtain suitable TCRs against a given antigen. In addition, it has also been found herein as a fourth advantage that a transgenic animal that carries multiple human HLA class I alleles allows to directly compare the strength of the T cell response that is associated with each of the human alleles against a given antigen (see Example 4), thereby also being able to identify a possible dominant T cell response. Thus, transgenic animals of the present invention allow to directly compare the strength of the T cell response while assessing the T cell response associated with multiple human HLA alleles at the same time. This "multiplexing" is a further advantage of the present invention. Finally, as a further advantage, a non-human transgenic animal as described herein can be used for discovery of novel epitopes (also HLA-A2 restricted epitopes) that have not yet been identified or which have theoretically been predicted but against which TCRs could not be experimentally raised so far (see Example 2 of the present application).

It is noted in this context that the illustrative experimental example of such a transgenic animal of the invention, the mouse termed "ABab.I mouse" (herein also be referred to as "multi-HLA mouse" or "HuTCR mouse") that comprises six (additional) human HLA alleles as a single haplotype, was developed starting from ABabDII mice background (Li et al., 2010, Nature Medicine, Vol. 16, No. 9 (2010), pages 1029-1034) that carry a single human HLA-A2 allele. So, this "multi-HLA-mouse" diversifies the HLA gene set and allows for epitope discovery and a broader TCR repertoire for non-HLA-A2 restricted TCR isolation. For this mouse, *in silico* screening for putative neoepitopes predicted to bind high-ranked HLA alleles selected the six alleles in ABab.I mice. In the course of the present invention, the *in silico* screening predicted 23 and 152 high-affinity TSAs (IC50 < 50 nM) from recurrent point mutations (n=266) that bound HLA-A2, and other frequent class I alleles (n=18), respectively. Specifically, epitope immunogenicity was tested in 4 out of 23 HLA-A2-binders in ABabDII mice. Only 1 out of 4 immunized epitopes elicited CD8+ T cell (CTL) responses. However, this was confirmed to be not endogenously processed when tested with TCRs raised from ABabDII mice. However, this form of reverse immunology is very laborious. A previous study on identifying epitopes from the vaccinia virus by Assarsson et al. in 2007 aligns with the *in silico* screen predicted data generated and examined in the course of the present invention. Thus, prediction algorithms offer a low probability to select immunogenic epitopes. Therefore, in the course of the present invention the ABab.I mouse model with a novel set of chimeric class I fusion alleles, namely HLA-A*03:01, A*11:01, B*07:02, B*15:01, C*04:01, and C*07:02, was developed using PiggyBac transposon strategy. The introduction of these six HLA alleles as a single genotype resembling a natural human situation broadened the TCR repertoire four-fold by enriching the peripheral pool with five times more unique and rarer V(D)J-TCRβ clonotypes than ABabDII mice. Ultimately, ABab.I mice of the present invention can be used as a versatile *in vivo* model system for epitope discovery, besides being a valuable tool to identify novel TCRs against high HLA-affinity tumour-specific antigens ($IC_{50}$ < 50 nM) derived from recurrent somatic point mutations.

**Definitions**

As used herein, the term "mammal" may refer to are a group of vertebrate animals that and characterized by the presence of mammary glands which in females produce milk for feeding (nursing) their young.

As used herein, the term "non-human mammal" may refer to any mammal that is not a human. Non-limiting examples of non-human mammal of the present invention include but are not limited to: rodents (e.g., mice or rats) dogs, felids, primates, rabbits, pigs, and ruminants (e.g., rodent, a dog, a felidae, a primate, a rabbit, a pig and a ruminant). Non-limiting examples of "rodents" are mice, rats, squirrels, chipmunks, gophers, porcupines, beavers, hamsters, gerbils, guinea pigs, degus, chinchillas, prairie dogs, and groundhogs. Non-limiting examples of "dogs" include members of the subspecies canis lupus familiaris as well as wolves, foxes, jackals, and coyotes. Non-limiting examples of "felides" include members of the two subfamilies: the pantherinae, including lions, tigers, jaguars and leopards and the felinae, including cougars, cheetahs, servals, lynxes, caracals, ocelots and domestic cats. The term "primates", as used herein, refers to all monkeys including for example cercopithecoid (old world monkey) or platyrrhine (new world monkey) as well as lemurs, tarsiers, apes and marmosets (Callithrix jacchus). Further exemplary non-human mammal of the present invention is the transgenic AB*ab*.I mouse generated in the course of the present invention, which can also be interchangeably referred to as "multi-HLA mouse" or "HuTCR mouse" elsewhere herein.

As used herein, the term "genome" may refer to genetic material of an organism (e.g., non-human mammal, e.g., mouse genome). For example, in the present context the term "genome" may refer to one haploid set of chromosomes with the genes they contain.

As used herein, the term "human leukocyte antigen" or "HLA" may refer to any of various (polymorphic) proteins that are encoded by genes of the major histocompatibility complex in humans and are found on the surface of many cell types (such as white blood cells). HLA class I alleles include HLA-A, HLA-B and HLA-C alleles (http://hla.alleles.org/genes/index.html). The human major histocompatibility complex (MHC) is divided into 3 regions on chromosome 6p21.3: class II (centromeric), class III, and class I (telomeric), with extended class I and class II regions on either side.

As used herein, the term "major histocompatibility complexes" or "MHC" may refer to a group of genes in mammals that code for cell-surface polymorphic glycoprotein molecules which display antigenic peptide fragments for T cell recognition and aid in the ability of the immune system to determine self from nonself. The MHC encodes highly polymorphic proteins, many of which are associated with the immune system. The products of classical polymorphic class I genes, human leukocyte antigen-A (HLA-A), HLA-B, and HLA-C, interact with T-cell receptor (TCR) molecules, as well as killer immunoglobulin-like receptors (KIRs) expressed on natural killer cells and some T cells.

As used herein, the term "allele" may refer to any of the alternative forms of a gene (e.g., human HLA gene) that may occur at a given locus. Each HLA allele name has a unique number corresponding to up to four sets of digits separated by colons. The length of the allele designation is dependent on the sequence of the allele and that of its nearest relative. Typically, an HLA allele name comprises the following elements: "HLA" prefix, hyphen ("-") used to separate gene name from HLA prefix, gene name (e.g., "A", "B", "C"), separator "*", field 1 depicting allele group (e.g., 03, 11, 01, 26, 24, 32, 02, 07, 15, 58, 40, 35, 08, 04, 07, 16, 03, 07 or 06 etc.), field separator ":", field 2 depicting specific HLA protein (e.g., 01 or 02 etc.). Exemplary human class I HLA genes of the present invention include HLA-A (Class I α-chain), HLA-B (Class I α-chain) and HLA-C (Class I α-chain). Further human class I HLA alleles of the present invention include HLA-E, HLA-F, HLA-G alleles as well as pseudogenes HLA-H, HLA-J, HLA-K, HLA-L, HLA-N, HLA-P, HLA-S, HLA-T, HLA-U, HLA-V, HLA-W and HLA-Y (http://hla.alleles.org/alleles/class1.html). Exemplary alleles of the present invention include any human class I and class II HLA alleles, e.g., selected from the group consisting of class I HLA-A, HLA-B and HLA-C alleles. Such alleles may be selected from the following exemplary human HLA allele groups: HLA-A*03, HLA-A*11, HLA-A*01, HLA-A*26, HLA-A*24, HLA-A*32 and HLA-A*02; HLA-B*07, HLA-B*15, HLA-B*58, HLA-B*40, HLA-B*35 and HLA-B*08; HLA-C*04, HLA-C*07, HLA-C*16, HLA-C*03, HLA-C*07 and HLA-C*06 alleles. Exemplary human HLA alleles of the present invention include but are not limited to: HLA-A*03:01, HLA-A*11:01, HLA-B*07:02, HLA-B*15:01, HLA-C*04:01, HLA-C*07:02 and HLA-A*02:01 alleles. According to http://hla.alleles.org/alleles/index.html there are currently 32330 HLA and related alleles described by the HLA nomenclature and included in the IPD-IMGT/HLA Database Release 3.46 (2021-10) Build 2d19adf (https://www.ebi.ac.uk/ipd/imgt/hla/). Any human class I HLA allele (e.g., HLA-A, HLA-B or HLA-C allele) is within the meaning of the present invention. Any human class II HLA allele (e.g., any allele selected from HLA-DRB1*13 group of alleles, e.g., HLA-DRB1*13:01) is within the meaning of the present invention. Exemplary class II HLA alleles of the present invention further include but are not limited to alleles of HLA-DRA, HLA-DRB1, HLA-DRB2-9, HLA-DQA1, HLA-DQA2, HLA-DQB1, HLA-DPA1, HLA-DPB1, HLA-DMA, HLA-DMB, HLA-DOA, HLA-DOB proteins (e.g., http://hla.alleles.org/alleles/class2.html). Further human class I and class II HLA alleles may also be queried at the IPD-IMGT/HLA Database as described above using "Allele Query Tool".

In this context, it is again noted that it is one advantage of the invention that is allows to mimic any given human (sub)population by integrating into the genome of the non-human animal any desired set of class I HLA alleles, for example, class I HLA alleles that have a high prevalence in the given population or geographic area.

In one such illustrative example, a transgenic animal of the invention comprises, integrated into its genome, the following HLA class I alleles: HLA-A*03:01, HLA-A*11:01, HLA-B*07:02, HLA-B*15:01, HLA-C*04:01, HLA-C*07:02 and HLA-A*02:01. As mentioned above, such a transgenic animal represents about 80 % of the US or European population with respect to their HLA class I genetic profile.

In another illustrative example a transgenic animal of the invention may include, integrated into its genome, a selection of HLA class I alleles that is representative for an (ethnic) Chinese population. Such a transgenic animal may include two or more of the following set of human HLA class I alleles: HLA-A*11:01 having 47.5 %-carrier frequency in the ethnic Chinese population (i.e., % of individuals that have the allele) and/or HLA-A*24:02 having 28.1 %-carrier frequency in an ethnic Chinese population and/or HLA-A*33:03 having 19.2 %-carrier frequency in an ethnic Chinese population and/or HLA-B*40:01 having 28.4%-carrier frequency in an ethnic Chinese population and/or HLA-B*46:01 having 25.1%-carrier frequency in an ethnic Chinese population and/or HLA-B*58:01 having 16.7%-carrier frequency in an ethnic Chinese population and/or HLA-B*15:02 having 12.5%-carrier frequency in an ethnic Chinese population and/or HLA-C*07:02 having 35.1 %-carrier frequency in an ethnic Chinese population and/or HLA-C*01:02 having 34.6%-carrier frequency in an ethnic Chinese population and/or HLA-C*03:04 having 21.9%-carrier frequency in an ethnic Chinese population may be selected. In this illustrative example, a transgenic animal may comprise two of the above-mentioned human HLA-A alleles, two of the above-mentioned human HLA-B alleles, and two of these human HLA-C alleles, for example, HLA-A*11:01, HLA-A*24:02, HLA-B*40:01, HLA-B*46:01, HLA-C*01:02 and HLA-C*03:04.

In another illustrative example a transgenic animal of the invention may comprise, integrated into its genome, a selection of HLA class I alleles that is representative for an (ethnic) Japanese population. Such a transgenic animal may include two or more of the following particular HLA class I alleles: HLA-A*24:02 (58.1% frequency in the Japanese population) and/or HLA-A*02:01 (27.4%) and/or HLA-B*52:01 (18.8%) and/or HLA-B*51:01 (17.0%) and/or HLA-B*35:01 (16.6%) and/or HLA-B*15:01 (14.8%) and/or HLA-B*40:02 (14.7%) and/or HLA-C*01:02 (31.6%) and/or HLA-C*03:03 (27.5%) and/or HLA-C*03:04 (23.8%). Also in this illustrative example, a transgenic animal may comprise two of the above-mentioned human HLA-A alleles, two of the above-mentioned human HLA-B alleles, and two of these human HLA-C alleles, for example, HLA-A*24:02, HLA-A*02:01, HLA-B*52:01, HLA-B*51:01, HLA-C*01:02 and HLA-C*03:03.

In a yet further illustrative example, a transgenic animal of the invention may comprise, integrated into its genome, a selection of HLA class I alleles that is representative for an (ethnic) Indian population. Such a transgenic animal may include two or more of the following HLA class I alleles: HLA-A*01:01 (28.5%) and/or HLA-A*11:01 (26.0%) and/or HLA-A*24:02 (25.4%) and/or HLA-B*40:06 (17.4%) and/or HLA-B*51:01 (14.4%) and/or HLA-B*52:01 (14.3%) and/or HLA-B*44:03 (14.3%) and/or HLA-C*06:02 (25.9%) and/or HLA-C*04:01 (25.4%) and/or HLA-C*07:02 (20.4%). Also in this illustrative example, a transgenic animal may comprise two of the above-mentioned human HLA-A alleles, two of the above-mentioned human HLA-B alleles, and two of these human HLA-C alleles, for example, HLA-A*01:01, HLA-A*24:02, HLA-B*40:06, HLA-B*51:01, HLA-C*06:02 and HLA-C*04:01.

As used herein, the term "functionally expressed" may refer to HLA allele/s that is/are expressed (e.g., on cell surface) and functioning as HLA protein/s, e.g., presenting peptides derived from endogenous protein (e.g., from inside the cell). The term "functionally expressed" may in particularly refer to HLA allele/s that is/are expressed (e.g., on cell surface, e.g., of the mammal of the present invention) and functioning as HLA protein/s, e.g., presenting peptides derived from endogenous protein (e.g., from inside the cell, e.g., of the mammal of the present invention) in that the corresponding MHC I polypeptides are expressed on the surface of cells, e.g., of the mammal of the present invention, and present MHC antigens to which the non-human mammal providing an antigen-specific CD8+ T cell response and, optionally, providing an antigen-specific CD4+ T cell response.

As used herein, the term "monocistron" may refer to a nucleic acid (e.g., RNA, e.g., fully processed) that codes for a single protein (e.g., HLA allele).

As used herein, the term "promoter" may refer to a regulatory region of DNA (e.g., located upstream of a gene), providing a control point for regulated gene transcription. One exemplary promoter of the present invention is a mammalian H-2Db promoter. Another exemplary promoter of the present invention is a viral CMV promoter. Other suitable promoters that can drive MHC I transgene expression and can be used herein include, but are not limited, the H-2Kb promoter (see Kimura et al, "Detailed analysis of the mouse H-2Kb promoter: enhancer-like sequences and their role in the regulation of class I gene expression Cell 1986 Jan 31;44(2):261-72), the pROSA-26 promoter (see Zambrowicz et al 'Disruption of Overlapping Transcripts in the Rosa Beta Geo 26 Gene Trap Strain Leads to Widespread Expression of Beta-Galactosidase in Mouse Embryos and Hematopoietic Cells', Proc Natl Acad Sci U S A, 94 (1997), 3789-94), the viral SV40 promoter or the mouse β2m promoter.

As used herein, the term "2A peptide linker" may refer to a class of 18 - 22 amino acid long peptides, which can induce ribosomal skipping during translation of a protein in a cell. Exemplary non-limiting 2A peptide linker of the present invention is an E2A peptide linker derived from equine rhinitis A virus (e.g., SEQ ID NO: 229).

As used herein, the term "epitope" may refer to a part of an antigen that is recognized by the immune system, specifically by antibodies, B cells, or T cells. The epitope is the specific piece of the antigen to which an antibody binds.

In the present context, the term "antigen" may refer to any substance that may be specifically bound by components of the immune system. Only antigens that are capable of eliciting (or evoking or inducing) an immune response are considered immunogenic and are called "immunogens".

The term "polypeptide" may be equally used herein with the term "protein". Proteins (including fragments thereof, preferably biologically active fragments, and peptides, usually having less than 30 amino acids, e.g., up to 10 or more amino acids, e.g., 4, 5, 6, 7, 8, 9, 10, 11, 12, 13, 14, 15, 16, 17, 18, 19, 20, 25 or 30 amino acids) comprise one or more amino acids coupled to each other via a covalent peptide bond (resulting in a chain of amino acids). The term "polypeptide" as used herein describes a group of molecules, which, for example, consist of more than 30 amino acids. Polypeptides may further form multimers such as dimers, trimers and higher oligomers, i.e. consisting of more than one polypeptide molecule. Polypeptide molecules forming such dimers, trimers etc. may be identical or non-identical. The corresponding higher order structures of such multimers are, consequently, termed homo- or heterodimers, homo- or heterotrimers etc. An example for a heteromultimer is an antibody molecule, which, in its naturally occurring form, consists of two identical light polypeptide chains and two identical heavy polypeptide chains. The terms "polypeptide" and "protein" also refer to naturally modified polypeptides/proteins wherein the modification is affected e.g. by post-translational modifications like glycosylation, acetylation, phosphorylation and the like. Such modifications are well known in the art.

The term "amino acid" or "amino acid residue" typically refers to an amino acid having its art recognized definition such as an amino acid selected from the group consisting of: alanine (Ala or A); arginine (Arg or R); asparagine (Asn or N); aspartic acid (Asp or D); cysteine (Cys or C); glutamine (Gln or Q); glutamic acid (Glu or E); glycine (Gly or G); histidine (His or H); isoleucine (He or I): leucine (Leu or L); lysine (Lys or K); methionine (Met or M); phenylalanine (Phe or F); pro line (Pro or P); serine (Ser or S); threonine (Thr or T); tryptophan (Trp or W); tyrosine (Tyr or Y); and valine (Val or V), although modified, synthetic, or rare amino acids may be used as desired. Generally, amino acids can be grouped as having a nonpolar side chain (e.g., Ala, Cys, He, Leu, Met, Phe, Pro, Val); a negatively charged side chain (e.g., Asp, Glu); a positively charged sidechain (e.g., Arg, His, Lys); or an uncharged polar side chain (e.g., Asn, Cys, Gln, Gly, His, Met, Phe, Ser, Thr, Trp, and Tyr).

For ease of reference, polypeptide variants (or mutants) of the invention may be referred to by the use of the following nomenclature: original amino acid(s): position(s): substituted amino acid(s). According to this nomenclature, for instance the substitution of S (serine) for F (phenylalanine) in position 722 can be shown as "S722F".

Generally, as used herein, the terms "polynucleotide" and "nucleic acid" or "nucleic acid molecule" are to be construed synonymously. Generally, nucleic acid molecules may comprise *inter alia* DNA molecules, RNA molecules, oligonucleotide thiophosphates, substituted ribo-oligonucleotides or PNA molecules. Furthermore, the term "nucleic acid molecule" may refer to DNA or RNA or hybrids thereof or any modification thereof that is known in the art (see, e.g., US 5525711, US 471 1955, US 5792608 or EP 302175 for examples of modifications). The polynucleotide sequence may be single- or double-stranded, linear or circular, natural or synthetic, and without any size limitation. For instance, the polynucleotide sequence may be genomic DNA, cDNA, mRNA, antisense RNA, ribozymal RNA or a DNA encoding such RNAs or chimeroplasts (Gamper et al., Nucleic Acids Res (2000), 28 (21): 4332-4339). Said polynucleotide sequence may be in the form of a vector, plasmid or of viral DNA or RNA.

The term "nucleic acid construct" may refer to a nucleic acid molecule, either single- or double-stranded, which is isolated from a naturally occurring gene or is modified to contain segments of nucleic acids in a manner that would not otherwise exist in nature or which is synthetic, which comprises one or more control sequences.

The term "control sequences" may refer to nucleic acid sequences necessary for expression of a polynucleotide encoding HLA alleles of the present invention. Such control sequences may include, but are not limited to, a leader, polyadenylation signal, promoter and transcription terminator.

The term "expression" may refer any step involved in the production of HLA proteins of the present invention e.g., by the non-human mammal of the present invention, including, but not limited to, transcription, post-transcriptional modification, translation, post-translational modification.

The term "polyadenylation signal" (or "pA") may refer to a poly(A) tail that is added to an nucleic acid (e.g., RNA) at the end of transcription. Exemplary pAs of the present invention is bGH pA (bovine growth hormone polyadenylation signal) and full-length H-2 Db pA.

The term "expression vector" may refer to a linear or circular nucleic acid molecule (e.g., DNA) that comprises a polynucleotide encoding HLA alleles of the present invention and are operably linked to control sequences that provide for their expression.

The term "host cell" may refer to any cell type (e.g., a bacterial cell) that is susceptible to transformation, transfection, transduction, or the like with a nucleic acid construct or expression vector comprising HLA alleles of the present invention. The term "host cell" encompasses any progeny of a parent cell that is not identical to the parent cell due to mutations that occur during replication.

The term "oocyte", as used herein, may refer to the female germ cell involved in reproduction, i.e. the ovum or egg cell. In accordance with the present invention, the term "oocyte" comprises both oocytes before fertilisation as well as fertilised oocytes, which are also called zygotes. Thus, the oocyte before fertilisation comprises only maternal chromosomes, whereas an oocyte after fertilisation comprises both maternal and paternal chromosomes. After fertilisation, the oocyte remains in a double-haploid status for several hours, in mice for example for up to 18 hours after fertilisation. For example, the oocyte of the present invention can be a fertilised oocyte.

The term "fertilised oocyte", as used herein, may refer to an oocyte after fusion with the fertilizing sperm. For a period of many hours (such as up to 18 hours in mice) after fertilisation, the oocyte is in a double-haploid state, comprising one maternal haploid pronucleus and one paternal haploid pronucleus. After migration of the two pronuclei together, their membranes break down, and the two genomes condense into chromosomes, thereby reconstituting a diploid organism. This fertilised oocyte, also referred to as a one-cell zygote and also the 2- cell and 4-cell stage zygote, are also encompassed by the term "fertilised oocyte", as used herein.

The term "fragment" may refer to a polypeptide having one or more (e.g., several, e.g., up to 10-20) amino acids absent from the amino and/or carboxyl terminus of a mature polypeptide.

As used herein, the term "TCR" may refer to T Cell Receptor.

As used herein, the term "TCR" may refer to T Cell Receptor.

As used herein, the term "pMHC" may refer to peptide bound Major Histocompatibility Complex.

As used herein, the term "pHLA" may refer to peptide bound Human Leukocyte Antigen.

As used herein, the term "CDR" may refer to Complementarity Determining Region.

As used herein, the term "ATT" may refer to Adoptive Transfer Therapy.

As used herein, the term "TSA" may refer to Tumor-Specific Antigen.

As used herein, the term "TAA" may refer to Tumor-Associated Antigen.

As used herein, the term "BCR" may refer to B Cell Receptor.

As used herein, the term "PD-1" may refer to Programmed cell Death protein-1.

As used herein, the term "MAGE-1" may refer to Melanoma-associated Antigen-1.

As used herein, the term "NY-ESO-1" may refer to New York ESOphageal Squamous cell carcinoma-1.

As used herein, the term "APC" may refer to Antigen Presenting Cell.

As used herein, the term "mIL-2/15" may refer to murine InterLeukin-2/15.

**Adaptive arm of the immune system**

The immune system provides the body a protective barrier by fighting against foreign invasions like bacteria, viruses, and parasites using innate and adaptive defence mechanisms. Innate immunity is the body's non-specific first line of defence in the detection and destruction of pathogens. Toll-like receptors (TLRs) sense pathogenic agents and recruit immune cells to the site for elimination by creating an inflammatory milieu together with macrophage-induced phagocytosis. Dendritic cells (DCs) are professional antigen-presenting cells (APCs) which present endogenous and exogenous antigens to major histocompatibility molecules (MHC) I and II and act as a bridge in the recruitment of the adaptive immune system. Several proinflammatory cues with the activation of multiple pathways like nuclear factor-$\kappa$B (NF-$\kappa$B), interferon (IFN)-regulatory factor (IRF), and activator protein-1 (AP-1) recruit the cells of the adaptive immune system. Even though the innate immune mechanisms act promptly with proinflammatory cytokine release such as IFN-$\alpha$, -$\beta$ (type I), and -$\gamma$ (type II), cell-mediated adaptive immunity is the most effective arm of the immune system in eliminating pathogens. The adaptive arm of the immune system consists of two cell-mediated responses, T and B cell- mediated immunity. T and B lymphocytes differ in their specificity and mechanisms of action. B cells, precursors to antibody-secreting cells, recognize pathogenic antigens through B cell receptor (BCR) and support the recruitment of helper CD4$^+$ T cells through MHC class II proteins to the site of infections. Naive CD8$^+$ T cell activation happens through the classical MHC class I presentation pathway by antigen-presenting DCs. CD4$^+$ T cells independently play a crucial role in cytotoxic CD8$^+$ T cell activation by providing a potent signal to antigen- presenting DCs via CD40L-CD40 interaction. Furthermore, the CD4$^+$ T cells help memory formation of the cytotoxic CD8$^+$ T cells by direct interaction through the CD40 receptor.

Both CD8$^+$ and CD4$^+$ conventional naive T cells interact with its cognate antigens presented on polymorphic MHC class I and II proteins through $\alpha\beta$ T cell receptors (TCRs). The naive TCR repertoire formed in the thymus primarily determines the precursor frequencies and distribution of distinct T cell clones. Quantification of TCR repertoire might provide insights into the diversity of T cell-mediated responses that an organism can mount.

**T cell receptor (TCR) and its diverse repertoire shapes T cell-mediated immunity**

The $\alpha\beta$ TCRs potentially produce up to $10^{20}$ (theoretical) combinations by random yet abundant variable (V), diversity (D), and joining (J) recombination events in the thymus. TCR $\alpha\beta^+$ T cells undergo positive and negative selection, whereas thymocytes are positively selected for adequate self-MHC interaction, whereas negatively selected for too high affinity towards any self-peptide MHC (pMHC). About 7.5% of thymocytes produce thymic signalling, from which about 3-5% survive selection to populate the periphery as naive T cells. Such T cells with a diverse array of $\alpha\beta$ TCRs interact intrinsically to pMHC (with co-stimulation via CD3-$\gamma$, -$\delta$, -$\varepsilon$, -$\zeta$, CD4 and CD8 chains) shape the naive CD8$^+$/CD4$^+$ repertoire to encounter foreign cognate antigens for T cell-mediated responses. MHC reactivity to about 5-20% of the preselection pool and about 10% of the peripheral T cell pool explains its intrinsic affinity towards TCRs. Additional factors such as the complementarity determining region (CDR) 1 and CDR2 encoded by TCR V$_\alpha$ and V$_\beta$ (germline-encoded) further instruct the germline bias of TCRs' bias towards MHC.

Some exceptions exist in which CDR1 and 2 are not the major contact domains to MHCs. Crystallographic structures show unconventional diagonal topology onto MHC, in which CDR1 and 2 of the TCR V$\alpha$ and V$_\beta$ dock MHC class I's $\alpha$2 and $\alpha$1 helix or MHC II's $\beta$and $\alpha$ helix. In such interactions, hypervariable CDR3 loops of V$_\alpha$ and V$_\beta$ are the main docking points to peptide-bound MHC (pMHC). Although existing pieces of evidence have shown that TCR and MHC genes inherently coevolve to interact with each other, distinct antigenic peptides processed and presented through class I/II antigen presentation pathways stringently define potential T cell effector functions through their CDR3$\alpha$ and CDR3$\beta$ interaction.

Thus, abundantly recombined CDR3-V$_\alpha$ and -V$_\beta$ clonotypes form a diverse TCR repertoire that enables unique pMHC interactions driving T cell-mediated immunity.

**pMHC-I/HLA-I-restricted antigen presentation pathway**

The MHC glycoproteins, known to be human leukocyte antigen (HLA) in humans, processes antigens and presents 8-15 amino acid (aa) in case of class I or 12-25 aa (8-10 aa core motif) in case of class II peptides on its surface. Two antigen presentation pathways assist the surface presentation of processed epitopes, peptide-loaded HLA-I (pHLA-I) and pHLA-II. HLA class I present endogenously processed epitopes like somatic point mutant, virus-derived, and aberrant frameshift proteins on its $\alpha 2$ and $\alpha 1$ domains to cytotoxic CD8[+] T cells (CTLs). HLA class II alleles present exogenously engulfed and processed epitopes like extracellular matrix proteins from the stroma and shredded viral proteins on its $\beta 1$ and $\alpha 1$ helix to helper CD4 T cells (T$_H$ cells). Professional APCs can take up exogenous antigens into the endogenous HLA I pathway by cross-presentation. Co-receptor interaction of CD8 to $\alpha 3$ domain of HLA I and CD4 to $\beta 2$ helix of HLA II is critical for T cell function.

Cellular, viral, aberrantly mutated, and misfolded post-translational proteins in the cytoplasm undergo proteasomal degradation. After proteasomal processing, the fragmented peptides enter into the endoplasmic reticulum (ER) through peptide-loading complex (PLC) proteins such as transporter-associated protein (TAP) and the chaperone tapasin. ER aminopeptidase associated with antigen processing (ERAAP) protein trims the amino terminus of the ER- resident peptides before loading onto MHCs. Calreticulin (CRT), Erp57, and PLC load the processed peptides onto MHC/HLAs. pMHC-I/HLA-I complexes are released into early endosomes and then transit to the cell surface through late endosomal compartments for T cell recognition by CD8[+] T cells (CTLs).

**Tumour Immunology**

**Immune system and cancer**

Cancer is a widely recognized genetic disease since oncogenic mutations, chromosomal rearrangements, and aberrations in gene expression are the most common causes detected in nearly one million cases. Whole sequencing studies of primary lung tumour samples identified more than 50,000 point mutations (adjacent non-tumour tissue as controls), including some mutational hot-spots, for example, Gly[12] site in the RAS gene. Emerging evidence proved that all cancers evolve by accumulating a small fraction of driver mutations that provide fitness and large numbers of passenger mutations that do not offer growth advantage. For decades, research into understanding the nature of cancer focused primarily on tumour cells alone. Oncogenic driver mutations make cancers thrive but not independently without additional factors supporting growth. Studies have confirmed the heterogeneous nature of tumour tissue, which makes dynamic interactions with the surrounding microenvironment, often regulate cancer progression. Tumour microenvironment with fibroblasts, inflammatory cells, vascular endothelial cells, and the extracellular matrix comprises the so-called "stroma". It is described in the literature that a chronic inflammatory state in the stroma promotes cancer development accumulated with inflammatory cells. One example being the stromal fibroblasts, in which the abrogation of transforming growth factor-$\beta$ (TGF-$\beta$) signalling induced stomach and prostate tumours. Under inflammatory states of progressing tumours, similar to wound healing, fibroblasts, macrophages, and endothelial cells are involved actively in assisting new blood vessel formation (as a part of stroma) to nourish tumours; a process known as "neo-angiogenesis".

CD8[+] and CD4[+] T cells require tumour-restricted peptide-loaded HLA-I (pHLA-I) and pHLA-II recognition, respectively for tumour rejection. Though CD8[+] T cells (CTLs) can elicit cytotoxic activity solely in most cases, in some models, CD4[+] T cells reject tumours in the absence of CTLs. CTLs release cytokines such as interferon-$\gamma$ (IFN-$\gamma$) and tumour necrosis factor-a (TNF- $\alpha$) to reject tumours by attacking tumour stroma and in some cases release cytotoxic molecules like perforin for tumour elimination, especially in virus-transformed tumours. Studies from many analyzed models, T cells use IFN-$\gamma$ as the critical effector molecule for tumour rejection. In 2017, a report by Kammertoens *et al.* deduced the mechanism of IFN-$\gamma$ on the tumour stroma, specifically on endothelial cells. The T cells through IFN-$\gamma$ induction cause tumour ischemia showcasing a physiological state of blood vessel regression, thereby inhibiting neo-angiogenesis. Another topic that links the immune system and cancer is the "immune surveillance" concept; still, a controversial theme that requires more evidence. In 2002, a study by Dunn *et al.*, based on methylcholanthrene (MCA)-induced spontaneous tumour model claimed to have proved cancer immune surveillance of tumour rejection and growth control by T cells. However, follow-up studies disproved the surveillance concept of tumour rejection as T cell-mediated. The first report proposed several other reasons for altered tumour development, such as tissue repair at the MCA site, the difference in steady-state IFN-$\gamma$ concentrations between control versus experimental groups, biased IFN-$\gamma$ protection due to different housing conditions of animal facilities. Further, tumour development in such models was called into question because either chemical-induced chronic inflammation or opportunistic infection could have been the reason for spontaneous growth, which did not resemble a naturally growing tumour in humans. In 2005, Willimsky *et al.* developed a sporadic tumour model induced by a viral transgenic dormant oncogene (reflecting a physiological tumour) to study the influence of T cells recognizing tumour cells in the context of immune surveillance. This study proved that immunogenic sporadic tumours do not escape T cell recognition but rather avoid T cell destruction by inducing tolerance, thus proposing the tumour immune surveillance effect towards virus-associated cancers is possible. Besides SV40 model, EBV- and HPV-associated tumours could be monitored by T cells, whereas immune surveillance of non-viral tumours needs more solid experimental evidence. Moreover, cautious data interpretation is necessary for reports that show the concept of immune surveillance in non-viral-related cancers.

In summary, the effect of T cells in attacking tumour cells is evident. Irrespective of T cell immune surveillance and its control over growing tumours, treatment option based on TCR-engineered CD8[+] and CD4[+] T cells using adoptive T cell therapy could give the best possible chance for tumour rejection.

## Adoptive T cell therapy (ATT) for cancer

In the past, many immunologists have been focusing on targeting self-antigens that were not tumour-specific using therapeutic vaccinations, however, with notable exceptions. Therapeutic vaccines against tumour-associated shared antigens by active and passive cancer immunotherapy did not yield expected success due to established tolerance mechanisms, suppression, and tumour micro-environmental factors. In contrast to cancer vaccinations, adoptive T cell therapy (ATT) has the possibility of rejecting large established tumours, specifically TCR-engineered CTLs (FasL$^+$) releasing effector molecules such as IFN-$\gamma$, but not perforin, have the potential to destroy surrounding stroma with complete eradication efficiency. Other than TCR-modified CTLs, the use of chimeric antigen receptor (CAR) engaged T cells (CAR T cells) in non-solid tumour clinical studies have shown success. Early phase clinical studies with CAR T cells targeted CD19 surface antigen in haematological malignancies. Though initial results targeting surface antigens in liquid tumours invited attention, challenges still exist in achieving similar success by targeting solid tumours.

CAR T cells have several obstacles to overcome to show efficacy without causing toxicity in solid cancers. These include single-chain variable fragments (scFvs) on CAR T cells to target unique tumour-associated antigens (TAAs) on the tumour cell surface without antigen loss. Next, CAR-engaged T cells should migrate to distant tumour sites for efficient homing. Besides this, circumvention of the immunosuppressive tumour microenvironment by T cells poses additional difficulties. A clinical study targeting folate receptor-a in ovarian cancers reported limited T cell homing to solid tumour sites, which can be enhanced by co-expressing the chemokine receptor 2 (CXCR2) in CAR T cells towards Gro-$\alpha$ chemokine. Blockade of TGF-$\beta$ signalling in prostate-specific membrane antigen targeting CAR T cells by co-expression of dominant-negative RII receptor increased their ability to infiltrate tumour stroma. In another clinically relevant pleural mesothelioma study in mice, CD28 CAR T cells restored their effector function through co-transduction of a dominant-negative programmed death-1 (PD-1) receptor or > 60% knockdown of PD-1 expression by short hairpin RNAs (shRNAs).

Although lessons learned from clinical studies on improving antigen selection, T cell migration, homing, and efficacy could apply to both CAR-based and TCR-modified adoptive T cell therapy, the most crucial concern is always the "safety". TCR-modified T cells stand out in terms of safety because such T cells can precisely recognize endogenously processed antigens on tumour cells or stroma through HLA-I or HLA-II-restricted manner. In ATT, to avoid adverse autoimmune effects by reducing the chance of cross-reactivity against unintended self-targets, the use of TCR-modified T cells gives us a superior advantage of targeting tumour-specific antigens. A unique class of such truly tumour-specific antigens could arise from non-synonymous somatic point mutations.

## Somatic point mutations can yield tumour-specific neoantigens

Carcinogenesis is an evolving multistage process that develops through a series of genomic instability events. Instability in the genome could cause uncontrolled cell growth, invasion, and metastasis. Such genomic changes include gene amplifications, chromosomal translocations, deletions, and collectively any somatic mutation.

Most tumour cells produce and process antigenic peptides, and these are expressed either on the cell surface or released into the extracellular matrix, the stroma. Studies have reported that a wide variety of peptide antigens are associated or shared among several cancer types, such as colon and prostate cancer, malignant melanoma, renal, breast, and lung carcinoma. In all cancer entities, tumour antigens mostly fit into two categories, tumour-specific antigens (TSAs) and tumour-associated antigens (TAAs). TSAs are a class of antigens exclusively expressed on tumour cells that include all abnormal protein structures derived from somatic mutations that can be the primary cause for malignant transformation. On the other hand, TAAs are overexpressed aberrant protein-derived antigens expressed not only on tumour cells but also a subpopulation of normal cells.

With the latest advancements in the next-generation sequencing of whole-genome and exome (all protein-coding regions), mutation identification and screening for TSAs have become easier and are highly important. Such TSAs are considered ideal and truly tumour-specific (referred to as neoantigens) targets for adoptive T cell therapy (ATT). The nature of TSAs derived from mutations varies from cancer to cancer and from individual to individual due to inter and intratumoral heterogeneity, respectively. An adult solid tumour entity bears around 50 mutations during primary diagnosis, whereas paediatric cancers and leukaemia acquire fewer mutations (10 on average). UV-associated melanomas of the skin carry 100-200 mutations. However, genomically unstable colorectal cancers can contain more than 1000 mutations. Above all, most mutations that frequently alter a coding protein are non-synonymous somatic point mutations caused by a single amino acid substitution such as R175H and R248Q in the TP53 gene and G12V and G13D in the RAS gene.

Regardless of the total number of somatic mutations in a tumour or its recurrent occurrence of specific mutations (hot-spots), abnormal proteins produced from a driver mutation may or may not create an epitope and induce immunogenicity. Many factors contribute to creating an epitope, such as antigen expression level, proteasomal peptide processing, transport (e.g., Figure 1), and its binding affinity to MHC/HLA determine if a mutant peptide might turn out to be a suitable epitope. The probability for a non-synonymous somatic mutation to pass through these stages to generate novel tumour-specific antigens is low. Probability speculation proved to be true in a vaccinia virus study that reported only 1 out of 14 predicted epitopes are indeed immunogenic. In detail, of about 100 vaccinia-encoded epitopes predicted to bind HLA-A*02:01 (HLA-A2) with $IC_{50}$ < 100 nM, only 50% of epitopes elicited CD8$^+$ T cell responses, and from these, only 15% proven for endogenously processing. 11% elicited CTL responses out of the 15% of processed epitopes, which explains that 1 out of every 14 predicted epitopes creates an immunogenic epitope. Comparing the vaccinia virus to physiological cancer with somatic mutations, i.e., with similar probability, about 4-5 mutant epitopes per cancer could be an estimate that carries 50 mutations. Since every human individual bears six HLA alleles, one can expect 20-25 epitopes per person.

Immunodominance also plays a role among dominant versus recessive mutant epitopes. The recessive epitopes may be targetable only if the dominant epitope is lost. One factor to the lack of immunodominance of recessive epitopes could be its affinity towards HLA; the dominant epitope could show a strong affinity to HLA alleles. On the other hand, some HLA alleles show prediction to bind more epitopes than others making the estimation difficult. In addition to point mutations, frameshift mutations causing novel reading frames might increase the chance of potential epitopes generated per individual.

Supportively, in cancer patients, various forms of TSAs have been distinguished as targets for CD8$^+$ T cells and were also examined in pre-clinical trials. Ample evidence available reporting that the cytotoxic T lymphocytes can effectively recognize tumour antigens resulting from mutations in various forms of cancers such as colorectal, head, and neck cancers (CASP-8), melanoma (β-catenin), squamous cell lung carcinoma (NFYC), and leukaemia. With all the above collective understanding, somatic mutations can yield potential neoepitopes for ATT of cancer; if proven to be naturally processed and presented.

## Recurrent somatic driver mutations as targets for ATT of cancer

The adoptive transfer of tumour antigen-specific T cells is one of the most promising and advanced medicinal products (ATMPs) in the area of cancer immunotherapy. However, problems still exist with the appropriate selection of antigens for a powerful ATT.

Over the past two decades, several preclinical studies represented to be tumour-specific have led to the development of diverse biological agents that block or inhibit various molecular targets derived from genetic abnormalities in cancers. Targeted cancer therapies such as monoclonal antibodies (mAbs), small-molecule drugs (SMDs), and cancer vaccines used tumour-associated and self-antigens as their targets.

Due to the on-target attack of cells expressing self-antigens, these therapies exhibited high toxicity, frequent drug resistance, and relapse, thereby dampening the overall therapeutic benefit. Hence, a careful selection of suitable tumour antigens still seems to pose a crucial challenge for an efficacious ATT. Therefore, targeting recurrent somatic driver mutations (TSAs), using TCR-engaged T cells might become a more precise strategy when compared to SMDs and mAbs.

Anders *et al.* demonstrated that monospecific CD8$^+$ T cells restricted to a viral epitope (SV40-T antigen) could reject large established tumours completely ($\geq$ 500 mm$^3$). Such complete tumour rejection might be possible probably by the elimination of escape variants. Targeting driver mutations (ancestor in cancer cell evolution) that generate homogenously expressed epitopes could also avoid resulting antigen loss variants.

Not every mutation is a driver mutation and can create an epitope. Driver mutations give tumours a growth advantage. Concerning tumour heterogeneity, driver mutations can occur recurrently across (intertumoural) and between (intratumoral) tumours. Recurrent neoepitopes are often derivatives of driver mutations shared (ancestral mutations) by all cancer cells and are ideal target antigens that could not be lost. However, selection and validation of the most promising neoepitope candidates for ATT is a tedious task.

In recent times, *in silico*-based identification of neoantigens from tumour whole-genome or exome sequencing datasets seems to assist neoepitope selection. In 2019, our bioinformatics study published a prediction screen identifying and ranking the binding affinities of putative neoepitopes to the HLA complex using TCGA datasets. We used affinity prediction algorithms based on machine learning as an opportunity to devise *off-the-shelf* T cell therapies for subgroups of cancer patients who share recurrent neoepitopes. Immunogenicity validation, concerning efficient processing and presentation, is inevitable for all predicted recurrent mutant epitopes. For this, humanized TCR-HLA mouse models could be utilized as a valuable tool not only to verify the immunogenicity of predicted epitopes but to discover novel epitopes by full-length protein-encoding gene immunization.

**Humanized mammalian models**

The fact that recurrent neoepitopes are ideal targets with their homogeneous expression on all cancer cells contributed to emerging ways to isolate TCRs for ATT of cancers. Identification of neoantigen-specific TCRs is possible from humans or from transgenic mammals such as transgenic mice, rats or pigs, to name only a transgenic mammals.

In humans, TCR $\alpha$ and $\beta$chains can be isolated by *in vitro* priming from HLA-matched healthy individuals or immunized candidates with mutant peptides. Anyway, in the former case, the precursor TCR repertoire against neoepitopes in healthy donors is unaffected by central tolerance mechanisms; deletion of T cells that bind only the wild-type self-counterparts with high affinity. So, TCR isolation in principle is possible by autologous priming, although the percentage of neoantigen-specific T cells as shown in melanoma, gastrointestinal, lung, and ovarian cancer patients is low; only about 1.2% of mutations are spontaneously recognized. Further, the expression level of neoantigens in the periphery is a decisive factor in whether the T cells are deleted or became anergic.

In transgenic animals, as experimentally exemplified herein by transgenic mice, isolation of TCRs from a naive repertoire could work after multiple immunizations; to produce memory responses *in vivo.* Also, TCRs come from a tumour-free host, providing the possibility to select high-affinity T cells from a naive precursor pool that could undergo deletion in tumour-bearing human individuals due to chronic antigen stimulation.

**Transgenic HLA-I mice with human HLA antigen presentation**

One example of a tumour-free human HLA host is the HLA-A2/D$^b$ transgenic mouse model, which served as a model to monitor human immune responses for TCR isolation and epitope identification. Diverse chimeric HLA-D$^b$/K$^b$ models were employed to understand heterologous immunity between viruses compared to humans. Though chimeric MHC/HLA transgenic models existed for some time, questions remain concerning mouse TCRs education on a chimeric single human HLA allele, overall TCR repertoire, and certain $V_{\alpha\beta}$ specificities gained or lost during thymic selection. Furthermore, severe on-target toxicities combined with fatality were reported in cancer patients treated with an affinity matured (in CDR3$\alpha$ region) anti-MAGE-A3 TCR derived from HLA-A2 transgenic mice, which cross-reacted to other shared epitopes of MAGE-A9 and -A12 antigens. Since murine TCRs are not negatively thymus-selected on a patient's HLA alleles and the human proteome, thorough on- and off-toxicity evaluation is necessary on such mouse-derived TCRs that might cross-react with normal human proteins. Besides, partially humanized murine TCRs are indeed immunogenic when transferred to humans.

Hence, for scrupulous prediction and translation of murine-derived therapeutics into human systems, a comprehensive humanization of mouse models expressing diverse human HLAs with human TCR gene loci seems inevitable. While the latter, transgenic HLA-A2/K$^b$ mice with a complete human TCR gene locus for efficient isolation of optimal affinity TCRs have been generated and established (Li, L.-P. et al. Transgenic mice with a diverse human T cell antigen receptor repertoire. Nat. Med. 16, 1029 (2010)).

**Human TCR transgenic mice as an ideal tool for isolating TCRs for ATT of cancer**

The necessity for humanization triggered the development of novel humanized transgenic mice named AB*ab*DII. The AB*ab*DII mice have the complete human TCR gene loci, knocked out for murine TCR and MHC I gene loci, and it is transgenic for human class I allele HLA- A2 (Li, L.-P. et al. Transgenic mice with a diverse human T cell antigen receptor repertoire. Nat. Med. 16, 1029 (2010)). Thus, making the model a valuable tool for TCR isolation against TAAs, and TSAs including viral epitopes. Even though its advanced humanization yielded functional CD8$^+$ T cell populations for optimal affinity TCR isolation, the AB*ab*DII model is limited as a source of obtaining only HLA-A2-restricted TCRs. Therefore, it is indeed a bottleneck that AB*ab*DII mice are not available with other HLA alleles. So, this makes humanized TCR mice expressing other class I alleles a requirement for epitope discovery, which would benefit TCR isolation for a large number of non-HLA-A2 individuals.

**Aims and objectives**

One of the main goals was to broaden the allelic diversity to study the human TCR repertoire enrichment against a complete human HLA haplotype in mice. The PiggyBac transposon (or PB, a mobile genetic element that efficiently transposes between vectors and chromosomes via a "cut and paste" mechanism) strategy introduced six HLA alleles into transcriptionally active sites as a single haplotype as in humans (every individual bears six HLA class I alleles). Another aim was to ensure surface expression of the newly introduced HLA alleles to analyse their presentation capacity. Ultimately, the novel mouse model of the present invention could become an in vivo epitope discovery system for identifying a range of tumour antigens restricted towards highly frequent (allele-wise) HLA genes, thereby isolating T cell receptors for ATT of cancer.

The ABab.I transgenic mouse model of the present invention is a novel tool for isolating TCRs restricted towards a broad spectrum of HLAs

Up to now, the ABabDII transgenic mouse model described in Li et al serves as a valuable system to isolate human TCRs, as it comprises the whole human T cell receptor gene loci. However, the model exhibits a limitation of isolation of only HLA-A2-restricted TCRs. To target diverse non-HLA-A2-binding antigens, and in parallel, broaden the HLA repertoire of the existing system for epitope discovery, it is necessary to include multiple human ABabDII mice. In this regard, one of the aims of the present invention was to create the "ABab.I transgenic mice" by introducing a novel set of chimeric class I fusion alleles (HLA-A*03:01, A*11:01, B*07:02, B*15:01, C*04:01, C*07:02) as a single genotype into the existing ABabDII mice (HLA-A2). Furthermore, the use of ABab.I mice was to discover previously not described immunogenic epitopes selected for presentation on any of the six HLA alleles post-processing.

In the course of the present invention, the goal was to develop a new transgenic AB*ab*.I mouse line as an *in vivo* model system for epitope discovery, besides also being a valuable tool to identify novel TCRs against high HLA-affinity tumour-specific antigens ($IC_{50}$ < 50 nM) derived from recurrent somatic point mutations.

In one embodiment, the invention provides a non-human mammal (e.g., transgenic ABab.I mouse of the present invention) comprising in its genome at least two or at least three human leukocyte antigen (HLA) class I alleles, wherein said at least two or at least three human HLA alleles are functionally expressed in that the corresponding MHC I polypeptides are expressed on the surface of cells of said mammal and present MHC antigens to which the non-human mammal providing an antigen-specific CD8+ T cell response and wherein said at least two or at least three human HLA class I alleles comprise: a) at least one human HLA-A allele; and/or b) at least one human HLA-B allele; and/or c) at least one human HLA-C allele; and/or d) (a) and (b); and/or e) (a) and (c); and/or f)(b) and (c); and/or g)(a), (b) and (c). For the sake of clarity, the possibility g) means that the transgenic mammal comprises in its genome at least one human HLA-A allele, at least one human HLA-B allele, and at least one human HLA-C allele.

In a further embodiment, the invention provides a nucleic acid construct comprising a nucleic acid encoding at least two or at least three human HLA class I alleles, wherein said nucleic acid construct is capable of functionally expressing said at least two or at least three human HLA alleles in that the corresponding MHC I polypeptides are expressed on the surface of cells of said mammal and present MHC antigens to which the non-human mammal provides an antigen-specific CD8+ T cell response are functionally expressed and optionally wherein said at least two or at least three human HLA class I alleles comprise: a) at least one human HLA-A allele; and/or b) at least one human HLA-B allele; and/or c) at least one human HLA-C allele; and/or d) (a) and (b); and/or e) (a) and (c); and/or f)(b) and (c); and/or g)(a), (b) and (c). For the sake of clarity, the possibility g) means that the nucleic acid construct comprises a nucleic acid encoding transgenic mammal comprises in its genome at least one human HLA-A allele, at least one human HLA-B allele, and at least one human HLA-C allele.

In a further embodiment, the invention provides an expression vector comprising a nucleic acid construct as described herein.

In a further embodiment, the invention provides a host cell (e.g., an isolated and/or recombinant host cell) comprising a nucleic acid construct as described herein and/or an expression vector as described herein.

In a further embodiment, the invention provides a method of modifying endogenous HLA alleles of a non-human mammal, the method comprising: transducing and/or transplanting a non-human mammal as described herein with a nucleic acid construct and/or an expression vector as described herein.

In a further embodiment, the invention provides a method of producing a non-human, mammalian oocyte carrying a modified target sequence in its genome, the method comprising the steps of introducing into a non-human, mammalian oocyte a nucleic acid construct and/or an expression vector as described herein.

In a further embodiment, the invention provides a method of producing a non-human mammal carrying a modified target sequence in its genome, the method comprising: (a) producing an oocyte as recited described herein; (b) analysing the offspring delivered by the non-human female host to which the oocyte obtained in (a) has been transferred for the presence of the modification.

In a further embodiment, the invention provides a non-human mammal produced and/or modified by a method of producing a non-human mammal carrying a modified target sequence in its genome as described herein. In a further embodiment, the invention provides a method of generating one or more T cell receptors that are capable of binding to an antigen of interest, the method comprising administering to a non-human animal as described herein an antigen of interest.

In a further embodiment, the invention provides a T cell receptor obtained by or obtainable by a respective method as described herein.

In a further embodiment, the invention provides a method of identifying an epitope to which an immune response can be elicited, the method comprising administering to a non-human animal as described herein an antigen of interest suspected to contain an epitope to which an immune response can be elicited.

In a further embodiment, the invention provides an epitope to which identifying an epitope to which an immune response can be elicited, the epitope being identified by a respective method as described herein.

In a further embodiment, the invention provides the use of a non-human animal as described herein for generating on or more T cell receptors that are capable of binding to an antigen of interest.

In a further embodiment, the invention provides the use of a non-human animal as described herein for identifying an epitope to which an immune response can be elicited.

In a further embodiment, the invention provides the use of a non-human animal as described herein for identifying one or more T cell receptors that are capable of binding to an antigen of interest.

The present invention as described herein, in particular the non-human mammal of the present invention (i.e., transgenic AB*ab*.I mouse of the present invention, which can also be interchangeably referred to as "multi-HLA mouse" or "HuTCR mouse" herein) and TCR isolation methods based thereon, have/provide one or more of the following advantages for TCR isolation:

- a broader TCR diversity and therefore provides for higher chances of success in isolating TCRs against any known tumour targets;

- a broader HLA coverage and therefore the non-human mammal of the present invention (which may also be referred to as multi-HLA mouse) covers about 80% of the EU/US population;

- improved and/or more extensive methods on TCR generation based thereon;

- PoC to generate high-affinity TCRs.

Furthermore, in some aspects/embodiments of the present invention the non-human mammal of the present invention (i.e., transgenic AB*ab*.I mouse of the present invention, which can also be interchangeably referred to as "multi-HLA mouse" or "HuTCR mouse" herein) and/or methods based thereon comprises/provides one or more of the characteristics/advantages:

- The extent of humanization (e.g., in mice) comprises HLA and TCR;

- The CD8 and CD4 interaction which is facilitated by chimeric HLAs as described herein; For example, chimeric HLA in the non-human mammal of the present invention facilitates CD8/CD4 co-receptor interaction. The non-human mammal of the present invention is extensively validated to function in human T cells;

- Gene editing (e.g., via the HLA alleles integration as described herein) which is based on random integration;

- The non-human mammal of the present invention and methods based thereon may be collectively referred to as a "platform" of the present invention, which has been already validated for TCR isolation for tumour target/s in the course of the present invention;

- Broad HLA coverage, e.g., 7 class I alleles (representing about 80% coverage in EU/US) and 1 class II allele as described herein;

- Higher (about 40%) T cell numbers (e.g., CD8), e.g., compared to a single HLA class I allele mouse;

- Broader and/or higher TCR diversity (e.g., very broad TCR repertoire);

- Potential for isolating high-affinity TCRs, e.g., which has been demonstrated for multiple tumour targets in the course of the present invention;

- TCR genes that are fully human.

The present invention is also characterized by the following items (e.g., embodiments):

1. A non-human mammal comprising in its genome at least two (e.g., at least three, at least four, at least five, at least six or at least seven etc.) human leukocyte antigen (HLA) class I alleles, wherein said at least two human HLA alleles are functionally expressed in that the corresponding MHC I polypeptides are expressed on the surface of cells of said mammal and present MHC antigens to which the non-human mammal provides an antigen-specific CD8+ T cell response and optionally wherein said at least two human HLA class I alleles comprise: **a)** at least one (e.g., at least two or at least three) human HLA-A allele; and/or **b)** at least one (e.g., at least two) human HLA-B allele; **c)** and/or at least one (e.g., at least two) human HLA-C allele; **d)** (a) and (b); and/or **e)** (a) and (c); and/or **f)** (b) and (c); and/or **g)** (a), (b) and (c).

2. The non-human mammal according to any one of the preceding items, wherein the mammal further comprises in its genome at least one human HLA class II allele.

3. The non-human mammal according to any one of the preceding items, wherein the mammal comprises in its genome at least 4, 5, 6 or 7 human HLA class I alleles.

4. The non-human mammal according to any one of the preceding items, wherein the mammal comprises in its genome:

    a) at least two different human HLA-A alleles, preferably at least three different human HLA-A alleles; and/or
    b) at least two different human HLA-B alleles; and/or
    c) at least two different human HLA-C alleles.

5. The non-human mammal according to any one of the preceding items, wherein the at least two human HLA class I alleles comprise one or more HLA-A class I alleles selected from the group consisting of (Europe/US): HLA-A*03 (e.g., HLA-A*03:01), HLA-A*11 (e.g., HLA-A*11:01), HLA-A*01 (e.g., HLA-A*01:01), HLA-A*26 (e.g., HLA-A*26:01), HLA-A*24 (e.g., HLA-A*24:02), HLA-A*32 (e.g., HLA-A*32:01) and HLA-A*02 (e.g., HLA-A*02:01), preferably the three HLA-A class I alleles are selected from human HLA allele groups consisting of: HLA-A*03, HLA-A*11 and HLA-A*02.

6. The non-human mammal according to any one of the preceding items, wherein the at least two human HLA class I alleles comprise one or more HLA-A class I alleles selected from the group consisting of (China): HLA-A*11 (e.g., HLA-A*11:01), HLA-A*24 (e.g., HLA-A*24:02) and HLA-A*33 (e.g., HLA-A*33:03), preferably HLA-A*24 (e.g., HLA-A*24:02) and HLA-A*33 (e.g., HLA-A*33:03).

7. The non-human mammal according to any one of the preceding items, wherein the at least two human HLA class I alleles comprise one or more HLA-A class I alleles selected from the group consisting of (Japan): HLA-A*24 (e.g., HLA-A*24:02), HLA-A*02 (e.g., HLA-A*02:01), HLA-A*11 (e.g., HLA-A*11:01) and HLA-A*31 (e.g., HLA-A*31:01), preferably HLA-A*24 (e.g., HLA-A*24:02) and HLA-A*31 (e.g., HLA-A*31:01).

8. The non-human mammal according to any one of the preceding items, wherein the at least two human HLA class I alleles comprise one or more HLA-A class I alleles selected from the group consisting of (India): HLA-A*01 (e.g., HLA-A*01:01), HLA-A*11 (e.g., HLA-A*11:01) and HLA-A*24 (e.g., HLA-A*24:02), preferably HLA-A*01 (e.g., HLA-A*01:01) and HLA-A*24 (e.g., HLA-A*24:02).

9. The non-human mammal according to any one of the preceding items, wherein the at least three HLA-A class I alleles are selected from human HLA allele groups consisting of: HLA-A*03 (e.g., HLA-A*03:01), HLA-A*11 (e.g., HLA-A*11:01), HLA-A*01 (e.g., HLA-A*01:01), HLA-A*26 (e.g., HLA-A*26:01), HLA-A*24 (e.g., HLA-A*24:02), HLA-A*32 (e.g., HLA-A*32:01) and HLA-A*02 (e.g., HLA-A*02:01), preferably the three HLA-A class I alleles are selected from human HLA allele groups consisting of: HLA-A*03, HLA-A*11 and HLA-A*02.

10. The non-human mammal according to any one of the preceding items, wherein the at least two HLA class I alleles comprise one or more HLA-B class I alleles selected from the group consisting of (Europe/US): HLA-B*07 (e.g., HLA-B*07:02), HLA-B*15 (e.g., HLA-B*15:01), HLA-B*58 (e.g., HLA-B*58:01), HLA-B*40 (e.g., HLA-B*40:01), HLA-B*35 (e.g., HLA-B*35:01) and HLA-B*08 (e.g., HLA-B*08:01), preferably the at least two HLA-B class I alleles are selected from human HLA allele groups consisting of: HLA-B*07 and HLA-B*15.

11. The non-human mammal according to any one of the preceding items, wherein the at least two HLA class I alleles comprise one or more HLA-B class I alleles selected from the group consisting of (China): HLA-B*40 (e.g., HLA-B*40:01), HLA-B*46 (e.g., HLA-B*46:01), HLA-B*58 (e.g., HLA-B*58:01) and HLA-B*15 (e.g., HLA-B*15:02), preferably HLA-B*40 (e.g., HLA-B*40:01) and HLA-B*46 (e.g., HLA-B*46:01).

12. The non-human mammal according to any one of the preceding items, wherein the at least two HLA class I alleles comprise one or more HLA-B class I alleles selected from the group consisting of (Japan): HLA-B*52 (e.g.,

HLA-B*52:01), HLA-B*51 (e.g., HLA-B*51:01), HLA-B*35 (e.g., HLA-B*35:01), HLA-B*15 (e.g., HLA-B*15:01) and HLA-B*40 (e.g., HLA-B*40:02), preferably HLA-B*52 (e.g., HLA-B*52:01) and HLA-B*51 (e.g., HLA-B*51:01).

13. The non-human mammal according to any one of the preceding items, wherein the at least two HLA class I alleles comprise one or more HLA-B class I alleles selected from the group consisting of (India): HLA-B*40 (e.g., HLA-B*40:06), HLA-B*51 (e.g., HLA-B*51:01), HLA-B*52 (e.g., HLA-B*52:01) and HLA-B*44 (e.g., HLA-B*44:03), preferably HLA-B*40 (e.g., HLA-B*40:06) and HLA-B*51 (e.g., HLA-B*51:01).

14. The non-human mammal according to any one of the preceding items, wherein the at least two HLA-B class I alleles are selected from human HLA allele groups consisting of: HLA-B*07 (e.g., HLA-B*07:02), HLA-B*15 (e.g., HLA-B*15:01), HLA-B*58 (e.g., HLA-B*58:01), HLA-B*40 (e.g., HLA-B*40:01), HLA-B*35 (e.g., HLA-B*35:01) and HLA-B*08 (e.g., HLA-B*08:01), preferably the at least two HLA-B class I alleles are selected from human HLA allele groups consisting of: HLA-B*07 and HLA-B*15.

15. The non-human mammal according to any one of the preceding items, wherein the at least two HLA class I alleles comprise one or more HLA-C class I alleles selected from the group consisting of (Europe/US): HLA-C*04 (e.g., HLA-C*04:01), HLA-C*07 (e.g., HLA-C*07:02), HLA-C*16 (e.g., HLA-C*16:01), HLA-C*03 (e.g., HLA-C*03:04), HLA-C*07 (e.g., HLA-C*07:01), and HLA-C*06 (e.g., HLA-C*06:02), preferably the two HLA-C class I alleles are selected form human HLA allele groups consisting of: HLA-C*04 and HLA-C*07.

16. The non-human mammal according to any one of the preceding items, wherein the at least two HLA class I alleles comprise one or more HLA-C class I alleles selected from the group consisting of (China): HLA-C*07 (e.g., HLA-C*07:02), HLA-C*01 (e.g., HLA-C*01:02) and HLA-C*03 (e.g., HLA-C*03:04), preferably HLA-C*01 (e.g., HLA-C*01:02) and HLA-C*03 (e.g., HLA-C*03:04).

17. The non-human mammal according to any one of the preceding items, wherein the at least two HLA class I alleles comprise one or more HLA-C class I alleles selected from the group consisting of (Japan): HLA-C*01 (e.g., HLA-C*01:02), HLA-C*03 (e.g., HLA-C*03:03) and HLA-C*03 (e.g., HLA-C*03:04), preferably HLA-C*01 (e.g., HLA-C*01:02) and HLA-C*03 (e.g., HLA-C*03:03).

18. The non-human mammal according to any one of the preceding items, wherein the at least two HLA class I alleles comprise one or more HLA-C class I alleles selected from the group consisting of (India): HLA-C*06 (e.g., HLA-C*06:02), HLA-C*04 (e.g., HLA-C*04:01), HLA-C*07 (e.g., HLA-C*07:02) and HLA-C*15 (e.g., HLA-C*15:02), preferably HLA-C*06 (e.g., HLA-C*06:02) and HLA-C*15 (e.g., HLA-C*15:02).

19. The non-human mammal according to any one of the preceding items, wherein the at least two HLA-C class I alleles are selected form human HLA allele groups consisting of: HLA-C*04 (e.g., HLA-C*04:01), HLA-C*07 (e.g., HLA-C*07:02), HLA-C*16 (e.g., HLA-C*16:01), HLA-C*03 (e.g., HLA-C*03:04), HLA-C*07 (e.g., HLA-C*07:01), and HLA-C*06 (e.g., HLA-C*06:02), preferably the two HLA-C class I alleles are selected form human HLA allele groups consisting of: HLA-C*04 and HLA-C*07.

20. The non-human mammal according to any one of the preceding items, wherein the mammal comprises in its genome six or seven human leukocyte antigen (HLA) class I alleles selected from human HLA allele groups comprising or consisting of:

a) HLA-A*03,
b) HLA-A*11,
c) HLA-B*07,
d) HLA-B*15,
e) HLA-C*04,
f) HLA-C*07 and
g) HLA-A*02.

21. The non-human mammal according to any one of the preceding items, wherein the seven human HLA alleles comprise or consist of:

a) HLA-A*03:01,
b) HLA-A*11:01,

c) HLA-B*07:02,
d) HLA-B*15:01,
e) HLA-C*04:01,
f) HLA-C*07:02 and
g) HLA-A*02:01.

22. The non-human mammal according to any one of the preceding items, wherein the at least two human HLA alleles are functionally expressed in that the corresponding MHC I polypeptide is expressed on the surface of cells of the mammal.

23. The non-human mammal according to any one of the preceding items, wherein the corresponding MHC I polypeptide is expressed on the surface of peripheral blood cells of the mammal.

24. The non-human mammal according to any one of the preceding items, wherein the corresponding MHC I polypeptide expressed on the surface of cells of the mammal present MHC present antigens to which the non-human mammal provides an antigen-specific CD8+ T cell response (e.g., wherein said antigen specific CD8+ T cell response is measured by any suitable means, e.g., FACS).

25. The non-human mammal according to any one of the preceding items, wherein the at least two human HLA alleles encode chimeric human/non-human mammal MHC molecules.

26. The non-human mammal according to any one of the preceding items, wherein the at least two HLA alleles encode a chimeric human/non-human mammal MHC I polypeptide, wherein the human portion of the chimeric polypeptide comprises $\alpha 1$ and $\alpha 2$ domains of a human MHC I polypeptide, wherein the non-human mammal portion of the chimeric polypeptide comprises the $\alpha 3$ domain, the transmembrane and the cytoplasmic domains of an endogenous non-human mammal MHC I polypeptide and wherein the non-human mammal expresses the chimeric human/non-human mammal MHC I polypeptide.

27. The non-human mammal according to any one of the preceding items, wherein the at least two HLA alleles encode a chimeric human/non-human MHC I polypeptide, wherein the human portion of the chimeric polypeptide comprises $\alpha 1$, $\alpha 2$, and $\alpha 3$ domains of a human MHC I polypeptide, wherein the non-human mammal portion of the chimeric polypeptide comprises transmembrane and cytoplasmic domains of an endogenous non-human mammal MHC I polypeptide and wherein the non-human mammal expresses the chimeric human/non-human mammal MHC I polypeptide.

28. The non-human mammal according to any one of the preceding items, wherein every one of the said at least two HLA alleles is encoded as a monocistron, preferably every one of said at least two HLA alleles encoded as monocistron is selected form human HLA allele groups comprising or consisting of:

a) HLA-A*03,
b) HLA-A*11,
c) HLA-B*07,
d) HLA-B*15,
e) HLA-C*04 and
f) HLA-C*07.

29. The non-human mammal according to any one of the preceding items, wherein each HLA allele is equipped with its mouse endogenous own H-2Db promoter (e.g., having or comprising SEQ ID NO: 227) and a polyadenylation signal (e.g., having or comprising SEQ ID NO: 228).

30. The non-human mammal according to any one of the preceding items, wherein the mammal comprises in its genome the full human T cell receptor (TCR) loci.

31. The non-human mammal according to any one of the preceding items, where the mammal is deficient in its endogenous T cell receptor (TCR) loci.

32. The non-human mammal according to any one of the preceding items, wherein the non-human mammal is selected from the group consisting of a rodent, a dog, a felidae, a primate, a rabbit, a pig and a ruminant.

33. The non-human mammal according to any one of the preceding items, wherein the rodent is a mouse or a rat.

34. A nucleic acid construct comprising a nucleic acid encoding at least two (e.g., at least three, at least four, at least five, at least six or at least seven etc.) human HLA class I alleles, wherein said nucleic acid construct is capable of functionally expressing said at least two human HLA alleles (e.g., in a non-human mammal, e.g., as described herein) in that the corresponding MHC I polypeptides are expressed on the surface of cells of a non-human mammal comprising the nucleic acid construct and present MHC antigens to which the non-human mammal provides an antigen-specific CD8+ T cell response and optionally wherein said at least two human HLA class I alleles comprise:

> a) at least one (e.g., at least two or at least three) human HLA-A allele; and/or
> b) at least one (e.g., at least two) human HLA-B allele; and/or
> c) at least one (e.g., at least two) human HLA-C allele; and/or
> d) (a) and (b); and/or
> e) (a) and (c); and/or
> f) (b) and (c); and/or
> g) (a), (b) and (c).

35. The nucleic acid construct according to any one of the preceding items, wherein the nucleic acid further encoding at least one human HLA class II allele.

36. The nucleic acid construct according to any one of the preceding items, wherein the nucleic acid construct comprises 4, 5, 6 or 7 human HLA class I alleles.

37. The nucleic acid construct according to any one of the preceding items, wherein the nucleic acid construct comprises

> a) at least two different human HLA-A alleles, preferably at least three different human HLA-A alleles; and/or
> b) at least two different human HLA-B alleles; and/or
> c) at least two different human HLA-C alleles.

38. The nucleic acid construct according to any one of the preceding items, wherein the at least two human HLA class I alleles comprise one or more HLA-A class I alleles selected from the group consisting of: HLA-A*03 (e.g., HLA-A*03:01), HLA-A*11 (e.g., HLA-A*11:01), HLA-A*01 (e.g., HLA-A*01:01), HLA-A*26 (e.g., HLA-A*26:01), HLA-A*24 (e.g., HLA-A*24:02), HLA-A*32 (e.g., HLA-A*32:01) and HLA-A*02 (e.g., HLA-A*02:01), preferably the three HLA-A class I alleles are selected from human HLA allele groups consisting of: HLA-A*03, HLA-A*11 and HLA-A*02.

39. The nucleic acid construct according to any one of the preceding items, wherein the at least two human HLA class I alleles comprise one or more HLA-A class I alleles selected from the group consisting of: HLA-A*11 (e.g., HLA-A*11:01), HLA-A*24 (e.g., HLA-A*24:02) and HLA-A*33 (e.g., HLA-A*33:03), preferably HLA-A*24 (e.g., HLA-A*24:02) and HLA-A*33 (e.g., HLA-A*33:03).

40. The nucleic acid construct according to any one of the preceding items, wherein the at least two human HLA class I alleles comprise one or more HLA-A class I alleles selected from the group consisting of: HLA-A*24 (e.g., HLA-A*24:02), HLA-A*02 (e.g., HLA-A*02:01), HLA-A*11 (e.g., HLA-A*11:01) and HLA-A*31 (e.g., HLA-A*31:01), preferably HLA-A*24 (e.g., HLA-A*24:02) and HLA-A*31 (e.g., HLA-A*31:01).

41. The nucleic acid construct according to any one of the preceding items, wherein the at least two human HLA class I alleles comprise one or more HLA-A class I alleles selected from the group consisting of: HLA-A*01 (e.g., HLA-A*01:01), HLA-A*11 (e.g., HLA-A*11:01) and HLA-A*24 (e.g., HLA-A*24:02), preferably HLA-A*01 (e.g., HLA-A*01:01) and HLA-A*24 (e.g., HLA-A*24:02).

42. The nucleic acid construct according to any one of the preceding items, wherein said at least two HLA-A class I alleles are selected from human HLA allele groups consisting of: HLA-A*03 (e.g., HLA-A*03:01), HLA-A*11 (e.g., HLA-A*11:01), HLA-A*01 (e.g., HLA-A*01:01), HLA-A*26 (e.g., HLA-A*26:01), HLA-A*24 (e.g., HLA-A*24:02), HLA-A*32 (e.g., HLA-A*32:01) and HLA-A*02 (e.g., HLA-A*02:01), preferably said at least three HLA-A class I alleles are selected from human HLA allele groups consisting of: HLA-A*03, HLA-A*11 and HLA-A*02.

43. The nucleic acid construct according to any one of the preceding items, wherein the at least two HLA class I alleles comprise one or more HLA-B class I alleles selected from the group consisting of: HLA-B*07 (e.g., HLA-B*07:02), HLA-B*15 (e.g., HLA-B*15:01), HLA-B*58 (e.g., HLA-B*58:01), HLA-B*40 (e.g., HLA-B*40:01), HLA-B*35 (e.g., HLA-B*35:01) and HLA-B*08 (e.g., HLA-B*08:01), preferably the at least two HLA-B class I alleles are selected from human HLA allele groups consisting of: HLA-B*07 and HLA-B*15.

44. The nucleic acid construct according to any one of the preceding items, wherein the at least two HLA class I alleles comprise one or more HLA-B class I alleles selected from the group consisting of (China): HLA-B*40 (e.g., HLA-B*40:01), HLA-B*46 (e.g., HLA-B*46:01), HLA-B*58 (e.g., HLA-B*58:01) and HLA-B*15 (e.g., HLA-B*15:02), preferably HLA-B*40 (e.g., HLA-B*40:01) and HLA-B*46 (e.g., HLA-B*46:01).

45. The nucleic acid construct according to any one of the preceding items, wherein the at least two HLA class I alleles comprise one or more HLA-B class I alleles selected from the group consisting of (Japan): HLA-B*52 (e.g., HLA-B*52:01), HLA-B*51 (e.g., HLA-B*51:01), HLA-B*35 (e.g., HLA-B*35:01), HLA-B*15 (e.g., HLA-B*15:01) and HLA-B*40 (e.g., HLA-B*40:02), preferably HLA-B*52 (e.g., HLA-B*52:01) and HLA-B*51 (e.g., HLA-B*51:01).

46. The nucleic acid construct according to any one of the preceding items, wherein the at least two HLA class I alleles comprise one or more HLA-B class I alleles selected from the group consisting of (India): HLA-B*40 (e.g., HLA-B*40:06), HLA-B*51 (e.g., HLA-B*51:01), HLA-B*52 (e.g., HLA-B*52:01) and HLA-B*44 (e.g., HLA-B*44:03), preferably HLA-B*40 (e.g., HLA-B*40:06) and HLA-B*51 (e.g., HLA-B*51:01).

47. The nucleic acid construct according to any one of the preceding items, wherein said at least two HLA-B class I alleles are selected from human HLA allele groups consisting of: HLA-B*07 (e.g., HLA-B*07:02), HLA-B*15 (e.g., HLA-B*15:01), HLA-B*58 (e.g., HLA-B*58:01), HLA-B*40 (e.g., HLA-B*40:01), HLA-B*35 (e.g., HLA-B*35:01) and HLA-B*08 (e.g., HLA-B*08:01), preferably the two HLA-B class I alleles are selected from HLA-B*07 and HLA-B*15.

48. The nucleic acid construct according to any one of the preceding items, wherein the at least two HLA class I alleles comprise one or more HLA-C class I alleles selected from the group consisting of: HLA-C*04 (e.g., HLA-C*04:01), HLA-C*07 (e.g., HLA-C*07:02), HLA-C*16 (e.g., HLA-C*16:01), HLA-C*03 (e.g., HLA-C*03:04), HLA-C*07 (e.g., HLA-C*07:01), and HLA-C*06 (e.g., HLA-C*06:02), preferably the two HLA-C class I alleles are selected form human HLA allele groups consisting of: HLA-C*04 and HLA-C*07.

49. The nucleic acid construct according to any one of the preceding items, wherein the at least two HLA class I alleles comprise one or more HLA-C class I alleles selected from the group consisting of (China): HLA-C*07 (e.g., HLA-C*07:02), HLA-C*01 (e.g., HLA-C*01:02) and HLA-C*03 (e.g., HLA-C*03:04), preferably HLA-C*01 (e.g., HLA-C*01:02) and HLA-C*03 (e.g., HLA-C*03:04).

50. The nucleic acid construct according to any one of the preceding items, wherein the at least two HLA class I alleles comprise one or more HLA-C class I alleles selected from the group consisting of (Japan): HLA-C*01 (e.g., HLA-C*01:02), HLA-C*03 (e.g., HLA-C*03:03) and HLA-C*03 (e.g., HLA-C*03:04), preferably HLA-C*01 (e.g., HLA-C*01:02) and HLA-C*03 (e.g., HLA-C*03:03).

51. The nucleic acid construct according to any one of the preceding items, wherein the at least two HLA class I alleles comprise one or more HLA-C class I alleles selected from the group consisting of (India): HLA-C*06 (e.g., HLA-C*06:02), HLA-C*04 (e.g., HLA-C*04:01), HLA-C*07 (e.g., HLA-C*07:02) and HLA-C*15 (e.g., HLA-C*15:02), preferably HLA-C*06 (e.g., HLA-C*06:02) and HLA-C*15 (e.g., HLA-C*15:02).

52. The nucleic acid construct according to any one of the preceding items, wherein said at least two HLA-C class I alleles are selected form human HLA allele groups consisting of: HLA-C*04 (e.g., HLA-C*04:01), HLA-C*07 (e.g., HLA-C*07:02), HLA-C*16 (e.g., HLA-C*16:01), HLA-C*03 (e.g., HLA-C*03:04), HLA-C*07 (e.g., HLA-C*07:01), and HLA-C*06 (e.g., HLA-C*06:02), preferably said at least two HLA-C class I alleles are selected form human HLA allele groups consisting of: HLA-C*04 and HLA-C*07.

53. The nucleic acid construct according to any one of the preceding items, wherein the mammal comprises in its genome seven human leukocyte antigen (HLA) class I alleles, wherein the seven human HLA are selected form human HLA allele groups comprising or consisting of:

    a) HLA-A*03,
    b) HLA-A*11,

c) HLA-B*07,
d) HLA-B*15,
e) HLA-C*04,
f) HLA-C*07 and
g) HLA-A*02.

54. The nucleic acid construct according to any one of the preceding items, wherein the seven human HLA alleles comprise or consist of:

a) HLA-A*03:01,
b) HLA-A*11:01,
c) HLA-B*07:02,
d) HLA-B*15:01,
e) HLA-C*04:01,
f) HLA-C*07:02 and
g) HLA-A*02:01.

55. The nucleic acid construct according to any one of the preceding items, wherein the HLA alleles encode chimeric human/non-human mammal MHC molecules.

56. The nucleic acid construct according to any one of the preceding items, wherein the at least two HLA alleles encode a chimeric human/non-human mammal MHC I polypeptide, wherein the human portion of the chimeric polypeptide comprises $\alpha$1 and $\alpha$2 domains of a human MHC I polypeptide, wherein the non-human mammal portion of the chimeric polypeptide comprises the $\alpha$3 domain, the transmembrane and the cytoplasmic domains of an endogenous non-human mammal MHC I polypeptide.

57. The nucleic acid construct according to any one of the preceding items, wherein the at least two HLA alleles encode a chimeric human/non-human MHC I polypeptide, wherein the human portion of the chimeric polypeptide comprises $\alpha$1, $\alpha$2, and $\alpha$3 domains of a human MHC I polypeptide, wherein the non-human mammal portion of the chimeric polypeptide comprises transmembrane and cytoplasmic domains of an endogenous non-human mammal MHC I polypeptide.

58. The nucleic acid construct according to any one of the preceding items, wherein every of the at least two HLA allele is encoded as a monocistron, preferably every one of the said at least three HLA alleles encoded as monocistron are selected form human HLA allele groups comprising or consisting of:

a) HLA-A*03,
b) HLA-A*11,
c) HLA-B*07,
d) HLA-B*15,
e) HLA-C*04 and
f) HLA-C*07.

59. The nucleic acid construct according to any one of the preceding items, wherein each HLA allele is equipped with its endogenous own H-2Db promoter and polyadenylation signal, preferably said at least two HLA alleles are selected form human HLA allele groups comprising or consisting of:

a) HLA-A*03,
b) HLA-A*11,
c) HLA-B*07,
d) HLA-B*15,
e) HLA-C*04 and
f) HLA-C*07.

60. The nucleic acid construct according to any one of the preceding items, wherein the endogenous own H-2Db promoter is a MHC H-2 class I promoter (e.g., having or comprising SEQ ID NO: 227).

61. The nucleic acid construct according to any one of the preceding items, wherein the polyadenylation signal (pA) is a bovine growth hormone (bGH) polyadenylation signal (e.g., having or comprising SEQ ID NO: 228), preferably said at least two HLA alleles are selected form human HLA allele groups comprising or consisting of:

a) HLA-A*03,
b) HLA-A*11,
c) HLA-B*07,
d) HLA-B*15,
e) HLA-C*04 and
f) HLA-C*07.

62. The nucleic acid construct according to any one of the preceding items, wherein the nucleic acid construct encodes 2A peptide linkers (e.g., having or comprising SEQ ID NO: 229) such that the encoded least two human MHC class I molecules are segregated by 2A peptide linkers, preferably said at least two HLA alleles are two or more selected from:

a) HLA-A*03,
b) HLA-A*11,
c) HLA-B*07,
d) HLA-B*15,
e) HLA-C*04 and
f) HLA-C*07.

63. The nucleic acid construct according to any one of the preceding items, further encoding a leader sequence (e.g., having or comprising SEQ ID NO: 230).

64. The nucleic acid construct according to any one of the preceding items, wherein said nucleic acid construct is according to any one of the Figures 1-22 disclosed herein (e.g., said nucleic acid construct is ITRs-flanked, e.g., with 5'-PiggyBac-ITR, e.g., having or comprising SEQ ID NO: 231 and 3'-PiggyBac-ITR, e.g., having or comprising SEQ ID NO: 232).

65. The nucleic acid construct according to any one of the preceding items, wherein the leader sequence is a human β2-microglobulin (β2m) leader sequence (e.g., having or comprising SEQ ID NO: 233)

66. The nucleic acid construct according to any one of the preceding items, wherein the nucleic acid construct is codon-optimized for expression in said non-human mammal.

67. An expression vector comprising the nucleic acid construct according to any one of the preceding items.

68. A host cell (e.g., recombinant and/or isolated and/or non-human host cell) comprising the nucleic acid construct according to any one of the preceding items and/or the expression vector according to any one of preceding items.

69. A method of modifying endogenous HLA alleles of a non-human mammal, said method comprising: transducing and/or transplanting said non-human mammal with the nucleic acid construct according to any one of preceding items and/or the expression vector according to any one of preceding items.

70. The method according to any one of the preceding items, wherein transducing is carried out by pronuclei microinjection.

71. A method of producing a non-human, mammalian oocyte carrying a modified target sequence in its genome (e.g., by the means of transposon-mediated targeting, e.g., as described in the experimental section herein), the method comprising the steps of introducing into a non-human, mammalian oocyte the nucleic acid construct (e.g., ITRs-flanked targeting vector (e.g., cassette bearing HLAs)) according to any one of preceding items and/or the expression vector according to any one of preceding items.

72. The method of according to any one of the preceding items, wherein the nucleic acid construct according to any one of preceding items and/or the expression vector according to any one of preceding items is injected into the nucleus/pronucleus of the oocyte or is introduced into the oocyte by electroporation.

73. A method of producing a non-human mammal carrying a modified target sequence in its genome, the method comprising: (a) producing an oocyte as recited in any one of preceding items; (b) analysing the offspring delivered by the non-human female host to which the oocyte obtained in (a) has been transferred for the presence of the modification.

74. The method according to any one of the preceding items, wherein the method comprises a transposon-mediated targeting step.

75. The method according to any one of the preceding items, wherein the non-human mammal is selected from the group consisting of a rodent, a dog, a felidae, a primate, a rabbit, a pig and a ruminant.

76. The method according to any one of the preceding items, wherein the rodent is a mouse or a rat.

77. A non-human mammal produced and/or modified by the method according to any one of the preceding items.

78. A method of generating one or more T cell receptors that are capable of binding to an antigen of interest, the method comprising administering to a non-human animal as defined in any one of the preceding items an antigen of interest.

79. The method according to any one of the preceding items, wherein the antigen of interest is a full-length polypeptide or a fragment of the full-length polypeptide.

80. The method according to any one of the preceding items, wherein the antigen is a tumour-specific antigen (TSA) or a tumour-associated antigen.

81. The method according to any one of the preceding items, further comprising isolating T cells binding the antigen of interest from cells of the non-human animal.

82. The method according to any one of the preceding items, further comprising isolating a nucleic acid encoding the T cell receptor binding the antigen of interest from the cells of the non-human animal.

83. The method according to any one of the preceding items, wherein the T cell receptors are human T cell receptors.

84. A T-cell receptor obtained by or obtainable by the method according to any one of the preceding items.

85. A method of identifying an epitope to which an immune response can be elicited, the method comprising administering to a non-human animal as defined in any of the preceding items an antigen of interest suspected to contain an epitope to which an immune response can be elicited.

86. The method according to any one of the preceding items, wherein the antigen of interest is a full-length polypeptide or a fragment of the full-length polypeptide.

87. The method according to any one of the preceding items, wherein the epitope is selected from the group consisting of a tumour-specific epitope, a viral epitope, a neoepitope and a human self-epitope.

88. The method according to any one of the preceding items, further comprising isolating T cells binding the antigen of interest from cells of the non-human animal.

89. The method according to any one of the preceding items, further comprising isolating a nucleic acid encoding the T cell receptor binding the antigen of interest from the cells of the non-human animal.

90. The method according to any one of the preceding items, further comprising recombinantly producing the T cell receptor and determining the epitope to which the T cell receptor binds.

91. The method according to any one of the preceding items, wherein the method is a method of unbiased epitope discovery.

92. The method according to any one of the preceding items, wherein the epitope is an MHC I restricted epitope.

93. An epitope to which identifying an epitope to which an immune response can be elicited, the epitope being identified by the method according to any one of the preceding items, preferably said epitope a MAGE-A1 epitope GTLEEVPTA (SEQ ID NO: 200).

94. The use of a non-human animal according to any one of the preceding items for generating one or more T cell receptors that are capable of binding to an antigen of interest.

95. The use of a non-human animal according to any one of the preceding items for identifying one or more T cell receptors that are capable of binding to an antigen of interest.

96. The use according to any one of the preceding items, wherein the antigen is selected from the group consisting of a tumour-specific antigen, a viral antigen, a neoepitope and a human self-antigen.

97. The use of a non-human animal according to any one of the preceding items for identifying an epitope to which an immune response can be elicited.

**EXAMPLES OF THE INVENTION**

In order that the invention may be readily understood and put into practical effect, some aspects of the invention are described by way of the following non-limiting examples.

**Example 1:**

Materials and Methods

Materials

*In silico* software algorithms

**Table 1:** List of web-based resources used for T cell epitope prediction-screen

| Web server | Link to the open-source repository |
| --- | --- |
| **NetMHC**<br>Prediction of peptide-MHC class I binding using artificial neural networks (ANNs) | http://www.cbs.dtu.dk/services/NetMHC/ |
| IEDB<br>Prediction of peptide-MHC class I binding using stabilized matrix methods (SMMs) and ANNs | https://www.iedb.org/home_v3.php |

Databases and datasets

**Table 2.** List of datasets retrieved for HLA frequencies, ranking, and cancer incidence

| Web server | Link to the open-source repository |
| --- | --- |
| Allele frequency net database (AFND)<br>Global HLA allele frequency data | http://allelefrequencies.net |
| National marrow donor program (NMDP) bioinformatics<br>HLA ranking datasets | https://bioinformatics.bethematchcli nical.org/hla-resources/ |
| Immunogenetics (IMGT)/HLA sequence database<br>Human major histocompatibility complex/human | https://www.ebi.ac.uk/ipd/imgt/hla/a llele.html |
| leukocyte antigen (HLA) sequences | |

(continued)

| Web server | Link to the open-source repository |
|---|---|
| Global cancer observatory (GLOBOCAN) dataset Global cancer incidence data | http://globocan.iarc.fr |

**Cell culture media, flasks, tubes, and plates**

Table 3: List of cell culture media. *standard media preparation: DMEM/RPMI + 10% (i.e. 50 ml per 500 ml) of heat-inactivated, sterile-filtered fetal bovine serum + 100 IU/ml penicillin and 100 μg/ml streptomycin. #mouse T cell culture medium (mTCM): *standard RPMI media + 1 mM sodium pyruvate + 0.1 mM non-essential amino acids solution + 2 mM HEPES buffer + 50 μM β-mercaptoethanol.

| Media | Manufacturer |
|---|---|
| DMEM, GlutaMAX™ supplemented * | Thermo Fisher Scientific, Rockford (USA) |
| RPMI 1640, GlutaMAX™ supplemented *# | Thermo Fisher Scientific, Rockford (USA) |

Table 4: List of consumables

| Flasks, tubes and, plates | Manufacturer |
|---|---|
| Cell culture flasks: 25, 75, and 150 cm$^2$ | TPP Products AG, Trasadingen (Switzerland) |
| Cell strainers: 40 and 70 μm | BD Biosciences GmbH, Heidelberg (Germany) |
| Syringes: 1, 5, and 10 ml | BD Biosciences GmbH, Heidelberg (Germany) |
| Plates: 96-well U- and flat- bottom | TPP Products AG, Trasadingen (Switzerland) |
| 24-well plates: tissue and non-tissue treated | BD Biosciences GmbH, Heidelberg (Germany) |
| 12-well and 48-well plates | BD Biosciences GmbH, Heidelberg (Germany) |
| Centrifugation tubes: 15 and 50 ml | TPP Products AG, Trasadingen (Switzerland) |
| FACS tubes, capped with 40 μm cell strainer | BD Biosciences GmbH, Heidelberg (Germany) |
| Cryogenic storage tubes and cell culture dishes | GreinerAG, Frickenhausen (Germany) |
| PCR reaction tubes (0.5, 1, and 2 ml) | Eppendorf AG, Hamburg (Germany) |
| Pipettes: 2, 5, 10, 25, and 50 ml | Starlab GmbH, Hamburg (Germany) |
| Pipette tips, TipOne®: 10, 200, and 1000 μl | Starlab GmbH, Hamburg (Germany) |
| EDTA - Mini blood collection tubes | Merck KGaA, Darmstadt (Germany) |
| 5PRIME Phase Lock Gel tubes | Quantabio Inc., Beverly (USA) |
| Pasteur Pipettes | DWK Life Sciences GmbH, Mainz (Germany) |

**Chemical reagents**

Table 5: List of chemical reagents

| Reagents | Manufacturer |
|---|---|
| Dimethyl sulfoxide (DMSO) | Merck KgaA, Darmstadt (Germany) |
| Fetal bovine serum (FBS) | PAN-Biotech GmbH, Aidenbach (Germany) |
| Lipofectamine® 2000 transfection reagent | Thermo Fisher Scientific, Rockford (USA) |

(continued)

| Reagents | Manufacturer |
|---|---|
| 2-mercaptoethanol | Thermo Fisher Scientific, Rockford (USA) |
| Dulbecco's phosphate-buffered saline (DPBS) | Sigma-Aldrich Chemie GmbH, St. Louis (USA) |
| Ammonium chloride ($NH_4Cl$) | Sigma-Aldrich Chemie GmbH, St. Louis (USA) |
| Potassium bicarbonate ($KHCO_3$) | Sigma-Aldrich Chemie GmbH, St. Louis (USA) |
| Ammonium acetate ($NH_4CH_3CO_2$) | Sigma-Aldrich Chemie GmbH, St. Louis (USA) |
| Natrium chloride (NaCl) | neoLab GmbH, Heidelberg (Germany) |
| Protamine sulfate salt from herring | Sigma-Aldrich Chemie GmbH, St. Louis (USA) |
| Phorbol 12-Myristate 13-Acetate (PMA) | Sigma-Aldrich Chemie GmbH, St. Louis (USA) |
| 4-(2-hydroxy-ethyl)-1-piperazine-ethanesulfonic acid (HEPES) | Thermo Fisher Scientific, Rockford (USA) |
| Ethylenediaminetetraacetic acid (EDTA) | neoLab GmbH, Heidelberg (Germany) |
| Sodium dodecyl sulfate (SDS) | Sigma-Aldrich Chemie GmbH, St. Louis (USA) |
| Tris hydrochloride (Tris-HCL) | Carl Roth GmbH, Karlsruhe (Germany) |
| Proteinase K | Sigma-Aldrich Chemie GmbH, St. Louis (USA) |
| Ribonuclease A (RNase A) | Sigma-Aldrich Chemie GmbH, St. Louis (USA) |
| Trypan blue staining 0.4% Solution | Thermo Fisher Scientific, Rockford (USA) |
| Phenol-chloroform-isoamyl alcohol mixture | Sigma-Aldrich Chemie GmbH, St. Louis (USA) |
| Glycogen | Thermo Fisher Scientific, Rockford (USA) |
| Freund's Adjuvant, Incomplete (IFA) | Sigma-Aldrich Chemie GmbH, St. Louis (USA) |
| CpG1826 oligonucleotide (tccatgacgttcctgacgtt) | TIB Molbio GmbH, Berlin (Germany) |
| Ethanol (99.9%) | AppliChem GmbH, Darmstadt (Germany) |
| 7AAD viability staining solution | BioLegend, Fell (Germany) |
| Protein transport inhibitor (with Brefeldin A) | BD Biosciences GmbH, Heidelberg (Germany) |
| CountBright™ absolute counting beads | Thermo Fisher Scientific, Rockford (USA) |
| Dynabeads™ mouse T-activator CD3/CD28 | Thermo Fisher Scientific, Rockford (USA) |

**Buffers**

**Table 6:** List of buffers

| Buffers | Composition |
|---|---|
| Fluorescence activated cell sorting (FACS) buffer | 1X PBS (pH 7.2) 1 % FBS (v/v) |
| Magnetic-activated cell sorting (MACS) buffer | 1X PBS (pH 7.2) 1 % FBS (v/v) |
|  | 2 mM EDTA (w/v) |
| Ammonium-Chloride-Potassium (ACK) lysis | $H_2O$ (pH 7.2 to 7.4) $NH_4Cl$ (0.15 M) |
| buffer for RBC lysis | $KHCO_3$ (10 mM) |

**Cell lines**

Table 7: List of cell lines

| Cell lines | Characteristics |
|---|---|
| A375 (ATCC CRL-1619) and SK-Mel-37 (ATCC HTB-70) melanoma cell lines | Homo sapiens, skin-derived epithelial origin HLA-A*02:01$^+$ NY-ESO$^+$ TRRAP$^+$ mutant (A375) and wild-type (SK-Mel-37) |
| Lymphoblastoid cell line (LCL) (ATCC CRL-9006), BM14 (provided by Dolores J. Schendel, Medigene AG, Munich | Homo sapiens, EBV-transformed, B cell origin HLA-A*03:01$^+$, B*07:02$^+$, and C*07:02$^+$ |
| MCA205 methylcholanthrene-induced fibrosarcoma cell line (Sigma-Aldrich-SCC173) | Mus musculus (C57BL/6), mesenchymal origin H-2 D$^{b+}$ and K$^{b+}$ |

**Cytokines**

Table 8: List of cytokines

| Cytokines | Manufacturer |
|---|---|
| Recombinant murine IFN-$\gamma$ | PeproTech, Hamburg (Germany) |
| Recombinant human/mouse IL-2 | Novartis Pharma AG, Basel (Switzerland) |
| Recombinant murine IL-15 | PeproTech, Hamburg (Germany) |

**Antibodies and Primers**

Table 9: List of antibodies

| Antibodies | Clone / Manufacturer |
|---|---|
| Anti-mouse CD3$\epsilon$ PE | 145-2C11 / BioLegend |
| Anti-mouse CD8a APC | 53-6.7 / BioLegend |
| Anti-mouse CD4 BV421 ™ | GK1.5 / BioLegend |
| Anti-mouse IFN-$\gamma$ BV421 ™ | XMG1.2 / BioLegend |
| Anti-human pan HLA-ABC PE | W6/32 / BioLegend |
| Anti-human $\beta$2 microglobulin PE | TÜ99 / Beckman Coulter |
| Anti-human HLA-A3 PE | GAP-A3 / eBioscience |
| Anti-human HLA-B7 FITC | BB7.1 / Abcam |
| Anti-human pan HLA-C unconjugated with anti-Mouse IgG3$\gamma$ PE | H-5 (unconjugated) with SB76b (secondary antibody) / Santa Cruz Biotechnology |
| Anti-mouse CD16/32 Fc receptor block | 93 / BioLegend |
| Anti-mouse CD28 unconjugated for cell culture | CD28.2 / BioLegend |
| Anti-mouse CD3 unconjugated for cell culture | OKT-3 / BioLegend |

**Table 10:** List of primers

| Primers | Sequence |
|---|---|
| ***Primers used for H-2 D<sup>b</sup> promoter activity assessment; HLAs switched for eGFP*** | |
| H-2D<sup>b</sup>For-Primer | SEQ ID NO: 1<br>GTCTGGCGCGCCTTCTTCTTCTACATA (AscI-site) |
| H-2D<sup>b</sup>Rev-Primer | SEQ ID NO: 2<br>CTCGCCCTTGCTCACCATCTGGGATCCCGGGGTGT |
| eGFPFor-Primer | SEQ ID NO: 3<br>ATGGTGAGCAAGGGCGAG |
| eGFPRev-Primer | SEQ ID NO: 4<br>GTCTTAATTAATTACTTGTACAGCTCGTCCATGC |
| ***Primers used for CMV promoter activity assessment; H-2D<sup>b</sup> switched for CMV promoter*** | |
| CMV-Forward Primer_AscI | SEQ ID NO: 5<br>GCT GGCGCGCC GTACGGGCCAGATATACGC |
| CMV-Reverse Primer_XmaI | SEQ ID NO: 6<br>GTC CCCGGG GTGGGTTCTCTAGTTAGCCAGA |
| ***Primers used for full-length H-2 D<sup>b</sup> polyadenylation signal manipulation*** | |
| For-H-2-Full-PA | SEQ ID NO: 7<br>ATCTGCATCCTGTAAGCTCCATGCTAC |
| Rev-H-2-Full-PA | SEQ ID NO: 8<br>GTCTTAATTAACCATCACAGATCAAAATGGACTGAGC |
| ***Primers used for full-length H-2 D<sup>b</sup> polyadenylation signal manipulation*** | |
| For-BGH-Full-PA | SEQ ID NO: 9<br>GACTGCTCTTCCAAACTGTGCCTTCTAGTTGCCAGCC |
| Rev-BGH-Full-PA | SEQ ID NO: 10<br>GTCTTAATTAACCATAGAGCCCACCGCATC |

(continued)

| Multiple cloning site (MCS) introduced into the pMA-V vector to clone six HLA alleles one after another (5'-to-3') | |
|---|---|
| SEQ ID NO: 11<br><br>CTATCGTAGGCGCGCCTCCGATGCGGCCGCAGTTATCGCGCGCGATTGTACGCCGG CGATTGT<br>ACCTGCAGGCTTAAGATTACCGGTGCCTTGAAGCTTGTTGCAGGTACCTCGGCGGAG CTCGAA GGCGTACTAGTGCTGATACGCGTCGTAGCTCGTTAATTAACGG<br>MCS sequence contains restriction sites (5'-to-3'): AscI, NotI, MauBI, MreI, SbfI, AflII, AgeI, HindIII, KpnI, SacI, SpeI, MluI, PacI. | |
| **Primers to amplify MCS** | |
| MCS-FP | SEQ ID NO: 12<br>CTATCGTAGGCGCGCCTCC |
| MCS-RP | SEQ ID NO: 13<br>CCGTTAATTAACGAGCTACGACGC |
| **Primers used for cloning six HLA alleles one after another into pMA-V (5'-to-3')** | |
| FP-H LA-A3-AscI | SEQ ID NO: 14<br>CCTAGGCGCGCCTTCTTCTAC |
| RP-HLA-A3-NotI | SEQ ID NO: 15<br>AGTACGCGGCCGCCCATAGAGCCCACCGCATC |
| FP-HLA-A 11-NotI | SEQ ID NO: 16<br>CGTAGCGGCCGCGCGCCTTCTTCTACATAAAACACAC |
| RP-HLA-A11-SbfI | SEQ ID NO: 17<br>CAGTTACCTGCAGGCCATAGAGCCCACCGCATC |
| FP-H LA-B7-SbfI | SEQ ID NO: 18<br>CAGCCCTGCAGGGCCTTCTTCTACATAAAACACACCC |
| RP-HLA-B7-AgeI | SEQ ID NO: 19<br>TGCAACCGGTCCATAGAGCCCACCGCATC |
| FP-HLA-B15-HindIII | SEQ ID NO: 20<br>CTAGTGAAGCTTCGCCTTCTTCTACATAAAACACACC |
| RP-HLA-B15-KpnI | SEQ ID NO: 21<br>TCGACAGGTACCCATAGAGCCCACCGCATC |

(continued)

| Primers used for cloning six HLA alleles one after another into pMA-V (5'-to-3') | |
|---|---|
| FP-H LA-C4-SacI | SEQ ID NO: 22<br>CTAGAGCTCCGCCTTCTTCTACATAAAACACACC |
| RP-HLA-C4-SpeI | SEQ ID NO: 23<br>GATACTAGTCCATAGAGCCCACCGCATC |
| FP-HLA-C7-SpeI | SEQ ID NO: 24<br>TCAGACTAGTCGCCTTCTTCTACATAAAACACACC |
| RP-HLA-C7-PacI | SEQ ID NO: 25<br>GACATTAATTAACCATAGAGCCCACCGCATC |

**Mice**

**Table 11:** List of transgenic and wild-type mice with relevant TCR loci and HLA genotype

| Mice | Genotype |
|---|---|
| ABabDII mice (Li et al., 2010) | Human TCR$^{+/+}$ loci: TCR A/B$^{+/+}$<br>Mouse TCR$^{-/-}$ loci: TCR a/b$^{-/-}$<br>Mouse MHC I : $\beta$2m$^{-/-}$, H-2 D$^{b-/-}$<br>Human HLA I : HLA-A*02:01$^{+/+}$ |
| ABab.I (transgenic mouse of the present invention) | Human TCR$^{+/+}$ loci: TCR A/B$^{+/+}$<br>Mouse TCR$^{-/-}$ loci : TCR a/b$^{-/-}$<br>Mouse MHC I : $\beta$2m$^{-/-}$, H-2 D$^{b-/-}$<br>Human HLA I : HLA-A*03:01/A*11:01$^{+/+}$,<br>HLA-B*07:02/B*15:01$^{+/+}$, |
| | HLA-C*04:01/C*07:02$^{+/+}$ and HLA-A*02:01$^{+/+}$ |
| C57BL6/N (wild-type, bred in-house) | Mouse MHC I : H-2 D$^{b+/+}$, H-2 D$^{b+/+}$ |
| HHD | MouseTCR$^{+/+}$ loci : TCR a/b$^{+/+}$<br>Mouse MHC I : $\beta$2m$^{-/-}$, H-2 D$^{b-/-}$<br>Human HLA I : HLA-A*02:01$^{+/+}$ |

**Laboratory equipment**

**Table 12:** List of equipment

| Equipment | Manufacturer |
|---|---|
| Light microscope, primovert | Carl Zeiss Microscopy GmbH, Jena (Germany) |
| Axiovert 40 C microscope | Carl Zeiss, Göttingen (Germany) |
| Beckman GS-6 series centrifuge | Beckman Coulter Inc., California (USA) |
| Heraeus biofuge pico centrifuge | Heraeus Instruments, Osterode (Germany) |
| Centrifuge, fresco 21 | Thermo Fisher Scientific, Rockford (USA) |
| Centrifuge, rico 21 | Thermo Fisher Scientific, Rockford (USA) |
| Centrifuge, megafuge 8R | Thermo Fisher Scientific, Rockford (USA) |
| Centrifuge, megafuge 40R | Thermo Fisher Scientific, Rockford (USA) |
| Thermomixer comfort | EppendorfAG, Hamburg (Germany) |
| Vortex mixer, Genie-2 | Scientific Industries Inc., New York (USA) |
| Vortex mixer, Sunlab® SU1900 | neoLab GmbH, Heidelberg (Germany) |
| Tabletop centrifuge 5415R/5415D | EppendorfAG, Hamburg (Germany) |
| Finnpipette™ (2-1000 μl) | Thermo Fisher Scientific, Rockford (USA) |
| IBS pipetboy acu | Integra Biosciences AG, Zizers (Switzerland) |
| Multi pipette® M4 | EppendorfAG, Hamburg (Germany) |
| Nano photometer, nanovolume N50 | Implen GmbH, Munich (Germany) |
| Cryogenic storage system, 24k | TecLab GmbH, Taunusstein (Germany) |
| Ice maker, FM-120KE-HC | Hoshizaki B.V., Swanley (UK) |

(continued)

| Equipment | Manufacturer |
|---|---|
| Neubauer chamber (improved) | Paul Marienfeld GmbH, Königshofen |
|  | (Germany) |
| Flow cytometer, FACSCelesta™ | BD Biosciences GmbH, Heidelberg (Germany) |
| Flow cytometer, FACSCanto™ II | BD Biosciences GmbH, Heidelberg (Germany) |
| FACS cell sorter, FACSAria™ I | BD Biosciences GmbH, Heidelberg (Germany) |
| Viessmann cold chamber | Kältetechnik AG, Saale (Germany) |
| Mettler AE 163 analytical balance | Mettler-Waagen GmbH, Gießen (Germany) |
| Milli-Q® advantage A10 | Merck KGaA, Darmstadt (Germany) |
| $CO_2$ incubator, ICOmed | Memmert, Schwabach (Germany) |
| Water bath, WNB / WNE / WPE | Memmert, Schwabach (Germany) |
| BVC fluid aspiration system | BrandTech Scientific, Inc., Essex (UK) |
| Safety workbench safe 2020 | Thermo Fisher Scientific, Rockford (USA) |
| Freezer / refrigerator, mediline | Liebherr GmbH, Bierbach an der Riß (Germany) |

**Software**

**Table 13:** List of commercial software

| Software | Manufacturer |
|---|---|
| GraphPad Prism software (version 8.4.3) | GraphPad Software, Inc., La Jolla (USA) |
| FlowJo™ version 10 (version 10.7.1) | TreeStar Inc., Olten (Switzerland) |
| BioRender (web-based) | BioRender Inc., Toronto (Canada) |
| ImmunoSEQ™ analyzer (web-based) | Adaptive Biotechnologies, Seattle (USA) |
| Mendeley reference manager (version 1.19.8) | Elsevier group, London (UK) |
| SnapGene gene analyzer (version 5.1.5) | GSL Biotech LLC., Illinois (USA) |

**Methods**

**In silico design and construction of chimeric HLA class I alleles**

ABab.I polycistronic transgene encoding six HLA alleles segregated by 2A peptide linkers was designed in silico and synthesized by GeneArt™, Thermo Fisher Scientific. HLA alleles in the transgene are chimeric human-mouse fusion proteins. The $\alpha$1 and $\alpha$2 domains are human cDNAs from respective HLA alleles, $\alpha$3 to cytoplasmic domains are murine cDNAs from the H- 2 Db gene. Human $\beta$2m is linked to the N-terminus by a 15-amino acid glycine-serine peptide linker. Modified HLA constructs with H-2 Db or CMV promoter, 3'-bGH polyadenylation signal variants, and HLA monochains with or without $\beta$2m were custom-cloned in-house. Phusion® DNA polymerase combined HLA amplicons by splicing by overhang extension (SOE) PCR. Six HLA alleles were cloned as a single haplotype sequentially using unique restriction enzyme sites into the pre-integrated multiple cloning site. PiggyBac ITRs-flanked ABab.I transgene was generated using 5'-Ascl and 3'-Pacl sites. Stbl3™ E. coli (Thermo Fisher Scientific, Rockford, USA) propagated long HLA plasmids containing repetitive sequences.

***In vitro* characterization of HLA-AB*ab*.I transgene by flow cytometric analysis**

Surface HLA class I expression was analyzed 48 hours after every transient transfection by flow cytometry.

MCA205 cells were treated with or without recombinant IFN-γ (100 ng/ml) in transfection experiments. 10 μl of Lipofectamine™ 2000 delivered the primary polycistronic AB*ab*.I transgene and all modified HLA constructs. In every case, two days post-transfection, cells were stained using fluorochrome-conjugated antibodies for human pan HLA-ABC and β2 microglobulin. MCA205/AB*ab*.I stable cell line was made by transfecting MCA205 cells using AscI-PacI digested, linearized DNA construct, followed by multiple enrichments of the HLA⁺ cell population through flow cytometry-based cell sorting using pan HLA-ABC antibody.

**Quantification of transgene mRNA using quantitative RT-PCR analysis**

MCA205 cells were transiently transfected as described earlier with polyadenylation signal- modified constructs driven by H-2 D$^b$ or CMV promoter. Two days later, total RNA in cell lysates was reverse transcribed using anchored oligo (dT) and random hexamers. cDNA amplification was quantified using SYBR® green dye with HLA-specific primers in the quantitative RT-PCR (qRT-PCR) reaction. As controls, 18s rRNA (housekeeping) and constitutive H-2 D$^b$ reactions were analyzed. From Ct values, difference of expression, ΔCt = Ct (HLA/H-2 D$^b$) - Ct (18s rRNA) was estimated. The percentage of change in expression was measured by normalizing it to a reference gene, mouse H-2 D$^b$ (set to 100%).

**Construction of genome targeting vectors**

Six chimeric HLA alleles, HLA-A*03:01, A*11:01, B*07:02, B*15:01, C*04:01, and C*07:02, as a natural HLA haplotype like in humans, were cloned into a minimal mammalian expression vector, the pMA plasmid. For mouse genome targeting, 5'-AscI and 3'-PacI sites were digested and blunted sequentially to insert hyperactive PiggyBac-restricted inverted terminal repeats (ITRs) to produce an oocyte targeting vector.

**Generation of the ABab.I transgenic mice using pronuclei injection technology**

In vitro transcribed (ivt) PiggyBac (PB) transposase mRNA targeted the ITR-flanked ABab.I transgene cassette into the recipient oocytes. 10-12 weeks old ABabDII male mice were mating partners. 6-10 weeks old ABabDII female mice were superovulated before mating to collect zygotes. NMRI mice served as pseudopregnant foster mothers. Donor HLA plasmids (ABab.I transgene) were co-prepared with the PB transposase (ivt mRNA) at a molar ratio of 2:1. Mixed DNA with ivt mRNA samples were pronuclei-microinjected into fertilized ABabDII oocytes as described. All mouse experiments were performed as per the standard guidelines approved by the Landesamt für Arbeitsschutz, Gesundheitsschutz und technische Sicherheit, Berlin.

**Genotyping of HLA I haplotype in the ABab.I Tg mice**

Genomic DNA was extracted from ear biopsies of transgenic mice at 95 °C in the presence of 0.05 M NaOH. HLA-specific primers directed against all six chimeric fusion alleles amplified fragments using a routine PCR with Taq DNA polymerase. 18s rRNA and human TCR αβ were control reactions. PCR amplicons were run on a 1.5% gel at 100 V for 45 minutes using an agarose gel electrophoresis system. PCR primers were:

| Primers | Sequence |
|---|---|
| *Primers used for HLA-A*03:01 PCR reaction (product length - 227 bp)* | |
| GP-A3-1 **(SEQ ID NO: 209)** | ACCGACAGAGTGGACCTGG |
| GP-A3-2 **(SEQ ID NO: 210)** | TCCCACTTTCTCTTGGTGATCTGG |
| *Primers used for HLA-A*11:01 PCR reaction (product length - 235 bp)* | |
| GP-A11-3 **(SEQ ID NO: 211)** | CAAAGCTCAGTCCCAGACAGAC |
| GP-A11-4 **(SEQ ID NO: 212)** | CCGCTTTGTAATCTGAGCAGC |
| *Primers used for HLA-B*07:02 PCR reaction (product length - 286 bp)* | |
| GP-B7-5 **(SEQ ID NO: 213)** | CCCAGATCTACAAGGCTCAGGCC |
| GP-B7-6 **(SEQ ID NO: 214)** | TAGGCCCTTCTCTGCTCAGC |
| *Primers used for HLA-B*15:01 PCR reaction (product length - 288 bp)* | |
| **GP-B15-7 (SEQ ID NO: 215)** | CAACACCCAGACATACAGAGAGTCC |

| GP-B15-8 **(SEQ ID NO: 216)** | ACGCACAGTCCTTCCAGATAGG |
| --- | --- |
| ***Primers used for HLA-C*04:01 PCR reaction (product length - 288 bp)*** | |
| GP-C4-9 **(SEQ ID NO: 217)** | CCCAGAAGTACAAGAGACAGGCC |
| GP-C4-10 **(SEQ ID NO: 218)** | GATAGGCTCTGCGCTGTTCG |
| ***Primers used for HLA-C*07:02 PCR reaction (product length - 211 bp)*** | |
| GP-C7-11 **(SEQ ID NO: 219)** | GAACCTAGATTCATCAGCGTCGG |
| GP-C7-12 **(SEQ ID NO: 220)** | CGGACTGGTTATAGTAGCCTCTGAG |
| ***Primers used for 18s rRNA - control PCR reaction (product length - 133 bp)*** | |
| 18s rRNA For **(SEQ ID NO: 221)** | GGCCGTTCTTAGTTGGTGGAGCG |
| 18s rRNA Rev **(SEQ ID NO: 222)** | CTGAACGCCACTTGTCCCTC |
| ***Primers (mixed) used for human TCR $\alpha\beta$ - control PCR reaction (product length-221+691 bp)*** | |
| TCR Alpha forward (SEQ ID NO: 223) + Beta forward (SEQ ID NO: 224) | ATGGCAAAAACCAA GTGGAG + CACATTAGGCCAGG AGAAGC |
| TCR Alpha reverse (SEQ ID NO: 225) + Beta reverse (SEQ ID NO: 226) | TTTGCTTTGTGTCT GCATCC + CCTGCTTAGTGGC TGAGTGG |

**Estimation of CD3+ CD8+/CD4+ absolute numbers in blood and lymphoid** organs

*Preparation of blood:* 50 μl blood per mouse (from each strain) was blocked for *Fc* receptors III/II by anti-CD16/32 antibody. Red blood cells were lysed by erythrocytes lysing buffer (ACK buffer) before staining and flow cytometry. *Preparation of lymphoid organs:* spleen and lymph nodes (axillary, brachial, mediastinal, inguinal, and mesenteric) from 8-12 weeks old mice were mashed and strained through a 70 μm cell strainer. ACK-lysed spleens and lymph nodes were pooled together by passing them through a 40 μm strainer. Staining of blood and lymphoid organs for T cell counts: samples were stained using fluorochrome-conjugated CD3, CD8, and CD4 antibodies. 11 μl volume (11,880 beads) of CountBright™ counting beads per sample were used for T cell quantification by flow cytometry.

$$\text{Absolute T cell numbers/μl of blood or per pooled mouse lymphoid organs} =$$

$$\frac{\text{no. of CD8/CD4 cell events}}{\text{no. of beads events}} \times \frac{\text{bead count of the lot (54,000 beads/50μl)}}{\text{volume of samples (μl)}}$$

**Enrichment of T cell population (CD3+ CD8+) using untouched FACS sorting**

Lymphoid cells and the whole blood from young AB*ab*DII or AB*ab*.I mice (8-12 weeks) were prepared and pooled using the protocol described earlier (refer 2.2.7, *preparation of blood and lymphoid organs).* CD3$^+$ cells were enriched from pooled cell suspensions through bead-based magnetic separation without columns using EasySep™ mouse T cell untouched isolation kit (Stemcell Technologies GmbH, Cologne, Germany). In brief, Fc receptors in cell suspensions ($2\times10^8$ cells/ml) were blocked by incubating with 50 μl/ml rat serum. Pooled cells from the spleen, lymph nodes, and blood were stained using a biotinylated antibody cocktail (50 μl/ml) directed towards non-T cells (CD3$^-$). Streptavidin-coated magnetic spheres (75 μl/ml) captured these CD3$^-$ cells onto the EasySep™ magnet, whereas the CD3$^+$ cells passed the magnetic field into the flow-through solution for collection. Untouched CD3$^+$ cell populations were surface stained with fluorescent antibodies specific for CD3, CD8, and CD4 markers. Flow cytometry-based sorting enriched CD3+ CD8+ T cells ($\geq$ 97% purity) for organic DNA (genomic) extraction using the phenol-chloroform method.

**TCR$\beta$ repertoire deep sequencing of ABab.I mice using ImmunoSEQ® technology**

Deep sequencing of TCR$\beta$ immune repertoire was performed at Adaptive Biotechnologies (Seattle, USA). ImmunoSEQ® platform precisely quantifies all V-J gene combinations in cell populations using a multiplex PCR-based assay with bias correction in the first step to minimize amplification bias. The assay also provides quantitative abundance data with a sensitivity of 1 in 200,000 T cells. 2-4 $\mu$g of genomic DNA/sample obtained from $3\times10^5$ mouse (average in mice) and 1.5 $\mu$g of genomic DNA/sample obtained from about $1.8\times10^5$ human CD8$^+$ T cells were deep sequenced. Three healthy human donors (age: 30, 48, and 60 years old) donated blood with informed consent. Blood collection and processing were performed according to human experimental guidelines under license EA4/046/10 (Ethics committee). Computational analysis was performed on the sequencing data by Adaptive Biotechnologies. ImmunoSEQ™ analyzer portal provided access. TCR sequences were designated based on Immunogenetics (IMGT) nomenclature. Statistical analysis was performed and analyzed as described for CD4$^+$ T cells using the R program.

**Retroviral transduction of murine splenocytes**

Ecotropic retrovirus (only infects mouse/rat cells) packaging cell line, Platinum-E[188] (Plat-E) was transfected at 80% cell confluency using Lipofectamine with pMP71 retroviral plasmids (3 $\mu$g/construct) encoding T cell receptor genes (configuration: TCR$\beta$-mC$\beta$-P2A-TCR$\alpha$-mC$\alpha$). 3 ml of virus supernatants were harvested twice at different time points using a 0.45 $\mu$m membrane filter for infecting murine AB*ab*DII splenocytes. Spleen and lymph nodes were prepared and pooled using the protocol described earlier (refer 2.2.7, *preparation of lymphoid organs*). Splenocytes were pre-activated with anti-CD3 (1 $\mu$g/ml) and anti-CD28 (0.1 $\mu$g/ml) antibodies in the presence of 40 IU/ml recombinant mouse IL-2 (cell concentrations adjusted to $2\times10^6$ cells/ml). Transductions were performed twice at 48- and 72-hours post-transfection. On first transduction, supernatants containing virus particles were first centrifuged (3000g, 90 minutes at 4 °C) onto retronectin-coated non-tissue culture 24-well plates. $2\times10^6$ pre-activated splenocytes in mTCM medium supplemented with 8 $\mu$g/ml protamine sulfate (ps), 10 $\mu$l of CD3/CD28 Dynabeads™, 40 IU/ml IL-2 were added onto virus-captured plates, and centrifuged for 30 minutes, 800g at 32 °C. On second transduction, 1 ml of supernatant was removed from the top layer of the 24-well plates, replaced with a fresh batch of virus (supplemented with 8 $\mu$g/ml ps, 40 IU/ml IL-2) harvested at 72 hours' time point, and centrifuged for 90 minutes, 800g at 32 °C. Post-second transduction, transduced splenocytes were adjusted to have cell concentrations of $2\times10^6$ cells/ml and supplemented with 50 ng/ml murine IL-15.

**Functional characterization of HLA alleles expressed in ABab.I Tg mice in vitro**

IFN-$\gamma$ production was monitored in the supernatants of co-culture assays by enzyme-linked immunosorbent assay (ELISA). Overnight (16 hours) co-cultures were performed with $5\times10^4$ TCR transduced effector T cells and $5\times10^4$ target cells (MCA205/AB*ab*.I tumour cells or peripheral blood cells/lymphoid organs from AB*ab*.I mice). Target cells were pulsed with peptides (1 $\mu$M/peptide) or with phorbol myristate acetate (PMA) and ionomycin (1 $\mu$M each) as a positive control.

**In vivo characterization of HLA-ABab.I transgene through peptide immunization**

A 200 $\mu$l injection suspension containing 100 $\mu$g of short peptides in PBS, 100 $\mu$l of incomplete Freund's adjuvant, and 50 $\mu$g CpG oligonucleotides were injected subcutaneously on both lateral sides of hind legs. 10- to 20-week-old young AB*ab*DII or AB*ab*.I mice (6-7 mice/group) were immunized with a 4-week interval between prime-boosts. Similar peptide mixtures were prepared and injected for further boosting regimens of all peptides. As standard controls, AB*ab*DII[1] and AB*ab*.I mice were immunized with 100 $\mu$g of MAGE-A1$_{278}$ KVLEYVIKV (SEQ ID NO: 26) peptide with a similar injection mixture. 7-10 days after every boost injection, peripheral blood cells from immunized mice were ACK-lysed and cultured with 1 $\mu$M respective specific or unspecific peptide in the presence of protein transport inhibitor for 5-6 hours. Post-cell fixation, cells were intracellularly stained for IFN-$\gamma$, surface stained for CD3, CD8 markers, and analyzed by flow cytometry.

**Statistical analysis**

GraphPad Prism software was used to generate standard curves, plotted graphs, and to calculate the level of statistical significance using mean and standard deviation (P values). FlowJo™ was used to analyze the raw flow cytometry data (.fcs files) with statistics on mean fluorescence intensity values.

**Results**

**Cancer mutanome screen yields potential neoantigens for adoptive T cell therapy**

Recurrent hot-spot mutations that could generate putative tumour-specific antigens (TSAs) were screened *in silico* using open-source servers (Figure 2, Table 1, and 2). 266 non-synonymous somatic point mutations' previously described in 40 distinct genes were selected as TSAs using literature research (Figure 2A). The chosen point mutations (occurring in ≥2 patients) were detected mostly by whole-exome sequencing analysis in 21 different human cancer entities; the mutation frequencies range from 0.1% to 39%. The NetMHC program predicted the affinity of mutant and wild-type epitopes to HLA alleles under applied selection criteria (Figure 2B). Out of these 266 mutants, 175 epitopes showed strong binding affinities *in silico* ($IC_{50}$: <50 nM) for at least one of the 18 highly populated HLA class I alleles (Figure 2C). The AB*ab*DII mouse model expressing HLA-A2 provided a platform to analyze the immunogenicity of the 23 HLA-A2-restricted binders (Figure 2D, left). From the 152 non-HLA- A2 binders, 68 putative epitopes bound 'HLA-A*0301-A*11:01, B*07:02-B*15:01, C*04:01- C*07:02', a novel class I haplotype similar to humans. The restriction status of these six HLA alleles against multiple (>5) mutant epitopes provided the rationale to create the AB*ab*.I mice (Figure 2D, right).

Human T cell receptors (TCRs) isolated from AB*ab*DII and AB*ab*.I mice can be clinically applied for adoptive T cell therapy (ATT) of cancers against endogenously processed mutant antigens.

**AB*ab*DII mouse model elicits HLA-A2-restricted neoantigen-specific T cell responses**

To address neoantigen-specific CD8$^+$ T cell responses restricted to HLA-A2-binders, we chose the mutation S722F frequently occurring in the N-terminus of the transformation/transcription domain-associated protein (TRRAP) gene. Serine to phenylalanine mutation at position 722 produced an HLA-A2 epitope with an $IC_{50}$ of 55 nM in our prediction-screen (Figure 3A and 3B). TRRAP protein is a transcription co-factor involved in cell transformation through MYC activation. 4% of melanoma cases with a total of 86,000 incidences/year express S722F driver mutation. Considering this 4% with S722F express HLA-A2 with an allele frequency of 26.5% in Europe, about 911 individuals were estimated (theoretical) for a possible ATT per year after stratification from prior treatment lines (Figure 3B). Likewise, a worldwide estimate of individuals/year with the TRRAP-S722F mutation using datasets from individual continents such as the U.S.A, South America, and Asia was calculated (data not shown). Mice have a TRRAP orthologue to ensure thymic deletion of wild-type epitope-restricted T cells (Figure 3C).

For TCR candidate discovery, young AB*ab*DII mice (n=7, 12-16 weeks) were immunized with mutant 9-mer peptides (Figure 3B, KLVFGSVFL, SEQ ID NO: 30) at 4-week intervals. CD8$^+$ T cell responses were analyzed in blood one week after every boost using intracellular staining, followed by culturing the T cells and IFN-γ capture assay to sort for peptide-specific T cells (Figure 4A). One-week post-4$^{th}$ injection (3$^{rd}$ boost) regimen, about 0.8% of T cells responded in blood (Figure 4B), which expanded up to about 17% in the spleen (Figure 4C) after T cell culturing under low peptide ($10^{-9}$ M) and IL- 2 (20 IU/ml) concentration for 10 days. Post-T cell culture, 12,000 IFN-γ$^+$ CD8$^+$ cells specific for S722F were captured after 2 hours of stimulation under $10^{-6}$ M peptide concentration (Figure 4D). No wild-type specific T cell responses were observed after *in vitro* re-stimulations (Figure 4B, 4C, and 4D). 5'-RACE PCR yielded one dominant TCRαβ pair (Figure 4E). 5'-RACE amplification procedure used primers annealing to constant TCRαβ and the anchored 5'- adapter region as previously described.

In Figure (Figure 4E) the following SEQ ID NOs apply to the listed CDR3 regions:

| TRRAP TCR chains | V(D)J recombined regions | CDR3 regions | Clonally dominant TCR chains |
|---|---|---|---|
| TCR Vα chain | TRAV17-1-TRAJ58 | SEQ ID NO: 31 CATDAEETSGSRLTF | 11/14 |
| TCR Vβ chain | TRBV27-1-TRBD2-2-TRBJ2-1 | SEQ ID NO: 32 CASSLAGIYNEQFF | 12/15 |
| Sub-clonally dominant TCR chains | | | |
| TCR Vα chain | TRAV38-2-1-TRAJ28-1 | **SEQ ID NO: 33** CAYRSPYSGAGSYQ | 2/20 |
| TCR Vα chain | TRAV26-2-TRAJ29-1 | **SEQ ID NO: 34** CILRDDSGNTPLVF | 1/23 |

(continued)

| Sub-clonally dominant TCR chains | | | |
|---|---|---|---|
| TCR Vβ chain | TRBV27-1-TRBD2-2-TRBJ2-1 | **SEQ ID NO: 35**<br>CASSLAGAYNEQFF | 3/15 |

Transduced T cells were co-cultured with peptide-titrated T2 (TAP$^{-/-}$) cells to ensure TCR interaction and its affinity to S722F. The dominant TCR $V_\alpha$17-$V_\beta$27 pair recognized S722F sensitive enough up to $10^{-11}$ M peptide concentration by producing IFN-$\gamma$ with no wild-type epitope recognition (Figure 4F, left). TRRAP$^+$ human cell lines were co-cultured with transduced T cells to verify proteasomal processing of epitope S722F Transduced T cells neither recognized A375 nor SK-Mel-37 cells; positive for the mutant and wild-type TRRAP, respectively. NY-ESO$_{157-165}$, the HLA-A2 epitope is expressed on melanoma cells A375 and SK-Mel-37. TCR-ESO (control TCR) recognized NY-ESO$_{157-165}$ epitope by producing IFN-$\gamma$ (Figure 4F, right).

Hence, we prove that the TRRAP mutation-derived neoantigen S722F$_{715-723}$ nonamer is not endogenously processed and presented on HLA-A2.

**ABab.I transgene designed to reflect natural human HLA haplotype**

The 23 mutated epitopes from genes, TRRAP, C-KIT, XPO1, FOXA1, RAC1, RAC2, RHOT1, TP53, MAP2K1, EGFR, TRAF7, SMO, SF3B1, FBXW7, SPOP, EZH2, FLT3, NFE2L2, TSHR, PTPN11, and NOTCH1 are predicted to bind to HLA-A2 (Figure 2C, pie chart). Mutant peptides from the first four genes were analyzed similarly for immunogenicity like TRRAP-S772F (Figure 4A). AB*ab*DII mice did not produce CD8$^+$ T cell responses after immunizations with C-KIT (K642E), XPO1 (E571K), and FOXA1 (D226N) epitopes (data not shown). The AB*ab*DII mice provide the opportunity to isolate TCRs restricted only to these 23 HLA-A2-binders.

8/152 non-HLA-A2 binders (Figure 2C, pie chart) are predicted to bind to at least one allele of a novel HLA haplotype, A*0301-A*11:01, B*07:02-B*15:01, and C*04:01-C*07:02 resembling HLA situation in humans (every individual bears six genes). To understand the biology of a whole human haplotype in mice and target the other 68 high-affinity mutants (Table 14), we designed AB*ab*.I transgene based on the *in silico* screen results to reflect a human HLA haplotype (Figure 5).

**Table 14:** *In silico* predicted mutant epitopes binding AB*ab*.I HLA haplotype.

| HLA restriction | Gene | Mutant peptide sequence (mutant AA in bold) / IC$_{50}$ nM | Wildtype peptide sequence (wild-type AA in bold) / IC$_{50}$ nM | Mutation frequency |
|---|---|---|---|---|
| **HLA-A*03:01** | EGFR | **SEQ ID NO: 36**<br>A**S**GAFGTVYK / 23 nM | **SEQ ID NO: 37**<br>**G**SGAFGTVYK / 46 nM | 3% |
| | KLF4 | **SEQ ID NO: 38**<br>KTYT**Q**SSHLK / 10 nM | **SEQ ID NO: 39**<br>KTYT**K**SSHLK / 12 nM | 1% |
| | PIK3CA | **SEQ ID NO: 40**<br>AISTRDPLS**K** / 44 nM | **SEQ ID NO: 41**<br>AISTRDPLS**E** / 19220 nM | 3.7% |
| | FOXA1 | **SEQ ID NO: 42**<br>SLSFN**N**CFVK / 29 nM | **SEQ ID NO: 43**<br>SLSFN**D**CFVK / 77 nM | 1.8% |
| | ABL1 | **SEQ ID NO: 44**<br>**K**VYEGVWKK / 31 nM | **SEQ ID NO: 45**<br>**E**VYEGVWKK / 239 nM | 2.3% |
| | JAK3 | **SEQ ID NO: 46**<br>G**L**LKTVSYK / 37 nM | **SEQ ID NO: 47**<br>E**L**LKTVSYK / 104 nM | 5.6% |
| | NOTCH1 | **SEQ ID NO: 48**<br>RV**P**HTNVVFK / 38 nM | **SEQ ID NO: 49**<br>RV**L**HTNVVFK / 15 nM | 5.5% |

| | | | | |
|---|---|---|---|---|
| | PPP2R1A | SEQ ID NO: 50<br>RMVRRAAASK / 26 nM | SEQ ID NO: 51<br>PMVRRAAASK / 742 nM | 9.6% |
| HLA-A*11:01 | TP53 | SEQ ID NO: 52<br>SSCMGSMNR / 35 nM | SEQ ID NO: 53<br>SSCMGGMNR / 58 nM | Hot-spot mutation<br>(freq. unknown) |
| | KRAS | SEQ ID NO: 54<br>VVGAVGVGK / 40 nM | SEQ ID NO: 55<br>VVGAGGVGK / 89 nM | 22% |
| | KRAS | SEQ ID NO: 56<br>VVGAFGVGK / 26 nM | SEQ ID NO: 57<br>VVGAGGVGK / 89 nM | 1% |
| | KRAS | SEQ ID NO: 58<br>VVGASGVGK / 35 nM | SEQ ID NO: 59<br>VVGAGGVGK / 89 nM | 4.7% |
| | HRAS | SEQ ID NO: 60<br>VVGAGIVGK / 43 nM | SEQ ID NO: 61<br>VVGAGGVGK / 89 nM | Activating mutation<br>(freq. unknown) |
| | EGFR | SEQ ID NO: 62<br>ASGAFGTVYK / 13 nM | SEQ ID NO: 63<br>GSGAFGTVYK / 29 nM | 3% |
| | LRRN3 | SEQ ID NO: 64<br>TIESLPNLKK / 44 nM | SEQ ID NO: 65<br>TIESLPNLKE / 32442 nM | 1.5% |
| | KLF4 | SEQ ID NO: 66<br>KTYTQSSHLK /11 nM | SEQ ID NO: 67<br>KTYTKSSHLK /12 nM | 1% |
| | XPO1 | SEQ ID NO: 68<br>KTVVNKLFK / 24 nM | SEQ ID NO: 69<br>KTVVNKLFE / 16111 nM | 1.8% |
| | FBXW7 | SEQ ID NO: 70<br>TVCCMHLHEK /22 nM | SEQ ID NO: 71<br>TVRCMHLHEK / 22 nM | 3.9% |
| | CTNNB1 | SEQ ID NO: 72<br>TTAPPLSGK / 11 nM | SEQ ID NO: 73<br>TTAPSLSGK / 10 nM | 4.0% |
| | CTNNB1 | SEQ ID NO: 74<br>TTAPALSGK / 16 nM | SEQ ID NO: 75<br>TTAPSLSGK / 10 nM | 1.3% |
| | CTNNB1 | SEQ ID NO: 76<br>TTAPFLSGK / 7 nM | SEQ ID NO: 77<br>TTAPSLSGK / 10 nM | 2.7% |
| | CTNNB1 | SEQ ID NO: 78<br>TTAPYLSGK / 10 nM | SEQ ID NO: 79<br>TTAPSLSGK / 10 nM | 1.3% |
| | PIK3CA | SEQ ID NO: 80<br>AISTRDPLSK / 12 nM | SEQ ID NO: 81<br>AISTRDPLSE / 27122 nM | 3.7% |
| | ABL1 | SEQ ID NO: 82<br>AMEVEEFLK / 36 nM | SEQ ID NO: 83<br>TMEVEEFLK / 38 nM | 1.1% |
| | ABL1 | SEQ ID NO: 84<br>SSATEYLEK / 9 nM | SEQ ID NO: 85<br>SSAMEYLEK / 9 nM | 2.8% |
| | FGFR3 | SEQ ID NO: 86<br>MTTNGRLPVK / 20 nM | SEQ ID NO: 87<br>KTTNGRLPVK / 25 nM | 2% |
| | JAK3 | SEQ ID NO: 88<br>GLLKTVSYK / 19 nM | SEQ ID NO: 89<br>ELLKTVSYK / 247 nM | 5.6% |
| | NOTCH1 | SEQ ID NO: 90<br>RVPHTNVVFK / 22 nM | SEQ ID NO: 91<br>RVLHTNVVFK / 14 nM | 5.5% |
| | PTPN11 | SEQ ID NO: 92<br>ATLAGLVQYY / 40 nM | SEQ ID NO: 93<br>ATLAELVQYY / 73 nM | 0.7% |
| HLA-B*07:02 | STK19 | SEQ ID NO: 94<br>NPIFRFSSL / 14 nM | SEQ ID NO: 95<br>DPIFRFSSL / 246 nM | 5% |
| | IDH2 | SEQ ID NO: 96<br>SPNGTIQNIL / 42 nM | SEQ ID NO: 97<br>SPNGTIRNIL / 23 nM | 8.7% |
| | NOTCH1 | SEQ ID NO: 98<br>FPRELSRVL / 11 nM | SEQ ID NO: 99<br>FLRELSRVL / 282 nM | 5.5% |

| | | | | |
|---|---|---|---|---|
| | NOTCH1 | **SEQ ID NO: 100**<br>SPVLHTNVV / 26 nM | **SEQ ID NO: 101**<br>SRVLHTNVV / 23262 nM | 5.2% |
| | PTPN11 | **SEQ ID NO: 102**<br>MVRSQRSAM / 9 nM | **SEQ ID NO: 103**<br>MVRSQRSGM / 59 nM | 0.7% |
| | TSHR | **SEQ ID NO: 104**<br>VPRFLTCNL / 30 nM | **SEQ ID NO: 105**<br>VPRFLMCNL / 47 nM | 12% |
| **HLA-B*15:01** | RAC2 | **SEQ ID NO: 106**<br>FQGEYIPTVF / 35 nM | **SEQ ID NO: 107**<br>FPGEYIPTVF / 6216 nM | Driver mutation (freq. unknown) |
| | TRAF7 | **SEQ ID NO: 108**<br>AQSYLYSSSY / 29 nM | **SEQ ID NO: 109**<br>AQSYLYSGSY / 33 nM | 2.3% |
| | SMO | **SEQ ID NO: 110**<br>VLAPIGLVF / 40 nM | **SEQ ID NO: 111**<br>VLAPIGLVL / 328 nM | 2.3% |
| | CTNNB1 | **SEQ ID NO: 112**<br>HSGATTTAPF / 40 nM | **SEQ ID NO: 113**<br>HSGATTTAPS / 9015 nM | 2.7% |
| | CTNNB1 | **SEQ ID NO: 114**<br>HSGATTTAPY / 43 nM | **SEQ ID NO: 115**<br>HSGATTTAPS / 9015 nM | 1.3% |
| | ABL1 | **SEQ ID NO: 116**<br>TQISSATEY / 44 nM | **SEQ ID NO: 117**<br>TQISSAMEY / 48 nM | 2.8% |
| | EZH2 | **SEQ ID NO: 118**<br>VQKNEFISEF / 31 nM | **SEQ ID NO: 119**<br>VQKNEFISEY / 37 nM | 5.6% |
| | GATA2 | **SEQ ID NO: 120**<br>YLCNACGFY / 47 nM | **SEQ ID NO: 121**<br>YLCNACGLY / 61 nM | 2.4% |
| | IDH2 | **SEQ ID NO: 122**<br>IQNILGGTVF / 39 nM | **SEQ ID NO: 123**<br>IRNILGGTVF /6936 nM | 8.7% |
| | NOTCH1 | **SEQ ID NO: 124**<br>FQSATDVAPF / 26 nM | **SEQ ID NO: 125**<br>FQSATDVAAF / 28 nM | 2.1% |
| | TSHR | **SEQ ID NO: 126**<br>RMAVLIFTEF /33 nM | **SEQ ID NO: 127**<br>RMAVLIFTDF / 45 nM | 2.4% |
| | TSHR | **SEQ ID NO: 128**<br>RMAVLIFTHF / 23 nM | **SEQ ID NO: 129**<br>RMAVLIFTDF / 45 nM | 2.4% |
| **HLA-C*04:01**<br>SB: strong-binder (< 1000 nM)<br>WB: Weak binder (> 1000 nM) | TP53 | **SEQ ID NO: 130**<br>YLDDRNTFL / SB | **SEQ ID NO: 131**<br>YLDDRNTFR / WB | Hot-spot mutation (freq. unknown) |
| | STK19 | **SEQ ID NO: 132**<br>SAPENPIFRF / SB | **SEQ ID NO: 133**<br>SAPEDPIFRF / WB | 5% |
| | TRRAP | **SEQ ID NO: 134**<br>VFGSVFLF / SB | **SEQ ID NO: 135**<br>VFGSVSLF / WB | 4% |
| | ZNF831 | **SEQ ID NO: 136**<br>FSDAQRPSF / SB | **SEQ ID NO: 137**<br>FSDAQRPSS / WB | 2% |
| | FOXA1 | **SEQ ID NO: 138**<br>RFENGCYL / SB | **SEQ ID NO: 139**<br>MFENGCYL / WB | 1.8% |
| | MED12 | **SEQ ID NO: 140**<br>IVDGAVFAVF / SB | **SEQ ID NO: 141**<br>IVDGAVFAVL / WB | 5.4% |
| | ABL1 | **SEQ ID NO: 142**<br>FYIITELM / SB | **SEQ ID NO: 143**<br>FYIITEFM / SB | 1.8% |
| | DNMT3A | **SEQ ID NO: 144**<br>YTDVSNMSHL / SB | **SEQ ID NO: 145**<br>YTDVSNMSRL / SB | 11% |
| | DNMT3A | **SEQ ID NO: 146** | **SEQ ID NO: 147** | 3.2% |

| | | YTDVSNMSCL / SB | YTDVSNMSRL / SB | |
|---|---|---|---|---|
| | FLT3 | **SEQ ID NO: 148**<br>RYIMSDSNYV / SB | **SEQ ID NO: 149**<br>RDIMSDSNYV / WB | 2.7% |
| | MET | **SEQ ID NO: 150**<br>MYDKEYDSV / SB | **SEQ ID NO: 151**<br>MYDKEYYSV / SB | 14% |
| | NOTCH1 | **SEQ ID NO: 152**<br>VYPEIDNRQCV / SB | **SEQ ID NO: 153**<br>VYLEIDNRQCV / WB | 5.5% |
| | PTPN11 | **SEQ ID NO: 154**<br>YYDLYGGEKFV / SB | **SEQ ID NO: 155**<br>YYDLYGGEKFA / SB | 0.7% |
| HLA-C*07:02<br>SB: strong-<br>binder (< 1000<br>nM)<br>WB: Weak<br>binder (> 1000<br>nM) | STK11 | **SEQ ID NO: 156**<br>SYLGVAEAL / SB nM | **SEQ ID NO: 157**<br>SDLGVAEAL / WB | 1.1% |
| | SMO | **SEQ ID NO: 158**<br>FVLAPIGLVF / SB nM | **SEQ ID NO: 159**<br>FVLAPIGLVL / SB nM | 2.3% |
| | MED12 | **SEQ ID NO: 160**<br>FAVFKAVFVL / SB nM | **SEQ ID NO: 161**<br>FAVLKAVFVL / SB nM | 5.4% |
| | RET | **SEQ ID NO: 162**<br>LAARNILVF / SB nM | **SEQ ID NO: 163**<br>LAARNILVA / WB nM | 6% |
| | TSHR | **SEQ ID NO: 164**<br>RFLTCNLAF / SB nM | **SEQ ID NO: 165**<br>RFLMCNLAF / SB nM | 12% |
| | TSHR | **SEQ ID NO: 166**<br>RMAVLIFTEF / SB nM | **SEQ ID NO: 167**<br>RMAVLIFTDF / WB nM | 2.4% |
| | TSHR | **SEQ ID NO: 168**<br>YTHSEYYNHAF / SB nM | **SEQ ID NO: 169**<br>YTHSEYYNHAI / WB nM | 3.5% |
| | TSHR | **SEQ ID NO: 170**<br>YAKVSTCLPM / SB nM | **SEQ ID NO: 171**<br>YAKVSICLPM / SB nM | 4.7% |

Prediction programs suggested: HLA-A*03:01 to bind a total of 8 mutant epitopes, and A*11:01 bound the maximum number of 21 epitopes. B*07:02 bound 6 and B*15:01 bound 12 epitopes, C*04:01 bound 13 epitopes, and C*07:02 showed *in silico* restriction to 8 epitopes (Figure 5A and Table 14).

The total number of individuals/year with the mutation based on three factors predict the possibility of ATT treatment worldwide (Figure 5B). about 11,220 A*03:01$^+$ individuals/year were estimated to bear mutations that create epitopes to target. Similarly, about 70,389 A*11:01$^+$, about 1,381 B*07:02$^+$, about 2,651 B*15:01$^+$, about 21,532 C*04:01$^+$, and about 10,451 C*07:02$^+$ per year were theoretically calculated as possible mutation bearing individuals (Figure 5C). In total, about 117,624 individuals/year were estimated worldwide carrying these 68 recurrent point mutations in their cancers; predicted epitopes as listed (Table 14) to bind at least one allele of the novel class I haplotype. Six highly represented HLA alleles in the AB*ab*.I transgene design in resemblance to human HLA architecture was encoded as a polycistronic expression cassette (Figure 5D).

**Polycistronic HLA transgene is transcribed to produce mRNA but not translated to express proteins**

Polycistronic primary AB*ab*.I and all modified constructs (Figure 6A) did not express the HLA alleles after staining with the pan HLA ABC antibody (Figure 6B). The HLA expression was measured 48 hours after transient transfection. Polycistronic, H-2 D$^b$ driven AB*ab*.I showed no surface HLA expression in MCA205 cells. 39% of cells expressed GFP. 98% of LCL-BM14 cells showed endogenous HLA expression (Figure 6B, I). LCL-BM14 are EBV-transformed lymphoblastoid B cells.

Six HLAs were switched with enhanced GFP (eGFP) gene or CMV promoter for H-2 D$^b$ to access promoter activity (Figure 6A, II). 13% of MCA205 cells expressed GFP driven by H-2 D$^b$, and 53% of cells expressed GFP from control pMP71-eGFP retroviral plasmid (Figure 6B, II). However, switching the H-2 D$^b$ promoter with CMV did not rescue the expression of the six HLAs (Figure 6B, II).

Next, the 500 bp H-2 D$^b$ polyadenylation (pA) sequence was replaced with a 1 kb full-length (FL) H-2 D$^b$ or synthetic 350 bp bovine growth hormone (bGH) pA to analyze for defects in transcription termination. H-2 D$^b$ or CMV promoter drove pA modified constructs, which were analyzed for surface expression using flow cytometric staining (Figure 6A, III). None of the pA modified constructs showed significant HLA surface expression after transient transfection (data not shown).

H-2 D$^b$ promoter- and CMV promoter-driven primary AB*ab*.I construct with all respective pA modifications (Figure 7A) produced mRNA in MCA205 cells (Figure 7B). HLA mRNA was detected using cDNA amplification with specific primers against three viral peptide linkers, P2A (front region), F2A (middle), and C2A (end region). The H-2 D$^b$ promoter-driven bGH pA construct produced up to 7% of HLA expression compared to that of mouse MHC expression (endogenous D$^b$ gene in MCA205 cells) after transient transfection. The H-2 D$^b$ promoter- driven FL pA and the primary AB*ab*.I construct respectively produced 6% and 2% of HLA expression (Figure 7B, I). A 193% of relative change in the mRNA production to that of the endogenous D$^b$ expression in CMV-driven bGH pA constructs was significantly measured. The FL pA and CMV-primary AB*ab*.I constructs did show a relative change, respectively, of 138% and 75% from basal H-2 D$^b$ (Figure 7B, II).

**HLA alleles are stably expressed when driven by its own 5' and 3' regulatory elements**

H-2 D$^b$ promoter driving six HLA alleles in polycistronic configuration with bGH pA did produce HLA mRNA, but not proteins (Figure 6 and 7).

Six HLA alleles were cloned with or without β2 microglobulin (β2m) as monocistrons (Figure 8A, I) to test their expression before they were placed together into a single class I haplotype, each with its regulatory elements (Figure 8A, II). Monochains without β2m (linked) were co- transfected exogenously with β2m plasmid. Respective leader sequences led HLAs to translocate in the β2m non-linked monochains, e.g., A*03 leader peptide was cloned instead of β2m leader sequence in the HLA-A*03:01 construct.

MCA205 cells were transfected with or without β2m-linked monochains (Figure 8A, I) in the presence or absence of IFN-γ. HLA monochains transfected without β2m (β2m co-transfected) did not show any significant difference in the surface expression of HLA alleles (data not shown). Here, all six monocistrons (as β2m-linked monochains) were stained using pan HLA ABC and anti-human β2m antibody and measured by flow cytometry in transientlytransfected MCA205 cells (Figure 8B).

Pan HLA ABC antibody stained transiently transfected MCA205 cells and the increase in HLA expression was in the range of 9- to 23-fold in A*03 (5.4% to 52%), A*11 (6% to 59%), B*07 (5.6% to 57%), B*15 (5.6% to 56%), C*04 (2.3% to 52%) and C*07 (2.6% to 48%) monochains with 100 ng/ml IFN-γ treatment for 48 hours during transfection (Figure 8B). Similarly, anti- human β2m stained transiently transfected MCA205 cells, the increase in HLA expression was in the range of 1.5- to 8-fold in A*03 (8.6% to 15%), A*11 (9.3% to 22%), B*07 (5.4% to 20%),

B*15 (3.9% to 18%), C*04 (1.6% to 12%) and C*07 (3.7% to 14.6%) monochains with 100 ng/ml IFN-γ treatment for 48 hours during transfection (Figure 8C).

The AB*ab*.I transgene, encoding six chimeric monocistrons, was transfected into the MCA205 cell line for HLA expression analysis. AB*ab*.I HLA haplotype encodes six alleles back-to-back in a head-to-tail configuration. All six genes comprise a promoter and a polyadenylation signal of their own (Figure 8A, II).

Two days after transfection, 16% of cells were positive for HLA alleles (Figure 9A, top). Flow cytometry sorting enriched HLA$^+$ population in the transfected cells. Two days post-sorting and culturing *in vitro,* cells were pre-treated with or without IFN-γ and stained using pan HLA ABC antibody. 41% of cells stained for HLA without IFN-γ, whereas 77% of MCA205 cells stained for HLA alleles after enrichment in the presence of IFN-γ for 48 hours in culture (Figure 9A, bottom). Simultaneously, the sorted cells (HLA$^+$) were stained using HLA-specific antibodies. The increase in HLA expression was in the range of 2- to 4-fold after staining with A*03-specific (19% to 73%), B*07-specific (17% to 69%), and pan HLA-C (32% to 69%) antibodies after 48 hours of 100 ng/ml IFN-γ treatment (Figure 9B, 9C, and 9D). AscI-PacI digested and linearized AB*ab*.I DNA was used for MCA205 transfection to produce a stable cell line, MCA205/AB*ab*.I. After multiple enrichments of HLA$^+$ cells by flow cytometry sorting at different time points, 92% of cells expressed the AB*ab*.I transgene (Figure 9E).

**PiggyBac transposon-mediated targeting produced AB*ab*.I founder mice with six HLAs as a single haplotype**

AB*ab*.I HLA cassette was shown to be both transiently (Figure 9A and 9B) and stably (Figure 8C) expressed on the surface of murine MCA205 cells *in vitro.*

*In vivo* integration of the HLA haplotype was achieved using the hyperactive PiggyBac (PB) transposon system. The PB transposase acts on the ITRs-flanked targeting vector (cassette bearing HLAs) by a cut-paste mechanism. The enzyme was co-transfected as mRNA for the excision (cut) of 5' and 3' ITR regions to release the insert (HLAs) for integration (paste) into transcriptionally active sites of the mouse genome (Figure 10).

After successful rounds of pronuclei injections, allele-specific PCRs confirmed the presence of six HLA alleles in two founder animals (Figure 11A). Ear biopsy DNA confirmed the genomic integration of alleles in F0_Q4115 and F0_Q4118 using genotyping PCRs (Figure 11A, left). As controls, 18s rRNA housekeeping gene and human TCR $\alpha\beta$ genes were checked (Figure 11A, right). Three additional founders: F0_Q6844, F0_Q8552, and F0_Q8556 were positive genotyped (data not shown). Stable homozygous AB*ab*.I mice strain from the Q6844 line was established according to the standard breeding pattern (Figure 11B).

HLA expression was stained and quantified in the peripheral blood cells of C57BL6/N, ABabDII, and AB*ab*.I mice using anti-human $\beta$2m antibody with LCL-BM14 cells (99% $\beta$2m$^+$) as control (Figure 12A and 12B). 3% and 7% of lymphocytes from AB*ab*DII mice, whereas 35% and 40% of lymphocytes from AB*ab*.I mice stained for HLA expression with the $\beta$2m antibody (Figure 12A). Shown here are two AB*ab*.I mice from founder line Q6844. Other founders tested for HLA expression included Q4115, Q4118, Q8552, and Q8556 (data not shown). Mouse to mouse variations was analyzed by staining multiple mice per strain. Mean fluorescence intensity (MFI) comparison of $\beta$2m revealed 4-times more HLA expression in AB*ab*.I compared to that in AB*ab*DII. 120-times higher MFI was observed in human HLA (non-chimeric) expressing LCL-BM14 cells (Figure 12C and 12D). One possible explanation could be that the folding characteristics of human HLA proteins differ from that of a chimeric fusion molecule, which could hinder epitope accessibility to $\beta$2m antibody, which needs to be further analyzed.

**ABab.I mice have higher CD8+ T cell counts compared with the single HLA-bearing ABabDII mice**

Phenotypic T cell characterization depicted increased thymic output/peripheral survival in AB*ab*.I mice (founder F0_Q6844 lineage) compared with AB*ab*DII and HHD mice (Figure 13). HHD mice have a mouse TCR repertoire selected on a human HLA-A2. CD8$^+$ CD4$^+$ profiling of peripheral blood cells (on CD3$^+$ cells) from four mouse strains by flow cytometry showed 13% and 14% of CD8$^+$ T cells in AB*ab*DII, around 6% in both HHD mice, 36% and 45% in AB*ab*.I, and 36% and 41% in C57BL6/N mice (Figure 13A). Shown here are two mice/strain. CD8$^+$ T cell fractions (%) in AB*ab*.I mice were comparable to that in C57BL6/N mice. On average, CD3$^+$ T cells were 90% CD4$^+$ in HHD mice, 64% in AB*ab*DII, 45% in AB*ab*.I, and 55% in C57BL6/N mice (Figure 13A). A significant number of AB*ab*.I mice accumulated 4-times more CD8$^+$ T cell numbers in the periphery than AB*ab*DII mice (Figure 13B). AB*ab*.I mice with high CD8$^+$ T cell counts were inter-crossed at F2 to produce a stable mouse line (Figure 11B). No significant difference in the CD4$^+$ T cell numbers was observed (Figure 13C). CD8/CD4 ratio was higher in AB*ab*.I mice, similar to that in C57BL6/N mice, 3-times more than that in AB*ab*DII mice (Figure 13D).

Comparison of CD8$^+$ and CD4$^+$ T cell populations in pooled lymphoid organs (on CD3$^+$ cells) showed 14% and 12% of CD8$^+$ T cells in AB*ab*DII with 62% and 67% of CD4$^+$ T cells, and 47% and 44% of CD8$^+$ T cells in AB*ab*.I with 39% of CD4$^+$ T cells (Figure 14A). On average, AB*ab*.I mice estimated to have 3.5-times more absolute CD8$^+$ T cell numbers in the major secondary lymphoid organs (Figure 14B) with no significant difference in absolute CD4$^+$ T cell numbers (Figure 14C). Similar to peripheral blood, CD8/CD4 ratio was higher in AB*ab*.I mice, 2.5-times more than that in AB*ab*DII mice (Figure 14D).

**Broader CD8+ T cell repertoire in ABab.I mice compared with ABabDII mice**

TCR$\beta$ repertoire deep sequencing quantified $11.2 \pm 2.7 \times 10^4$ and $4.8 \pm 0.80 \times 10^4$ numbers of unique in-frame amino acid (aa) clonotypes in the CD8$^+$ T cells of AB*ab*.I and AB*ab*DII mice, respectively (Figure 15A). From the three age-unmatched humans, the 30-year-old donor had similar numbers of clonotypes, $11.4 \pm 4.1 \times 10^4$, compared to AB*ab*.I mice (Figure 15A). The total number of unique aa clonotypes in human donors had a high variance. Considering widespread differences in age (30, 50, and 65 years old), we excluded human data from the clone-size analysis. AB*ab*.I mice had 8.5% of rare clones, whereas AB*ab*DII had 5.7% rare clones (Figure 15B). However, AB*ab*DII mice had three times more small clones of 4.1% than AB*ab*.I mice with 1.4% of small clones (Figure 15B). Sequencing of the complete repertoire with full coverage was not possible using ImmunoSEQ™ deep sequencing. So, the estimation of the total TCR repertoire in CD8$^+$ T cells of mice and humans was determined using computational statistics. The iChao1 estimator calculated the total number of clonotypes per mouse (observed and undetected) using information from clones that occurred only once or twice. iChao1 estimates true species richness using lower bound rarely occurring ones. The first human donor, a 30-year-old individual had the most diverse repertoire with up to $2 \times 10^6$ clonotypes with $1 \times 10^6$ on average (n=3). After humans, AB*ab*.I mice had $0.66 \times 10^6$ clonotypes on average (n=5) with a diverse repertoire. AB*ab*DII had the lowest estimation with $1.8 \times 10^5$ clonotypes (Figure 15C). V$_\beta$ (Figure 16A and J$_\beta$ (Figure 16B) gene usages were analyzed present in both out-of-frame and in-frame TCR$\beta$ clonotypes. The latter represents the preselection pool, which is an unbiased measure of the diversity of all possible V(D)J recombination events that could have occurred before positive selection. In AB*ab*DII mice, and so, thereby in AB*ab*.I mice, most of the detected V$_\beta$ segments were found to be rearranged, except for TRBV5-1 and TRBV6-1, which were previously reported for non-existence or lack of expression (Figure 16A). AB*ab*DII and AB*ab*.I mice showed no difference in any preferred V$_\beta$ usage in the preselection pool. Similarly, in humans, the out-of-frame V$_\beta$ gene usage pattern was comparable without any selection preference.

Both in the pre- and post-selection pool, some V$_\beta$ genes were either over or underrepresented in mice. Over-represented V$_\beta$ genes include TRBV20, TRBV21, TRBV23, TRBV27, and TRBV28. Underrepresented V$_\beta$ genes include TRBV6-2/6-3, TRBV6-4, TRBV7-9, TRBV9-1, and TRBV10-1, mostly closer to the 5' end (Figure 16A, top). Single nucleotide polymorphisms might be a reason for this difference in representation. Preferential overrepresentation of two V$_\beta$ genes such as TRBV7-3 and TRBV12-3/12-4 in AB*ab*DII and AB*ab*.I mice was observed compared to humans (Figure 16A, top).

In-frame V$_\beta$ genes represent the post-selection pool with similar frequencies, except some genes in the 5' region such as TRBV2-1 and TRBV4-1, and TRBV7-9 were highly selected-in by positive selection, whereas, V$_\beta$ genes such as TRBV7-3, TRBV20/21/23/24, and TRBV25 were selected-out in the thymus (Figure 16A, bottom). Only one V$_\beta$ gene in each strain, V$_\beta$4-1 in AB*ab*.I and V$_\beta$12-3/12-4 in AB*ab*DII mice, seem to be predominantly represented in the post- selection pool in one or the other mice (Figure 16A, bottom).

J$_\beta$ gene usages were also non-random and similar between the transgenic mice and humans (Figure 16B), although some J$_\beta$ segments were used more frequently in mice than in humans: TRBJ1-2, TRBJ2-1, and TRBJ2-3 (Figure 16B, top). Notably, J$_\beta$2-07 in the 3' end was represented high with ~ 30 % total usage in the post-selection pool in mice than in humans (Figure 16B, bottom).

**ABab.I mice enrich longer CDR3-containing T cell receptors**

The V(D)J recombination event in the TCR$\beta$ chain generates CDR3 diversity and is responsible for antigen recognition in conjugation with the VJ events in the TCR$\alpha$ chain. AB*ab*.I mice had enriched significantly longer CDR3$\beta$ regions than AB*ab*DII mice.

Considering shorter CDR3 lengths, in AB*ab*DII, 5.5% of clonotypes expressed TCRs with 33 bp, 11.5% with 36 bp, and 21% with 39 bp CDR3 sequences. In AB*ab*.I, lengths corresponded to 4.9% with 33 bp, 10% with 36 bp, and 19% with 39 bp CDR3 sequences. In AB*ab*.I mice, a higher percentage of clones expressed longer CDR3 sequences than AB*ab*DII mice, 23% versus 21% with 48 bp, 10% versus 8.3% with 51 bp, and 3.7% versus 3.4% with 54 bp (Figure 17A). However, humans produced the lengthiest CDR3$\beta$ sequences compared to mice (Figure 17A). On average, AB*ab*.I mice had TCRs with 42 $\pm$ 0.1 bp CDR3 lengths compared with 41.5 $\pm$ 0.06 bp CDR3 lengths in AB*ab*DII mice, but not longer than humans with an average CDR3 of 43.5 $\pm$ 0.18 bp (Figure 17B).

**Natural human HLA haplotype in ABab.I mice educates a wide array of unique clones**

Shared clones were compared between mouse strains and among humans using Jaccard similarity index estimation. Jaccard index (J) evaluates the shared immune repertoire between samples. J index ranges from 0 to 1, and the score is calculated based on the formula: the total number of shared clones divided by the total number of unique clones across two samples, J (A, B) = A $\cap$ B/A $\cup$ B. High shared clonality within strains (AB*ab*.I with AB*ab*.I or AB*ab*DII with AB*ab*DII) were detected than across strains (AB*ab*.I with AB*ab*DII or vice versa). AB*ab*.I mice shared ~ 6.4% of clones among each other, Jaccard index: 0.0636 $\pm$ 0.003, and AB*ab*DII mice shared ~ 5% of clones within their group, Jaccard index: 0.0498 $\pm$ 0.004 (Figure 18A). Humans shared more clones with AB*ab*.I mice (Jaccard index: 0.006 $\pm$ 0.001) than with AB*ab*DII mice (Jaccard index: 0.005 $\pm$ 0.001) or among each other with no statistically significant difference (Figure 18A).

Both AB*ab*DII and AB*ab*.I produced more shared TCR$\beta$ clones among each other than they shared with humans, likely the possibility of similar genetic background with human TCR gene loci. Notably, AB*ab*.I mice shared fewer clones with AB*ab*DII, Jaccard index: 0.04 $\pm$ 0.003 than within their group, 0.0636 $\pm$ 0.003 (Figure 18A).

AB*ab*.I mice generated more unique clones compared with AB*ab*DII mice. Pooled analysis from 5 mice per strain determined the total number of shared and unique clones. AB*ab*DII and AB*ab*.I mice shared less than 100,000 clones (0.8 $\times$ 10$^5$) between each other. AB*ab*.I mice produced almost 4-times more unique and rare clones (AB*ab*.I: 7.5 $\times$ 10$^5$) than AB*ab*DII mice: 2 $\times$ 10$^5$ TCR$\beta$ clones (Figure 18B).

**HLA alleles in ABab.I mice can efficiently present epitopes and trigger T cell responses ex vivo**

To determine epitope presentation by HLA alleles in the newly generated AB*ab*.I mice, peripheral blood or lymphoid organ cells isolated from the mice ex *vivo* were co-cultured with TCR-transduced T cells recognizing model epitopes. Previously described (A3-, A11-, B7-, B15-, and C7-restricted), or in-house generated (C4-restricted) TCRs against a panel of model antigens acquired from literature resources were used in co-culture assays (Figure 19A). The following SEQ ID NOs apply to Figure 19A:

| HLA restriction | TCR chains | V(D)J recombined regions | CDR3 regions | Epitope sequences (predicted $IC_{50}$) |
|---|---|---|---|---|
| A*03:01 | TCR Vα chain TCR Vβ chain | TRAV9-2-TRAJ30-1 TRBV13-1-TRBD2-1-TRBJ2-3 | **SEQ ID NO: 181** CALSDRERDDKIIF **SEQ ID NO: 182** CASSHEGLAGEFF | **SEQ ID NO: 183** KMRMRRMRR (29.18 nM) |
| A*11:01 | TCR Vα chain TCR Vβ chain | TRAV3-3-TRAJ17-1 TRBV4-1-TRBD2-1-TRBJ2-1 | **SEQ ID NO: 184** CAVSGGTNSAGNKLTF **SEQ ID NO: 185** CASSRDWGPAEQFF | **SEQ ID NO: 186** VVGAVGVGK (65.47 nM) |
| B*07:02 | TCR Vα chain TCR Vβ chain | TRAV17-1-TRAJ12-1 TRBV7-9-TRBD1-1-TRBJ2-1 | **SEQ ID NO: 187** CATVIRMDSSYKLIF **SEQ ID NO: 188** CASSLIGVSSYNEQFF | **SEQ ID NO: 189** TPRVTGGGAM (3.86 nM) |
| B*15:01 | TCR Vα chain TCR Vβ chain | TRAV17-1-TRAJ47-2 TRBV6-5-TRBD1-1-TRBJ2-5 | **SEQ ID NO: 190** CAESEYGNKLVF **SEQ ID NO: 191** CASSYRQQETQYF | **SEQ ID NO: 192** SAFRCFIVY (118.20 nM) |
| C*04:01 | TCR Vα chain TCR Vβ chain | TRAV26-2-TRAJ49-1 TRBV15-2-TRBD2-2-TRBJ2-1 | **SEQ ID NO: 193** CILRDNTGNQFYF **SEQ ID NO: 194** CATSRDGSSYNEQFF | **SEQ ID NO: 195** QYDPVAALF (499.34 nM) |
| C*07:02 | TCR Vα chain TCR Vβ chain | TRAV13-1-TRAJ34-1 TRBV25-1-TRBD2-1-TRBJ2-7 | **SEQ ID NO: 196** CAASGATDKLIF **SEQ ID NO: 197** CASSGGHEQYF | **SEQ ID NO: 198** VRIGHLYIL (647.00 nM) |

Co-culture supernatants were analyzed for murine IFN-γ to assess effector T cells for HLA allele recognition bound with its respective model epitope. As positive control, MCA205/AB*ab*.I cells expressing six HLA alleles as in AB*ab*.I mice were used as target cells to present model epitopes to transduced T cells. After 16 hours of co-culture, IFN-γ was measured in the supernatant to confirm the functional activity. All five model TCRs recognized the respective peptide HLA combination (pHLA-TCR) and produced IFN-γ in the range of 9437 ± 863 pg/ml - 11,877 ± 299 pg/ml (Figure 19B).

Peripheral blood and lymphoid organ cells used as target cells to measure pHLA-TCR interaction in AB*ab*.I mice ex *vivo*. Post-recognition of blood pHLAs, all TCR-transduced T cells produced IFN-γ, and the lowest release was 4168 ± 308 pg/ml, and the highest was 7873 ± 212 pg/ml (Figure 19C), proving functional activity. Similarly, pHLAs from secondary lymphoid organs were recognized (spleen, lymph nodes as target cells), all model TCR-transduced T cells released IFN-γ in the range of 5540 ± 216 pg/ml - 8054 ± 270 pg/ml (Figure 19C).

PMA/Ionomycin activated protein kinase C and opened calcium ($Ca^{2+}$) channels in transduced T cells for maximum IFN-$\gamma$ release. Whereas, in untransduced T cells, no IFN-$\gamma$ release (only low background) was measured (Figure 19A and 19B). HLA-A2 (present in AB*ab*.I mice) restricted TCR, T1367-transduced T cells were used in co-cultures as an internal reference control in all presentation assays. A model TCR against CMV pp65 epitope was isolated in- house from immunized HuTCR mice expressing HLA-C*04:01 as monochain (refer to discussion section 4.5). All model epitopes (Figure 19A) used in co-culture assays were taken forward for *in vivo* immunizations into AB*ab*.I mice to analyze CD8$^+$ T cell responses by IFN-$\gamma$ release using intracellular staining.

### AB*ab*.I mice mount immune responses against peptide antigens *in vivo*

We analyzed AB*ab*.I mice for antigen-specific CD8$^+$ T cell responses against described model antigens (Figure 19A) using peptide immunization (Figure 20). All six model antigens were *in silico* predicted to be strong-binders to their respective HLA alleles. Intracellular staining measured about 1.8% of CD8$^+$ T cells produced IFN-$\gamma$ against the mutated calreticulin (mCALR) protein-derived epitope, KMRMRRMRR (SEQ ID NO: 173). About 6.9% of CD8$^+$ T cells released IFN-$\gamma$ against the mutated KRAS protein-derived G12V epitope, VVGAVGVGK (SEQ ID NO: 174). About 1.4% against B*07:02 bound CMV epitope, TPRVTGGGAM (SEQ ID NO: 175), about 0.2% against B*15:01 bound HPV epitope, SAFRCFIVY (SEQ ID NO: 176), about 0.3% against C*04:01 bound CMV epitope, QYDPVAALF (SEQ ID NO: 177), and about 0.23% against MAGE-A12 epitope, VRIGHLYIL (SEQ ID NO: 178) (Figure 20). The MAGE-A1 epitope, KVLEYVIKV (SEQ ID NO: 179) bound HLA-A2 elicited ~ 1% of CD8$^+$ T cell response. The unspecific peptide used in *in vitro* restimulations was a NY- ESO epitope, APRGPHGGAASGL (SEQ ID NO: 180). No T cell responses were observed in any case against the unspecific peptide proving a specific response towards the immunized peptide. CD8$^+$ T cell responses against anti-CD3/CD28 beads were in the range of 1.7% - 14% (Figure 20) Thus, CD8$^+$ T cell responses towards a panel of described epitopes in the AB*ab*.I mouse model depicts *in vivo* functionality of the human HLA haplotype.

### Discussion

In the course of the present invention, we generated the 'AB*ab*.I transgenic mouse model' using the PiggyBac transposon strategy with six HLA class I alleles on the 'humanized TCR' mouse background. We confirmed the human HLA haplotype in mice by genotyping the six HLAs using allele- specific PCRs. We confirmed the phenotype by staining with pan- and HLA-specific antibodies, as much as available, using flow cytometry analysis. We provided evidence supporting the increased thymic output in AB*ab*.I mice by profiling CD8$^+$ and CD4$^+$ T cells in the periphery. We characterized the immune repertoire using ImmunoSEQ™ deep sequencing and observed that AB*ab*.I mice have a four-fold broader T cell receptor repertoire than the AB*ab*DII mice. We proved the antigen presentation efficiency of all six HLA alleles present in AB*ab*.I mice using IFN-$\gamma$ release assays by the effector CD8$^+$ T cells ex *vivo* and *in vivo.*

### Computational neoantigen prediction and the need for epitope immunogenicity validation - mutant neoepitope S722F is not endogenously processed

We predicted 175 class I mutant epitopes out of 266 mutations to bind 18 highly ranked (population-wise) HLA alleles. All 266 mutations were somatic point mutations that occurred recurrently more than twice in different cancer entities. We focused only on recurrent driver mutations as they are present in every cancer cell throughout the disease and yield truly tumour-specific antigens. Earlier evidence compared chemotherapy by a drug inactivating the cancer-driving oncogene with tumour-specific CD8$^+$ ATT treatment and proved relapse-free tumour elimination happens by eradicating escape variants with tumour-stroma destruction. Based on this, we reasoned that in our study, by raising TCRs against recurrent neoantigens derived from driver mutations, complete eradication of tumours without antigen loss and relapse might be possible.

In our *in silico* screen, we marked epitopes as strong-binders with an $IC_{50} < 50$ nM criterion. pMHC affinity ranges between $IC_{50}$ <1 nM to >20,000 nM. Engels *et al.* investigated pMHC affinities with therapy outcome on tumour rejection versus relapse. The study reported that strong pMHC affinities with $IC_{50}$ <10 nM caused tumour rejection in every case and relapsed if $IC_{50}$ > 100 nM. Subsequently, two reviews highlighted the importance of selecting high-affinity ($IC_{50}$ <50 nM) class I epitopes for CD8$^+$ ATT treatments in the context of a tripartite interaction between peptide-bound-MHC and TCR. Based on these studies, in our prediction screen, we selected mutant neoepitopes with an $IC_{50}$ of less than 50 nM to raise TCRs against these recurrent antigens using AB*ab*DII and AB*ab*.I (generated as a part of the present invention). Not all predicted neoantigens yield immunogenic epitopes. Epitope immunogenicity combines natural endogenous processing for pMHC presentation with the ability to trigger CD8$^+$ T cell responses. *In silico* defined epitopes must be evaluated with caution as they could generate false positives. In our study, we predicted TRRAP-S722F driver mutation to contain a putative neoantigen, a 9-mer epitope. The S722F mutant epitope triggered an immune response in AB*ab*DII mice. TCRs isolated from mice were specific for S722F and recognized low amounts of the exogenously loaded peptide. However, they did not react to the melanoma cell line that naturally expressed the mutated gene confirming that the 9-mer epitope is not endogenously processed and presented. By this reverse immunology approach, we proved that the S722F-predicted epitope is not endogenously processed. In addition to the TRRAP mutant (S722F), predicted neoepitopes from genes, C-KIT (K642E), XPO1 (E571K), and FOXA1 (D226N) did not elicit CD8+ T cell responses in AB*ab*DII mice (own data not shown, unpublished).

Several other findings confirmed our observation that reverse immunology (first predicting epitopes and at last investigating endogenous processing) is not the best way to choose target antigens for adoptive T cell therapy. In an epitope immunogenicity study of the vaccinia virus, ~ 100 epitopes showed good predicted binding affinities to HLA-A2 with an $IC_{50}$ of < 100 nM. Only 50% of those elicited CD8+ T cell responses after HLA-A2 transgenic mice immunization. 15% of response-eliciting peptides further got processed by the proteasome. Out of this, 11% elicited spontaneous CD8+ responses upon vaccinia virus infection, proving that only 1/14 of the predicted fraction created an immunogenic epitope[126]. In another melanoma RNA vaccination trial, CD8+ T cell responses were relatively low compared with CD4+ T cell responses, which might be due to more promiscuous class II peptide presentation by the MHC. In yet another long peptide melanoma vaccination trial, only 16% of the predicted neoepitopes elicited CD8+ T cell responses.

Software programs do not properly predict endogenous epitope modifications in the antigen presentation pathway using their algorithms. These alterations inside proteasomal-, post- proteasomal-, and peptide transport-compartments are decisive factors to determine epitopes as immunogenic and targetable. One study showed proteasomal epitope destruction of a proposed epitope. Another example reported that ERAP-trimmed epitopes managed easier escape than not-trimmed epitopes. In a third study, although the human individual showed CD8+ T cell responses against the CDK4 mutant antigen, it was later verified in an experimental cancer model to be a relatively poor target for T cell therapy.

Thus, artificial network algorithms cannot accurately predict proteasomal processing, peptide transport, N-terminal trimming by ERAP enzymes, or splicing mechanisms to define human neoantigens for ATT of cancers.

## Polycistronic transgene design requires mRNA stability and protein misfolding considerations - lessons learned

Polycistronic primary AB*ab*.I transgene segregated by viral 2A linker elements showed no surface protein expression from any of the six HLA alleles. So, we manipulated the primary construct driven by the H-2 $D^b$ promoter with three polyadenylation signals (short $D^b$, full-length $D^b$, and bGH pA). Simultaneously, we replaced the promoter sequence in these three constructs with the viral CMV promoter. In all of these six constructs, though we quantified significant amounts of HLA mRNA by qRT-PCR in H-2 $D^b$ primary constructs, 30x more in CMV-primary constructs, we did not detect cell surface protein expression by flow cytometry. Multiple reasons could have caused this untranslatable situation of HLA mRNAs affecting its cell surface presentation. Such considerations could be mRNA instability of the long HLA transcript, inefficient excision of viral 2A linkers, and unfavorable post-translational modifications causing misfolding of nascent polypeptide chains.

We contemplated that mRNA stability was affected by a single common 3'-UTR region regulating all six HLA alleles. Moreover, recent evidence confirmed that native 2A linkers functioned inefficiently by not self-cleaving themselves to release proteins; of note, our primary transgene contained native 2A peptide linkers. A study on sequence divergence versus homology reported that transient misfolding does occur in proteins derived from polycistronic mRNAs, whereas they disappear in the case of low sequence identity; HLA class I chimeric chains maintain 90-98% homology. In line with this, a recent review postulated that open questions exist on how nascent polypeptides from polycistrons fold without forming aggregates for cellular clearance.

Based on these factors, we decided to clone and express all six HLA alleles as monocistrons driven by their own 5'- and 3' regulatory elements (DNA sequence of the AB*ab*.I transgene construct in the founder mice - SEQ ID NO: 208).

## Protein characterization of chimeric HLAs in AB*ab*.I mice and the need for monochain- specific antibodies - human lymphoblastoid cell lines express high levels of HLA proteins

Single HLA alleles were cloned from the primary polycistronic transgene and recloned as monocistrons for a successful surface expression. To confirm cell surface expression of HLAs, always a pan antibody or an antibody against the human β2m chain present in all chimeric alleles were stained. There are no specific antibodies that exist against the unique HLA alleles in the AB*ab*.I mice, which can be used for flow cytometry analysis, except for HLA-A*03:01 and HLA-B*07:02, which makes validation of HLA proteins difficult.

Notably, both pan HLA and β2m antibodies stained different HLA chains with different intensities, especially the β2m antibody stained HLA genes in lymphoblastoid cell lines (LCL) with high fluorescence intensity profile (MFI) than the chimeric alleles in AB*ab*.I mice. Notably, the HLA genes in LCL lines are fully human; pHLA stabilization happens by human proteome-derived antigens. HLA alleles in AB*ab*.I mice are chimeric; pHLA stabilization occurs here by mouse proteosome-derived antigens. This difference in the expression pattern raised the question, whether it was the consequence of peptides on the HLA stability that make the β2m antibody stain the fully human HLAs better with an increased shift in the mean fluorescence intensity.

Interestingly, a study using HLA antibodies from multiparous women confirmed the impact of peptides on HLA reactivity by antibodies. Based on this, we speculated, the more stable the pHLAs are on the surface, the more they can be detectable through antibody staining. Thus, we could relate alloantibody staining differences to our β2m staining difference between LCLs and AB*ab*.I mice. Another possible explanation could be that the chimeric HLAs in AB*ab*.I mice fold differently in an unnatural fashion, which could obstruct epitope accessibility by the β2m antibody, which requires further analysis.

On a different note, a fully human HLA expression in transgenic mice without β 2m linked as a fusion protein could enhance surface stability, thereby improving detection. However, the maintenance of mouse CD8α interaction with the α3 domain of the chimeric mouse-human HLA heavy chain is crucial for CD8$^+$ T cell co-activation.

**Generation of AB*ab*.I transgenic mice using pronuclei injection technology**

We produced two versions of AB*ab*.I transgene-targeting vectors for injections into AB*ab*DII donor oocytes. The first vector flanked the Rosa26 guide RNA sequences for CRISPR-Cas9 recognition, and the second vector flanked inverted terminal repeat sequences for hyperactive PiggyBac recognition on both 5' and 3' ends, respectively.

Our exploratory attempts of using CRISPR-Cas9 technology through homology-independent targeted integration (HITI) failed even after several trials. Injections for random integration with vector backbone-free naked DNA transgene were not successful as well. For HITI with CRISPR-Cas9, we reasoned that a better strategy could have been to use homologous arms flanking the transgene cassette for efficient integration into the Rosa26 locus. Until now, the HITI method showed high integration efficiency only in the post-mitotic neurons but not in oocytes, so it requires in-depth optimization to make a successful attempt in mouse embryos. DNA strand breaks in the mouse genome during pronuclei microinjection procedures are the primary cause of random transgene integration (multiple copies) in a head-to-tail fashion. During chromosomal end-joining, the break-point ends take up the available DNA transgene as donor template to ligate the junctions. We speculate that the large size of the AB*ab*.I transgene with homologous sequences (~ 17 kb) could have been the reason for failed naked transgene integration as concatemers. The use of the PiggyBac transposon strategy might enable us to target one copy per locus.

Hence, simultaneously we utilized hyperactive PiggyBac transposase to catalyze AB*ab*.I transgene cassette with ITRs for integration into the mouse genome. We successfully produced five founders, Q4115, Q4118, Q6844, Q8552, and Q8556. The first founder, Q4115, did not germline transmit the HLA alleles in its genome to F1 generation in nearly 250 genotyped mice. We believe that PiggyBac catalyzed the transgene for integration into the genome after multiple cell divisions of the embryo or late integration, which resulted in a mosaic founder mouse not being able to germline transmit to the F1 generation as per Mendelian genetics. Nonetheless, we managed to achieve germline transmission in all the other founders, Q4118, Q6844, Q8552, and Q8556.

We investigated the phenotype of different founder mice based on the absolute numbers of CD8$^+$ and CD4$^+$ T cells in the blood and the secondary lymphoid organs (data not shown for other founders except Q6844 line). The Q6844 lineage showed the highest and consistent CD8$^+$ T cell numbers across generations from F1 until F4 providing indirect evidence for thymic and likely peripheral HLA expression. Significant HLA expression is necessary for positive selection in the thymus and T cell maintenance in the periphery. Hence, we hypothesized that the high CD8$^+$ T cell numbers (Q6844 lineage) reflect efficient positive thymic selection and increased TCR repertoire diversity. Besides, the impact on the homeostatic proliferation of T cells is another interesting question to address. For this, it is crucial to consider CD44 staining as an activation marker to understand homeostasis in the periphery based on thymic output and the number of precursor T cells that come into circulation.

However, using next-generation ImmunoSEQ repertoire sequencing, we managed to deep sequence the TCRβ immune repertoire of the CD8$^+$ T cells of AB*ab*.I in comparison with AB*ab*DII mice.

**Next-generation deep sequencing of AB*ab*.I and AB*ab*DII mice - CD8$^+$ TCRβ immune-sequencing**

Having observed the differences in the absolute CD8$^+$ T cell numbers between AB*ab*.I and AB*ab*DII mice, we deep sequenced the TCRβ repertoire in these strains to understand whether the increase in CD8$^+$ T cell numbers resulted in a broader TCR repertoire. Being this the case, we could exclude the possibility that the increase in CD8$^+$ T cell numbers in AB*ab*.I compared to AB*ab*DII mice is a result of increased homeostatic expansion. Of note, AB*ab*DII mice bear a single HLA-A2, whereas AB*ab*.I mice have additionally a complete human HLA haplotype of six alleles. Thus, we investigated whether or not the introduction of multiple class I alleles into the humanized TCR mice made the repertoire broader and also how broad it was compared with AB*ab*DII mice.

We proved using iChao1 estimation that AB*ab*.I mice have a diversified repertoire and on average, four times broader than that of AB*ab*DII mice. While we deep-sequenced approximately two-fold more numbers of in-frame amino acid clonotypes in AB*ab*.I mice versus AB*ab*DII mice ($11.2 \pm 2.7 \times 10^4$ versus $4.8 \pm 0.80 \times 10^4$), the iChao1 estimator theoretically extrapolated the repertoire to be four times broader; back-calculated to a complete mouse repertoire by including clones that occurred only once or twice (lower bound events). By theoretical extrapolations, we showed that AB*ab*.I mice used diverse V and J genes and produced close to $1 \times 10^6$ clonotypes compared with AB*ab*DII, which only generated a maximum of $2.57 \times 10^5$ clonotypes (~ four times lower). More diversity in AB*ab*.I mice reflected higher percentages of rare and unique clones and that the thymic selection indeed selected novel V(D)J TCRβ combinations on multiple HLA alleles. Thus, the expression of six HLA alleles in AB*ab*.I mice selected a highly diverse human TCR repertoire, likely because of enhanced positive selection and overall thymic output, which is reduced in AB*ab*DII mice.

The out-of-frame TCR repertoire represents the preselection pool before positive selection and gives a precise estimate, how frequently individual V and J segments are rearranged in AB*ab*DII and AB*ab*.I mice. As expected, the preselection (out-of-frame) TCR repertoire was nearly identical between AB*ab*DII and AB*ab*.I mice, because the human TCR gene loci and the recombination enzymes were the same. The post-positive selection (in-frame) repertoire reflected, on one hand, the frequency by which individual V and J segments were rearranged, and on the other hand, revealed that some $V_\beta$ gene segments were overrepresented in AB*ab*.I mice, for example, TRBV4-1, whereas those were underrepresented in AB*ab*DII mice. Similarly, TRBV12-3/12-4 is overrepresented in AB*ab*DII, but not in AB*ab*.I mice.

We have made an apparent connection between multiple HLA expression and their impact on repertoire diversity. Looking from the TCR side, we speculate that some $V_\beta$ genes could have a higher intrinsic affinity towards some but not other HLA alleles. A critical requirement for positive selection is the interaction between the CDR1 and CDR2 regions with the selecting MHC class I molecule. The 40-50 $V_\beta$ genes differ in their CDR1 and CDR2 regions and the MHC class I genes are highly polymorphic. Therefore, one can assume that the intrinsic affinity for some $V_\beta$ gene segments is higher for some MHC I alleles but lower for others and that the MHC and TCR gene loci co-evolved to ensure more efficient positive selection. This hypothesis was supported by Chen *et al*. They sequenced the human CD4[+] TCR repertoire in mice selected on either a single mouse or a single human MHC class II molecule. They found that the species-compatible human MHC class II selected a broader CD4[+] TCR repertoire compared to the species-incompatible mouse MHC molecule I-A[b]. This was explained by, on average, the increased inherent affinity of the human TCRs for the human MHC class II.

In analogy, one might assume that some $V_\beta$ gene segments have a higher inherent affinity to HLA-A2, whereas other $V_\beta$ gene segments have a lower intrinsic affinity to HLA-A2, but in turn a higher inherent affinity for any of the other six MHC class I alleles in AB*ab*.I mice. This then would mean, that certain TCRs can only poorly be selected by HLA-A2, but are then more efficiently selected by the other MHC I alleles in AB*ab*.I mice.

Chen *et al*. also observed that TCRs selected on mouse MHC II had, on average, shorter CDR3 sequences compared to human TCRs that were selected on human MHC II molecules. The hypothesis is that different inherent affinities of $V_\beta$ gene segments for MHC were adjusted by CDR3 lengths, i.e. a particular $V_\beta$ gene segment with a relatively low inherent affinity for MHC gets better selected if it carries a shorter CDR3. We extend these findings, because a TCR repertoire selected on multiple class I alleles contained, on average, slightly larger CDR3 regions compared to the TCR repertoire selected only on a single MHC class I allele, in our case HLA-A2. The best explanation is that some $V_\beta$ segments have an increased inherent affinity for any of the six novel MHC class I alleles in ABab.I mice, which allows for the selection of TCRs with a slightly larger CDR3 region. This not only explained the diversity in AB*ab*.I compared with AB*ab*DII mice, but could also result in TCRs with higher specificity. This assumption is based on studies in mice that were deficient in terminal deoxynucleotidyl transferase (TdT) expression, which resulted in TCRs with short CDR3s. These TCRs tended to be more promiscuous and cross-reactive. Despite the fact that the TCR repertoire in AB*ab*DII is relatively diverse, we speculate that the TCR repertoire in AB*ab*DII mice is not optimal because the CDR3 regions are relatively short[21]. In contrast, thymocytes in AB*ab*.I mice can choose one out of six additional class I alleles for getting selected. Therefore, we assume that different MHC alleles in the thymus compete with each other for selecting a given T cell clone. If this is correct, T cell clones that are not being able to be selected by HLA-A2 could be selected by any of the other six MHC class I alleles for which they have an increased inherent affinity and then also allowing longer CDR3s being accepted by not impeding positive selection.

It should be noted, however, that the hypothesis that the T cells in AB*ab*.I mice find its HLA fit with optimal intrinsic affinity at a price of reduced selection on HLA-A2 remains speculative so far. This hypothesis is difficult to prove because it is also not known whether different MHC class I alleles can select similar diverse TCR repertoire.

Therefore, we are in the process of generating and characterizing mice with single HLA alleles of those that are contained in AB*ab*.I mice. By TCR repertoire sequencing of mice expressing single HLA alleles on the same background, we can verify whether or not certain $V_\beta$ segments have a different inherent affinity for different HLA alleles[21,248]. For this, single AB*ab*-A*03, AB*ab*- A*11, AB*ab*-B*07, AB*ab*-B*15, AB*ab*-C*04, and AB*ab*-C*07 founder lines were generated and require characterization before deep sequencing (own data not shown, unpublished). In these mice, we can prove or disprove whether certain $V_\beta$ gene segments are preferentially selected or preferentially ignored by individual MHC I alleles.

However, mice, in general, select shorter CDR3 TCRs than humans. The reason for this could be higher TdT and exonuclease activity in humans compared to mice or a longer time window of expression. The TdT-exonuclease machinery with the recombination activating genes (RAG) evolved to magnify the addition or deletion of nucleotides (more editing); such increased activity generates higher numbers of unique TCRs with lengthier CDR3s impacting the overall repertoire.

Because ABab.I mice generated four times more unique clones than ABabDII, they also shared more TCRβ clonotypes (around 1000 compared to around 500) with the repertoire of human donors. The numbers of shared clones between ABab.I mice are surprisingly high, as they were not HLA-matched. The high clonal overlap is likely to be explained by the non-random V and J usage. In humans, the theoretical number of TCRβ sequences is $5 \times 10^{11}$. If V-J rearrangement was random, less than five shared TCRβ clones would be expected between any two individuals, but in fact, around 10,000 shared clones have been detected, consistent with the detection of only 0.1% of the theoretical $5 \times 10^{11}$ in human peripheral blood. Our data in ABab.I mice support the data in humans and suggest TCRα/TCRβ combinatorial diversity is larger than previously anticipated. We also deep-sequenced the TCRα repertoire from these two mouse groups. Preliminary data analysis showed that the TCRα repertoire is also more diverse in ABab.I compared to ABabDII mice (own data not shown, unpublished). Detailed analysis is still required to reveal combinatorial diversity between these mice.

In summary, a more diverse repertoire, closer to humans, might be achieved through extended humanization of the human TCR gene loci mouse model.

## Functional characterization of ABab.I mice *ex vivo and in vivo*

We showed that ex *vivo* derived APCs from ABab.I mice can efficiently present model epitopes on all six HLA alleles and triggered effector T cells to produce IFN-γ. Due to the lack of HLA- specific antibodies, we devised this *in vitro* co-culture assay to estimate both HLA expression and its functionality by activating model-epitope-restricted TCR-modified T cells. APCs from cells of blood, spleen, and lymph nodes were used as targets to effectors, as they recapitulate the physiological circulating immune system when it comes to immunization.

The purpose of generating ABab.I mice was to isolate T cell receptors to treat cancer patients targeting tumour-restricted antigens. Here, we show not only ex *vivo* but *in vivo* by immunizing ABab.I mice with a wide range of model antigens that each were known to bind to one of the six HLA alleles, represented in the mice. These epitopes were selected to cover different antigen categories such as a frame-shift derived mutant (mCALR on A*03), point mutant (KRAS-G12V on A*11), viral (CMV-pp65 on B*07 and C*04, HPV-E5 on B*15), and tumour- associated (MAGE-A12 on HLA-C*07:02) epitopes.

Although most epitopes selected for the panel were proven to be endogenously processed, mice responded stronger to mutant KRAS and viral epitopes than others, irrespective of the predicted $IC_{50}$ nM values. It is not a concern for processed epitopes, but for epitopes not proven to be processed, DNA or Adenovirus (encoding antigens) immunization expressing complete protein sequence can leverage the ABab.I mouse model as a platform to discover novel epitopes.

In conclusion, the ABab.I mouse model expressing six HLA alleles as like in a natural human situation can become an incomparable novel tool to isolate novel T cell receptors to be used clinically in ATT of cancer.

## Understanding the biology behind a full human HLA haplotype in a mouse model

The ABab.I model with its diverse TCRβ repertoire has proven to be a valuable tool to isolate unique T cell receptors for therapy. However, understanding the TCRα locus repertoire might determine the combinatorial diversity of the overall combination of TCRs that ABab.I mice could produce. By this, we can also study how comparable is the TCR diversity in ABab.I mice to the theoretically possible combinations of TCR diversity (~ $10^{15}$ clonotypes).

Different intrinsic $V_\beta$ affinity towards some but not other human HLA alleles could be the next possible step to understand skewness during positive selection in the thymus. For this, comparison of deep sequencing data from ABab.I mice with single ABab-animals on humanized TCR background, ABab-A*03, ABab-A*11, ABab-B*07, ABab-B*15, ABab-C*04, and ABab-C*07 mice will allow us to clearly understand if there is a bias in TCR affinity towards HLAs and whether different HLA alleles can select similar or dissimilar TCR repertoires.

Knocking-out HLA-A2 should be the next event from single ABab- and ABab.I mice to generate pure lines devoid of the A2 gene; the ideal representation of a normal HLA class I loci in humans.

## Epitope discovery of non-HLA-A*02:01-restricted antigens using ABab.I mice

Reverse immunology based on epitope prediction is a concern. Thus, ABab.I mice provide an opportunity to identify immunogenic epitopes and isolate T cell receptors simultaneously. Direct immunology by immunizing ABab.I

mice with DNA, adenovirus, or mRNA encoding the complete protein for epitope discovery would yield TCRs restricted to such newly discovered epitopes for ATT.

**AB*ab*.I mice as a novel tool to isolate human TCRs for ATT of cancer**

The prediction screen pointed out the number of individuals per year with the mutation. In principle, AB*ab*.I mice immunized with such mutant epitopes will yield a range of high- to optimal affinity TCRs to be taken forward to clinical applications, however, information on endogenous is necessary.

Hence, AB*ab*.I mice are not entitled only to understand human repertoire shaped on a natural class I HLA environment, but also a novel model to isolate T cell receptors against a wide variety of antigens for adoptive T cell therapy.

**Example 2: Unbiased discovery of the new MAGE-A1 epitope GTLEEVPTA (SEQ ID NO: 200)**

In this example a new MAGE-A1 epitope was screened for in the AB*ab*.I mice.

Firstly, an immune response to MAGE-A1 restricted by HLA-A*02 in ABab-I mice was studied following vaccination (Figure 21A). An ABab.I mouse was immunized with full-length MAGE-A1 mutated at position 279 (V→D) to destroy the dominant HLA-A*02-restricted epitope KVLEYVIKV (278-286) (SEQ ID NO: 199). On day 7 after the 3rd immunization, peripheral blood was stimulated with MAGE-A1-expressing cells and stained intracellularly for IFNg (Figure 21A).

Thereafter, the epitope of MAGE-A1 was located to which one of the TCRs, termed TCR1 that as isolated from the respective ABab.I mouse binds. For this purpose (location of the epitope), TCR1-transduced human T cells were co-cultured with K562-A2 cells expressing MAGE-A1, fragments of MAGE-A1 (F1-F3) or loaded with KVLEYVIKV (KVL). F1-F3 span fragments of MAGE-A1 as follows: F1 1-97, F2 98-222, F3 223-309. It was found that the TCR1 recognizes a HLA-A*02-restricted MAGE-A1 epitope that is located in the N-terminal fragment formed by amino acid residues 1-97 of MAGE-A1 (Figure 21B).

Finally, for determination of the exact epitope, TCR1-transduced human T cells were co-cultured with K562-A2 cells loaded with the predicted nonamer binders (Table 15) or with KVLEYVIKV (KVL). It was found that MAGE-A1-reactive TCR1 recognizes the nonamer GTLEEVPTA (SEQ ID NO: 200) (Figure 21C). It is to be noted here that the nonamer GTLEEVPTA was predicted as a MAGE-A1 epitope in US patent application US2003/0148973A1, however, no HLA-A2 restricted TCRs could be raised against this epitope in US 2003/0148973 but only TCRs against the four epitopes shown in Table IV of US 2003/0148973 A1. Notably, in US 2003/0148973 A genetically modified mice that express a human HLA-A2 MHC molecule were immunized with mature MAGE-A1 protein, meaning essentially the same approach for immunization with MAGE-A1 that was used here was also employed in US 2003/0148973. This clearly shows the non-human mammalian animals of the present invention such as mice of the present invention allow to raise CD8[+] TCRs against MHC I presented peptides/antigen against which no TCR could have been generated.

Table 15: Predicted nonamer binders.

| Short name | Sequence | Affinity (nM) | Rank (%) |
|---|---|---|---|
| SLH | **SEQ ID NO: 201** SLHCKPEEA | 928.5 | 4 |
| AQQ | **SEQ ID NO: 202** AQQEALGLV | 726.6 | 3.5 |
| ALG | **SEQ ID NO: 203** ALGLVCVQA | 1377.2 | 5 |
| GLV | **SEQ ID NO: 204** GLVCVQAAT | 1188.4 | 4.5 |
| ATS | **SEQ ID NO: 205** ATSSSSPLV | 555.8 | 3 |
| GTL | **SEQ ID NO: 200** GTLEEVPTA | 573.9 | 3 |
| STS | **SEQ ID NO: 206** STSCILESL | 1199 | 4.5 |

(continued)

| Short name | Sequence | Affinity (nM) | Rank (%) |
|---|---|---|---|
| KVL | **SEQ ID NO: 207** KVLEYVIKV | 6.91 | 0.06 |

Summarizing, a novel MAGE-A1 epitope was identified in an unbiased manner, which is different from the known dominant HLA-A*02-restriced epitope KVL 278-286. This further proves that the ABab.I mouse allows for identification of novel epitopes as well as shows that ABab.I mouse of the present invention can generate TCRs against epitopes that are not generated by other means (e.g., US2003/0148973A1).

**Example 3: Immunization Experiment (*in vivo* immune response analysis)**

To analyze CD8$^+$ killer T cell responses *in vivo,* we immunized the multi-HLA mice with described model epitopes as peptides (given in the table, 9- & 10-mers). All six model epitopes were *in silico* (using software) predicted to be strong binders to their respective HLA alleles.

**Table 16:** Model peptides used for immunization that are presented by HLA alleles present in the multi-HLAABab.I mice

| HLA restriction | Epitope sequences (predicted IC$_{50}$) with gene information |
|---|---|
| A*03:01 | (SEQ ID NO: 173) KMRMRRMRR (29.18 nM) Encoding gene: mCALR frame-shift-p7 |
| A*11:01 | (SEQ ID NO: 174) VVGAVGVGK (65.47 nM) Encoding gene: KRAS G12V |
| B*07:02 | (SEQ ID NO: 175) TPRVTGGGAM (3.86 nM) Encoding gene: CMV pp65 |
| B*15:01 | (SEQ ID NO: 176) SAFRCFIVY (118.20 nM) Encoding gene: HPV E5 |
| C*04:01 | (SEQ ID NO: 177) QYDPVAALF Encoding gene: CMVpp65 |
| C*07:02 | (SEQ ID NO: 178) VRIGHLYIL (647.00 nM) Encoding gene: MAGE-A12 |

Procedure: young mice were peptide-immunized (minimum twice) with intervals of at least 4-weeks between injections. Seven days post-booster injections, PBMCs from multi-HLA mice were stimulated in vitro with either the same peptides used for immunization (right), or unspecific peptides (left), or CD3/CD28 beads (middle, positive control). Intracellular flow cytometry staining measured the amount of IFN-$\gamma$ cytokine released by the CD8$^+$ T cells after reacting to their respective epitopes. By flow cytometry, CD8$^+$ T cells were in vitro measured for CD3, CD8, and intracellularly IFN-$\gamma$ expression. Dot plots in the figure depict CD8$^+$ IFN-$\gamma^+$ T cells (gated on CD3$^+$ lymphocytes). As an unspecific peptide, NY-ESO peptide (APRGPHGGAASGL) was used (as a negative control) only for *in vitro* stimulations.
One flow cytometry plot out of multiple responders is given. Out of 6-7 mice/epitope, 6 mice responded against KRAS (HLA-A*11:01 restriction), 3 mice against MAGE-A1 (HLA-A*02:01 restriction, as explained in Example 2 above) and HPV (HLA-B*07:02 and/or HLA-B*15:01 restriction), 1 mouse responded against mCALR (HLA-A*03:01 restriction), CMV (HLA-C*04:01 restriction, and MAGE-A12 (HLA-C*07:02 restriction) peptides. Immune responses are analyzed through flow cytometry in the blood (PBMCs) of immunized mice. For that intracellularly IFN-gamma cytokine proteins are stained using antibodies together with CD3 and CD8 antibodies. When PBMCs are re-stimulated in the blood *in vitro* (7 days post-booster injections) again with the respective peptides that were used initially for the immunization, responses against the specific peptide and not against unspecific peptides are seen. Beads were used as a positive control to show the fitness of our T cells in the blood of our multi-HLA mice.

**Example 4: Analysis of T cell response of HuTCR mice for individual HLA class I alleles**

In this example the T cell response of ABab.I mice for the individual HLA class I alleles integrated into their genome was analyzed.

For this purpose, 11 ABab.I mice were immunized several times with DNA encoding full-length MAGE-A1 which was mutated at position 279 (V→D) to destroy the dominant HLA-A*02-restricted epitope KVLEYVIKV (278-286) (SEQ ID NO: 199), cf., Example 2 in this respect. Then, blood T-cells obtained from the mice were cocultured with cell lines expressing different single or dual HLAs as well as MAGE-A1. The number of interferon γ secreting cells (IFNg$^+$) of CD8$^+$ T-cells was measured by intracellular staining. By so doing, the T cell response of HLA-A*02:01, HLA-A*03:01, HLA-A*11:01 were analyzed individually, while the combined T-cell response was analyzed for the alleles HLA-B*07:02 and HLA-B*15:01 as well as for the alleles HLA-C*04:01 and HLA-C*07:02.

The results of this experiment are shown in Fig. 25. Mice having a positive T cell response are indicated by circles located above the dashed background line while mice having a negative T cell response are indicated by circles located below the dashed background line. As evident from Fig. 25, for all of individually tested HLA class I alleles (HLA-A*02:01, HLA-A*03:01, HLA-A*11:01) and also for the jointly tested HLA class I alleles (HLA-B*07:02 and HLA-B*15:01 and HLA-C*04:01 and HLA-C*07:02, respectively), ABab.I mice were found with an active T-cell response directed towards a MAGE-A1-derived epitope in blood.

Thus, also this experiment confirms the ABab.I mice of the invention are able to elicit a CD8$^+$ T cell response for all human HLA class I alleles that the mice carry. In this context, it is also noted that the amount of active T cells in blood is usually lower than the amount of active T cells found in the spleen of mice. Thus, based on the results of the T cell count in blood, it is safe to assume that the number of active CD8+ T cells in the spleen of the immunized ABab.I mice and thus the active T cell response is even higher.

In addition, the results depicted in Fig. 25 show that a transgenic animal that carries multiple human HLA class I alleles allows to directly compare the strength of the T cell response that is associated with each of the human alleles. In the case of MAGE-A1 as antigen, the HLA-A*02:01 restricted CD8$^+$ T cell response is the strongest, both in terms of quantity (7 out of 11 immunized mice showing a positive T cell response) and in terms of biological activity (with the strongest IFNg$^+$ response of the T cells), cf., Example 2. Thus, transgenic animals of the present invention allow to directly compare the strength of the T cell response while assessing the T cell response associated with multiple human HLA alleles at the same time. This "multiplexing" is a further advantage of the present invention.

One skilled in the art would readily appreciate that the present invention is well adapted to carry out the objects and obtain the ends and advantages mentioned, as well as those inherent therein. Further, it will be readily apparent to one skilled in the art that varying substitutions and modifications may be made to the invention disclosed herein without departing from the scope and spirit of the invention. The compositions, methods, procedures, treatments, molecules and specific compounds described herein are presently representative of certain embodiments are exemplary and are not intended as limitations on the scope of the invention. Changes therein and other uses will occur to those skilled in the art which are encompassed within the spirit of the invention are defined by the scope of the claims. The listing or discussion of a previously published document in this specification should not necessarily be taken as an acknowledgement that the document is part of the state of the art or is common general knowledge.

The invention illustratively described herein may suitably be practiced in the absence of any element or elements, limitation or limitations, not specifically disclosed herein. Thus, for example, the terms "comprising", "including," "containing", etc. shall be read expansively and without limitation. Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the invention claimed. Thus, it should be understood that although the present invention has been specifically disclosed by exemplary embodiments and optional features, modification and variation of the inventions embodied herein may be resorted to by those skilled in the art, and that such modifications and variations are considered to be within the scope of this invention.

The invention has been described broadly and generically herein. Each of the narrower species and sub-generic groupings falling within the generic disclosure also form part of the invention. This includes the generic description of the invention with a proviso or negative limitation removing any subject matter from the genus, regardless of whether or not the excised material is specifically recited herein.

**Claims**

1. A non-human mammal comprising in its genome at least two (e.g., at least three, at least four, at least five, at least six or at least seven) human leukocyte antigen (HLA) class I alleles, wherein said at least two human HLA alleles are functionally expressed in that the corresponding MHC I polypeptides are expressed on the surface of cells of said mammal and present MHC antigens to which the non-human mammal provides an antigen-specific CD8+ T cell response and optionally wherein said at least two human HLA class I alleles comprise:

   a) at least one human HLA-A allele; and/or
   b) at least one human HLA-B allele; and/or
   c) at least one human HLA-C allele; and/or
   d) (a) and (b); and/or
   e) (a) and (c); and/or
   f) (b) and (c); and/or
   g) (a), (b) and (c).

2. The non-human mammal of claim 1, wherein the mammal further comprises in its genome at least one human HLA class II allele.

3. The non-human mammal of any one of claims 1 to 2, wherein the at least two human HLA class I alleles comprise one or more HLA-A class I alleles selected from the group consisting of:

   a) HLA-A*03,
   b) HLA-A*11,
   c) HLA-A*01,
   d) HLA-A*26,
   e) HLA-A*24,
   f) HLA-A*32,
   g) HLA-A*02,
   h) HLA-A*33, and
   i) HLA-A*31,

   preferably said at least two HLA class I alleles are selected from human HLA allele groups consisting of: HLA-A*03, HLA-A*11 and HLA-A*02.

4. The non-human mammal of any one of claims 1 to 3, wherein the at least two HLA class I alleles comprise one or more HLA-B class I alleles selected from the group consisting of:

   a) HLA-B*07,
   b) HLA-B*15,
   c) HLA-B*58,
   d) HLA-B*40,
   e) HLA-B*35,
   f) HLA-B*08,
   g) HLA-B*46,
   h) HLA-B*52, and
   i) HLA-B*51,

   preferably said at least two HLA class I alleles are selected from human HLA allele groups consisting of: HLA-B*07 and HLA-B*15.

5. The non-human mammal of any one of claims 1 to 4, wherein the at least two HLA class I alleles comprise one or more HLA-C class I alleles selected from the group consisting of:

   a) HLA-C*04,
   b) HLA-C*07,
   c) HLA-C*16,
   d) HLA-C*03,

e) HLA-C*07,
f) HLA-C*06,
g) HLA-C*01, and
h) HLA-C*15,

preferably said at least two HLA class I alleles are selected form human HLA allele groups consisting of: HLA-C*04 and HLA-C*07.

6. The non-human mammal of any one of claims 1 to 5, wherein the mammal comprises in its genome six or seven human leukocyte antigen (HLA) class I alleles selected form human HLA allele groups comprising or consisting of:

a) HLA-A*03,
b) HLA-A*11,
c) HLA-B*07,
d) HLA-B*15,
e) HLA-C*04,
f) HLA-C*07 and
g) HLA-A*02.

7. The non-human mammal of claim 6, wherein the seven human HLA class I alleles comprise or consist of:

a) HLA-A*03:01,
b) HLA-A*11:01,
c) HLA-B*07:02,
d) HLA-B*15:01,
e) HLA-C*04:01,
f) HLA-C*07:02 and
g) HLA-A*02:01.

8. The non-human mammal of any one of the forgoing claims, wherein every one of the at least two HLA alleles is encoded as a monocistron, preferably wherein every one of said at least two HLA alleles encoded as monocistron is selected form human HLA allele groups comprising or consisting of:

a) HLA-A*03,
b) HLA-A*11,
c) HLA-B*07,
d) HLA-B*15,
e) HLA-C*04 and
f) HLA-C*07.

9. The non-human mammal of any one of the foregoing claims, wherein the mammal comprises in its genome the full human T cell receptor (TCR) loci.

10. The non-human mammal of claim 9, where the mammal is deficient in its endogenous T cell receptor (TCR) loci.

11. A nucleic acid construct comprising a nucleic acid encoding at least two (e.g., at least three, at least four, at least five, at least six or at least seven) human HLA class I alleles, wherein said nucleic acid construct is capable of functionally expressing said at least two or at least three human HLA alleles in that the corresponding MHC I polypeptides are expressed on the surface of cells of a non-human mammal comprising the nucleic acid construct and present MHC antigens to which the non-human mammal provides an antigen-specific CD8+ T cell response and optionally wherein said at least two human HLA class I alleles comprise:

a) at least one human HLA-A allele; and/or
b) at least one human HLA-B allele; and/or
c) at least one human HLA-C allele; and/or
d) (a) and (b); and/or
e) (a) and (c); and/or
f) (b) and (c); and/or

g) (a), (b) and (c).

12. The nucleic acid construct of claim 11, wherein every one of the at least two HLA alleles is encoded as a monocistron, preferably every one of said at least two HLA alleles encoded as monocistron are selected form human HLA allele groups comprising or consisting of:

a) HLA-A*03,
b) HLA-A*11,
c) HLA-B*07,
d) HLA-B*15,
e) HLA-C*04 and
f) HLA-C*07.

13. A method of generating one or more T cell receptors that are capable of binding to an antigen of interest, the method comprising administering to a non-human animal as defined in any one of claims 1 to 10 an antigen of interest.

14. A method of identifying an epitope to which an immune response can be elicited, the method comprising administering to a non-human animal as defined in any one of claims 1 to 10 an antigen of interest suspected to contain an epitope to which an immune response can be elicited.

15. An epitope to which an immune response can be elicited, the epitope being identified by the method of claim 14.

## Figure 1

# Figure 2

Figure 3

**A**

p.Serine722→phenylalanine
(c.2165C→T)
Hot-spot mutation
Abrogates p53 interaction

TRRAP

FAT Domain

PI3/4 Kinase Domain

**B**

| TRRAP nonamer (wild-type and mutant) epitopes | NetMHC 3.2 prediction affinity score restricted to HLA-A*02:01 | Mutation frequency (Melanoma) | Melanoma incidence/year (Europe) | Frequency of HLA-A*02:01 allele (Europe) | Total no. of individuals/year with the mutation (possible ATT treatment) (Europe) |
|---|---|---|---|---|---|
| KLVFGSV S→(F) L Wild-type (Mutant) | 55 nM→29 nM Wild-type (Mutant) | 4% (0.04) | 86,000 | 26.5% (0.265) | ~ 911 patients |

**C**

S722→F (c.2165C→T)

```
TRRAP_Homosap   700 MGSNVELSNLYLKLFKLVFGSVSLFAAENEQMLKPHLHKIVNSSMELAQT   749
                    ||||||||||||||||||||||||||||||||||||||||||||||||||
TRRAP_Musmusc   700 MGSNVELSNLYLKLFKLVFGSVSLFAAENEQMLKPHLHKIVNSSMELAQT   749
```

# Figure 4

**A**

*TCR discovery pipeline of TRRAP S722F mutant*

| Week 1 | Week 4 | Week 8 | Week 12 | Week 13 | Week 15 |
|--------|--------|--------|---------|---------|---------|
| prime injection | 2nd injection | 3rd injection | 4th injection | T cell culture of whole splenocytes | IFN-γ capture of antigen-specific T cells, TCR isolation & characterization |

## Figure 4 (continued)

Week 13 – CD8⁺ T cell response in blood against S722F (7 days post 4th injection)

EP 4 215 042 A1

Figure 4 (cont.)

**C**

## Week 15 – CD8⁺ T cell response in spleen against S722F (10 days post T cell culture)

w/o peptide

Anti-CD3/CD28 beads

Wild-type peptide

Mutant S722F peptide

CD8

IFN-γ

**D**

## Week 15 – IFN-γ⁺ S722F-reactive T cells sort for TCR isolation

Wild-type peptide

Mutant S722F peptide

12,000 cells sorted

CD8

IFN-γ

| TRRAP TCR chains | V(D)J recombined regions | CDR3 regions | Clonally dominant TCR chains |
|---|---|---|---|
| TCR Vα chain | TRAV17-1-TRAJ58 | CATDAEETSGSRLTF | 11/14 |
| TCR Vβ chain | TRBV27-1-TRBD2-2-TRBJ2-1 | CASSLAGIYNEQFF | 12/15 |
| | | | Sub-clonally dominant TCR chains |
| TCR Vα chain | TRAV38-2-1-TRAJ28-1 | CAYRSPYSGAGSYQ | 2/20 |
| TCR Vα chain | TRAV26-2-TRAJ29-1 | CILRDDSGNTPLVF | 1/23 |
| TCR Vβ chain | TRBV27-1-TRBD2-2-TRBJ2-1 | CASSLAGAYNEQFF | 3/15 |

F

Titration of TRRAP epitopes on T2 cells

— Mutant-S722F
— Wild-type-S722

IFN-γ pg/ml

Peptide concentration

A375 (TRRAP mutant)
SK-Mel-37 (TRRAP wild-type)
PMA/Iono

IFN-γ pg/ml

Untransduced
TCR-TRRAP (S722F) (TRAV17/TRBV27)
TCR-ESO

EP 4 215 042 A1

Figure 4 (cont.)

A

Figure 5

| *In silico* screen of 266 putative mutant epitopes against 18 HLA class I alleles | | | | | | | | |
|---|---|---|---|---|---|---|---|---|
| Top 6 high ranked HLA-A | Mutant epitopes predicted | Allele frequency (Europe) | Top 6 high ranked HLA-B | Mutant epitopes predicted | Allele frequency (Europe) | Top 6 high ranked HLA-C | Mutant epitopes predicted | Allele frequency (Europe) |
| A*02:01 | 23 | 26% | B*07:02 | 6 | 10% | C*07:01 | 9 | 16% |
| A*03:01 | 8 | 13% | B*35:01 | 6 | 7% | C*04:01 | 13 | 12% |
| A*01:01 | 5 | 12% | B*15:01 | 12 | 5% | C*07:02 | 8 | 12% |
| A*11:01 | 21 | 7% | B*27:05 | 7 | 5% | C*03:04 | 6 | 5% |
| A*23:01 | 3 | 4% | B*40:01 | 5 | 4% | C*16:01 | 21 | 4% |
| A*26:01 | 3 | 3% | B*58:01 | 9 | 2% | C*01:02 | 12 | 3% |

# Figure 5 (cont.)

# Figure 5 (cont.)

LS: Leader sequence; Linker: 15-aa polypeptide chain; TM: Transmembrane domain; CYT: Cytoplasmic domain; pA: Polyadenylation signal

eGFP: enhanced GFP; CMV: cytomegalovirus; FL D^b pA and bGH pA: Full-length H-2 D^b and bovine growth hormone polyadenylation signal

# Figure 6 (cont.)

**A**

**Polycistronic primary AB*ab*.I transgene**

eGFP: enhanced GFP; CMV: cytomegalovirus; FL D<sup>b</sup> pA and bGH pA: Full-length H-2 D<sup>b</sup> and bovine growth hormone polyadenylation signal

# Figure 7 (cont.)

**B**

**I** Polyadenylation signal modified H-2 D^b driven constructs

**II** Polyadenylation signal modified CMV driven constructs

LS: Leader sequence; Linker: 15-aa polypeptide chain; TM: Transmembrane domain; CYT: Cytoplasmic domain; bGH pA: bovine growth hormone polyadenylation signal

## Figure 8 (cont.)

### I  Surface expression profile of HLA monochains

B

Pan HLA-ABC

SSC-A

## Figure 8 (cont.)

# Figure 9

**II ABab.I transgene with six chimeric HLA monocistrons as a single class I haplotype**

**A**

Untransfected | 48 hrs post transfection

MCA205 cells + ABab.I transgene

99,6 | 0 | 81,0 | 15,8

48 hrs post flow cytometry sorting

Without IFN-γ | With 100ng/mL IFN-γ

Untransfected MCA205 cells

99,5 | 0,058 | 99,2 | 0,12

MCA205 cells + ABab.I transgene

52,9 | 41,3 | 19,1 | 77,0

SSC-A

Pan HLA-ABC

## Figure 9 (cont.)

**48 hours post flow cytometry sorting, stained for HLA-specific antibodies**

**Figure 9 (cont.)**

**Figure 9 (cont.)**

**Figure 9 (cont.)**

E

Transfection with AscI-PacI linearized ABab.I transgene
Staining after multiple flow sort enrichments

Untransfected

ABab.I transgene expressing
MCA205 stable cell line

# Figure 10

# Figure 11

**B**

ABab.I founder
**F0**

ABabDII
Homozygous strain

ABab.I F3 generation

ABab.I F3 generation

**ABab.I homozygous generation**

Germline transmission | **F1**

**F4**

ABab.I F1 generation

ABab.I F1 generation

**F2**

ABab.I F2 generation

ABab.I F2 generation

* Mice with high CD8+ T cell numbers were crossed

**F3**

Backcrossing to NMRI mice for homozygosity assessment

Breeding pairs that produced only positive F3 offsprings with ABab.I genotype were further inter-crossed

Figure 12

# Figure 13

# Figure 13 (cont.)

Figure 14

## Figure 15

# Figure 16

A

TRBV

## Figure 16 (cont.)

**B**

TRBJ

# Figure 17

Figure 18

**A**

*Model TCRs restricted towards epitopes presented by HLA alleles in ABabI mice*

| HLA restriction | TCR chains | V(D)J recombined regions | CDR3 regions | Epitope sequences (predicted $IC_{50}$) | References |
|---|---|---|---|---|---|
| A*03:01 | TCR Vα chain<br>TCR Vβ chain | TRAV9-2-TRAJ30-1<br>TRBV13-1-TRBD2-1-TRBJ2-3 | CALSDRERDDKIIF<br>CASSHEGLAGEFF | KMRMRRMRR (29.18 nM) | 217 |
| A*11:01 | TCR Vα chain<br>TCR Vβ chain | TRAV3-3-TRAJ17-1<br>TRBV4-1-TRBD2-1-TRBJ2-1 | CAVSGGTNSAGNKLTF<br>CASSRDWGPAEQFF | VVGAVGVGK (65.47 nM) | 218 |
| B*07:02 | TCR Vα chain<br>TCR Vβ chain | TRAV17-1-TRAJ12-1<br>TRBV7-9-TRBD1-1-TRBJ2-1 | CATVIRMDSSYKLIF<br>CASSLIGVSSYNEQFF | TPRVTGGGAM (3.86 nM) | 219 |
| B*15:01 | TCR Vα chain<br>TCR Vβ chain | TRAV17-1-TRAJ47-2<br>TRBV6-5-TRBD1-1-TRBJ2-5 | CAESEYGNKLVF<br>CASSYRQQETQYF | SAFRCFIVY (118.20 nM) | 219 |
| C*04:01 | TCR Vα chain<br>TCR Vβ chain | TRAV26-2-TRAJ49-1<br>TRBV15-2-TRBD2-2-TRBJ2-1 | CILRDNTGNQFYF<br>CATSRDGSSYNEQFF | QYDPVAALF (499.34 nM) | 220 (epitope), in-house TCR generation, own data, unpublished |
| C*07:02 | TCR Vα chain<br>TCR Vβ chain | TRAV13-1-TRAJ34-1<br>TRBV25-1-TRBD2-1-TRBJ2-7 | CAASGATDKLIF<br>CASSGGHEQYF | VRIGHLYIL (847.00 nM) | 221 |

Figure 19

# Figure 19 (cont.)

# Figure 20

# Figure 21

**A.**

**B.**

**C.**

# Figure 22

**Figure 23**

HuTCR mice

TCR

CD4

CD8   Chimeric HLA

Mouse

Human

**Figure 24**

HuTCR mice

Single HLA class I  Multi-HLA class I

CD4

CD8

22.1  45.2

Gated on CD3[+]

**Figure 25**

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | BOUCHERMA R ET AL: "HLA-A*01:03, HLA-A*24:02, HLA-B*08:01, HLA-B*27:05, HLA-B*35:01, HLA-B*44:02, and HLA-C*07:01 Monochain Transgenic/H-2 Class I Null Mice: Novel Versatile Preclinical Models of Human T Cell Responses", THE JOURNAL OF IMMUNOLOGY, WILLIAMS & WILKINS CO, US, vol. 191, no. 2, 15 July 2013 (2013-07-15), pages 583-593, XP002744179, ISSN: 0022-1767, DOI: 10.4049/JIMMUNOL.1300483 [retrieved on 2013-06-17] | 15 | INV. A01K67/027 C07K14/725 C07K14/74 C12N15/85 |
| Y | * page 584 * | 1-15 | |
| X | EP 3 058 819 A1 (TAIHO PHARMACEUTICAL CO LTD [JP]) 24 August 2016 (2016-08-24) | 1-3, 13-15 | |
| Y | * claims 6,8-11; figure 1 * | 1-15 | |
| X | MANUEL A FRIESE ET AL: "Opposing effects of HLA class I molecules in tuning autoreactive CD8+ T cells in multiple sclerosis", NATURE MEDICINE, vol. 14, no. 11, 26 October 2008 (2008-10-26), pages 1227-1235, XP055119299, ISSN: 1078-8956, DOI: 10.1038/nm.1881 * page 1232 * | 1,3,9 | TECHNICAL FIELDS SEARCHED (IPC) A01K C07K C12N |
| Y | WO 2014/083173 A1 (MAX DELBRÜCK CT FÜR MOLEKULARE MEDIZIN MDC BERLIN BUCH [DE]) 5 June 2014 (2014-06-05) * claims 1,2 * | 1-10 | |

-/--

The present search report has been drawn up for all claims

| Place of search | Date of completion of the search | Examiner |
|---|---|---|
| Munich | 16 June 2022 | Deleu, Laurent |

EPO FORM 1503 03.82 (P04C01)

Europäisches Patentamt
European Patent Office
Office européen des brevets

# EUROPEAN SEARCH REPORT

**Application Number**

EP 22 15 2603

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | ALTER IDAN ET AL: "HLA class I haplotype diversity is consistent with selection for frequent existing haplotypes", PLOS COMPUTATIONAL BIOLOGY, vol. 13, no. 8, 28 August 2017 (2017-08-28), page e1005693, XP055931701, DOI: 10.1371/journal.pcbi.1005693 Retrieved from the Internet: URL:https://journals.plos.org/ploscompbiol/article/file?id=10.1371/journal.pcbi.1005693&type=printable> * the whole document * | 3-8,12 | |
| Y | SANCHEZ-MAZAS ALICIA ET AL: "A New HLA Map of Europe: Regional Genetic Variation and Its Implication for Peopling History, Disease-Association Studies and Tissue Transplantation", HUMAN HEREDITY., vol. 76, no. 3-4, 2013, pages 162-177, XP055932076, CH ISSN: 0001-5652, DOI: 10.1159/000360855 Retrieved from the Internet: URL:https://www.karger.com/Article/Pdf/360855> * the whole document * | 3-8,12 | **TECHNICAL FIELDS SEARCHED (IPC)** |
| Y | WO 2021/083807 A1 (UNIV HEIDELBERG [DE]) 6 May 2021 (2021-05-06) * figure 3 * | 11,12 | |
| Y | US 2018/116190 A1 (VORONINA VERA [US] ET AL) 3 May 2018 (2018-05-03) * claims 1-18 * | 2 | |

The present search report has been drawn up for all claims

| Place of search | Date of completion of the search | Examiner |
|---|---|---|
| Munich | 16 June 2022 | Deleu, Laurent |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 4 215 042 A1**

## ANNEX TO THE EUROPEAN SEARCH REPORT
## ON EUROPEAN PATENT APPLICATION NO.

EP 22 15 2603

This annex lists the patent family members relating to the patent documents cited in the above-mentioned European search report.
The members are as contained in the European Patent Office EDP file on
The European Patent Office is in no way liable for these particulars which are merely given for the purpose of information.

16-06-2022

| Patent document cited in search report | | Publication date | Patent family member(s) | | Publication date |
|---|---|---|---|---|---|
| EP 3058819 | A1 | 24-08-2016 | CN | 105578876 A | 11-05-2016 |
| | | | EP | 3058819 A1 | 24-08-2016 |
| | | | ES | 2874501 T3 | 05-11-2021 |
| | | | HK | 1223507 A1 | 04-08-2017 |
| | | | JP | 6236461 B2 | 22-11-2017 |
| | | | JP | WO2015056774 A1 | 09-03-2017 |
| | | | US | 2016227750 A1 | 11-08-2016 |
| | | | WO | 2015056774 A1 | 23-04-2015 |
| WO 2014083173 | A1 | 05-06-2014 | AU | 2013351062 A1 | 04-06-2015 |
| | | | AU | 2018203663 A1 | 07-06-2018 |
| | | | AU | 2020200092 A1 | 30-01-2020 |
| | | | CA | 2891967 A1 | 05-06-2014 |
| | | | CN | 104853765 A | 19-08-2015 |
| | | | EP | 2925349 A1 | 07-10-2015 |
| | | | GB | 2508414 A | 04-06-2014 |
| | | | HK | 1214942 A1 | 12-08-2016 |
| | | | JP | 6615612 B2 | 04-12-2019 |
| | | | JP | 2015536665 A | 24-12-2015 |
| | | | US | 2015307585 A1 | 29-10-2015 |
| | | | WO | 2014083173 A1 | 05-06-2014 |
| WO 2021083807 | A1 | 06-05-2021 | NONE | | |
| US 2018116190 | A1 | 03-05-2018 | US | 2015342163 A1 | 03-12-2015 |
| | | | US | 2018116190 A1 | 03-05-2018 |

For more details about this annex : see Official Journal of the European Patent Office, No. 12/82

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5525711 A
- US 4711955 A
- US 5792608 A
- EP 302175 A
- US 20030148973 A1
- US 20030148973 A

**Non-patent literature cited in the description**

- **LI et al.** *Nature Medicine,* 2010, vol. 16 (9), 1029-1034
- **MOORE et al.** Humanization of T cell-mediated immunity in mice. *Sci. Immunol.,* 2021, vol. 6, 4026
- **KIMURA et al.** Detailed analysis of the mouse H-2Kb promoter: enhancer-like sequences and their role in the regulation of class I gene expression. *Cell,* 31 January 1986, vol. 44 (2), 261-72
- **ZAMBROWICZ et al.** Disruption of Overlapping Transcripts in the Rosa Beta Geo 26 Gene Trap Strain Leads to Widespread Expression of Beta-Galactosidase in Mouse Embryos and Hematopoietic Cells. *Proc Natl Acad Sci U S A,* 1997, vol. 94, 3789-94
- **GAMPER et al.** *Nucleic Acids Res,* 2000, vol. 28 (21), 4332-4339
- **LI, L.-P. et al.** Transgenic mice with a diverse human T cell antigen receptor repertoire. *Nat. Med.,* 2010, vol. 16, 1029